# EUROPEAN PATENT APPLICATION

(11) **EP 4 434 986 A1**
(43) Date of publication of application: **25.09.2024**
(21) Application number: 22894954.1
(22) Date of filing: 18.11.2022
(51) Int. Cl.: C07D 471/04, C07D 487/04, A61K 31/437, A61K 31/4375, A61K 31/517, A61P 1/04, A61P 9/00, A61P 1/16, A61P 29/00

(54) **15-PGDH INHIBITOR AND USE THEREOF**

(30) Priority: 18.11.2021 CN 202111372073; 15.07.2022 CN 202210837015; 10.11.2022 CN 202211408212
(71) Applicant: Wuhan Humanwell Innovative Drug Research and Development Center Limited Company, Wuhan, Hubei 430075 (CN)
(72) Inventor: ZHANG, Xuejun, Wuhan, Hubei 430075 (CN); LI, Xueqiang, Wuhan, Hubei 430075 (CN); WANG, Hongqiang, Wuhan, Hubei 430075 (CN); YE, Dabing, Wuhan, Hubei 430075 (CN); WANG, Meng, Wuhan, Hubei 430075 (CN); AN, Dan, Wuhan, Hubei 430075 (CN); GAO, Zhenxing, Wuhan, Hubei 430075 (CN); ZHAO, Xin, Wuhan, Hubei 430075 (CN); LI, Li'e, Wuhan, Hubei 430075 (CN); YANG, Jun, Wuhan, Hubei 430075 (CN)
(74) Representative: Haseltine Lake Kempner LLP
(86) International application number: PCT/CN2022/132863
(87) International publication number: WO 2023/088425

(57) **Abstract**

Provided are a 15-PGDH inhibitor and the use thereof. The 15-PGDH inhibitor is a heterocyclic compound as represented by formula I, a solvate thereof, a pharmaceutically acceptable salt thereof, a solvate of a pharmaceutically acceptable salt thereof, or a prodrug thereof. The compound has a good inhibitory effect on 15-PGDH.

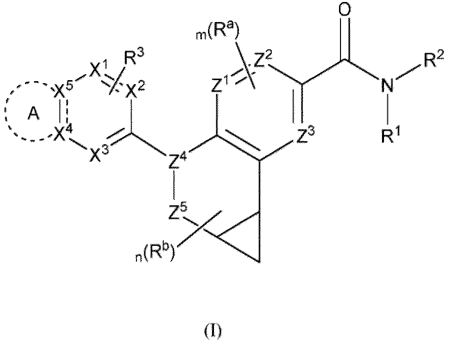

## Description

The present application claims the right of the priorities of Chinese patent application 202111372073X filed on November 18, 2021, Chinese patent application 2022108370158 filed on July 15, 2022, and Chinese patent application 2022114082124 filed on November 10, 2022. The contents of the above Chinese patent applications are incorporated herein by reference in their entireties.

### TECHNICAL FIELD

The present disclosure belongs to the field of medicine, specifically, the present disclosure relates to a 15-PGDH inhibitor and a use thereof.

### BACKGROUND

The 15-hydroxyprostaglandin dehydrogenase (15-PGDH) gene spans approximately 31 kb on the 4q34 to q35 of chromosome 4 and contains 7 exons with a molecular weight of 29 kD. 15-PGDH consists of 266 amino acids and belongs to the short-chain dehydrogenases (SDR) family, which is a dimer composed of two identical subunits, but some people believe that 15-PGDH only has enzymatic activity when 15-PGDH exists as a monomer. 15-PGDH is a key enzyme in the degradation and inactivation of prostaglandins (PGs) and related eicosane bioactive substances, which is widely found in normal tissues such as lungs, kidneys, gastrointestinal tracts, thyroids, prostates, and placentas in humans and mammals. On the one hand, 15-PGDH can catalyze the oxidation of active 15-hydroxyprostaglandin into 15-keto-prostaglandin with greatly reduced activity, and on the other hand, 15-PGDH can degrade a number of other non-prostaglandin polycyclic aromatic hydrocarbons in the presence of NAD⁺ coenzyme factors, reducing carcinogens and procarcinogens produced under physiological or pathological conditions through oxidative reactions.

There are currently no drugs on the market for the treatment of numerous conditions including fibrosis through the 15-PGDH inhibitory pathway. Therefore, the development of novel compounds that can inhibit the activity of 15-PGDH is of positive significance for the treatment of diseases.

### CONTENT OF THE PRESENT INVENTION

The technical problem to be solved by the present disclosure is to overcome the shortcomings of the prior art in that there are no drugs for the treatment of numerous conditions including fibrosis through the 15-PGDH inhibitory pathway, and therefore a 15-PGDH inhibitor and a use thereof is provided. The compounds of the present disclosure have good inhibitory effect on 15-PGDH.

The present disclosure solves the above technical problem by the following technical solutions.

The purpose of the present disclosure is to provide a novel compound for use as a 15-PGDH inhibitor.

A first aspect of the present disclosure provides a heterocyclic compound of formula I, a solvate, a pharmaceutically acceptable salt, a solvate of the pharmaceutically acceptable salt, or a prodrug thereof: 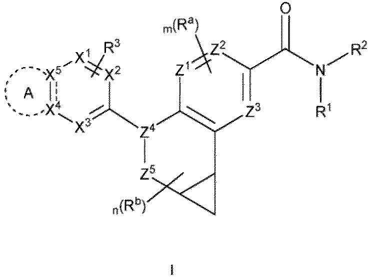 wherein
Z¹, Z², Z³, Z⁴, and Z⁵ each independently represent a ring atom;
Z¹, Z², Z³, Z⁴, and Z⁵ are each independently N, NH, O, S, CH₂, CH, or C;
R¹ and R² are each independently hydrogen, C₁-C₆ alkyl, or C₃-C₈ cycloalkyl,
or, R¹ and R² together with the N atom to which they are attached form a 3- to 11-membered heterocycloalkyl ring; wherein the 3- to 11-membered heterocycloalkyl ring is further substituted by 0, 1, or more than one (e.g., 2 or 3) R¹⁻¹ groups; when there is more than one substituent, the substituents are the same or different;
each R¹⁻¹ is independently halogen, hydroxyl, amino, nitro, cyano, carbonyl, oxo (=O), carboxyl, C₁-C₆ alkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups; R^{a} and R^{b} are each independently halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, or halo-C₁-C₆ alkoxy;
m and n are each independently 0, 1, 2, or 3;
X¹, X², X³, X⁴, and X⁵ each independently represent a ring atom;
X¹, X², X³, X⁴, and X⁵ are each independently N, O, S, CH₂, CH, or C;
the bond between X⁴ and X⁵ is a single bond or a double bond;
R³ is hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
in the group moiety 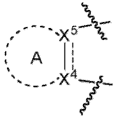 formed by the connection of X⁴ and X⁵, A is absent, or A together with the ring atoms X⁴ and X⁵ forms a 3- to 11-membered heterocycloalkyl ring, 5-membered heteroaromatic ring, or 6-membered heteroaromatic ring; the heteroatom is independently selected from one or more than one of N, O, and S;
when A is absent, X⁴ and X⁵ are each independently substituted by R⁴ or R⁵;
when A is present, the A is further substituted by R³⁻¹; the R³⁻¹ substitution is one or more than one substitution, and when there is more than one substituent, the substituents are the same or different;
R⁴ and R³⁻¹ are each independently hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
R⁵ is hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally substituted by 1 or 2 R⁵⁻¹ groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
each R⁵⁻¹ is independently hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups.

A second aspect of the present disclosure provides a compound of formula I', a solvate, a pharmaceutically acceptable salt, a solvate of the pharmaceutically acceptable salt, or a prodrug thereof: 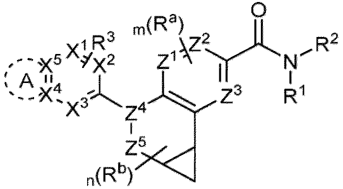 in formula I', Z¹, Z², Z³, Z⁴, and Z⁵ each independently represent a ring atom;
Z¹, Z², Z³, Z⁴, and Z⁵ are each independently N or C;
R¹ and R² are each independently hydrogen, C₁-C₆ alkyl, or C₃-C₈ cycloalkyl;
or, R¹ and R² together with the N atom to which they are attached form a 3- to 11-membered heterocycloalkyl ring; wherein the C₃-C₈ cycloalkyl or 3- to 11-membered heterocycloalkyl ring is further substituted by R¹⁻¹;
each R¹⁻¹ is independently halogen, hydroxyl, amino, nitro, cyano, carbonyl, oxo (=O), carboxyl, C₁-C₆ alkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
R^{a} and R^{b} are each independently halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, or halo-C₁-C₆ alkoxy;
m and n are each independently 0, 1, 2, or 3;
X¹, X², X³, X⁴, and X⁵ each independently represent a ring atom;
X¹, X², X³, X⁴, and X⁵ are each independently N or C;
each R³ is independently halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, or halo-C₁-C₆ alkoxy;
in the group moiety 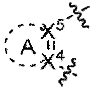 formed by the connection of X⁴ and X⁵, A is absent, or A together with the ring atoms X⁴ and X⁵ forms a 3- to 11-membered heterocycloalkyl ring, 5-membered heteroaromatic ring, or 6-membered heteroaromatic ring; the heteroatom is selected from one or more than one of N, O, and S;
when A is absent, X⁴ and X⁵ are each independently substituted by R⁴ or R⁵;
when A is present, the A is further substituted by R³⁻¹; the R³⁻¹ substitution is one or more than one substitution, and when there is more than one substituent, the substituents are the same or different;
R⁴, R⁵, and R³⁻¹ are each independently hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups.

In the present disclosure, the definitions of some substituents in the heterocyclic compound of formula I may be described as follows, and the definitions of unmentioned substituents are as described in any one of the above embodiments.

In a preferred embodiment of the present disclosure, the heteroatom in the 3- to 11-membered heterocycloalkyl ring, 5-membered heteroaromatic ring, and 6-membered heteroaromatic ring is one or more than one of N, O, or S, and the number of heteroatoms is 1, 2, 3, or 4.

In a preferred embodiment of the present disclosure, 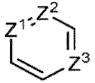 is phenyl or "a 6-membered heteroaromatic ring having 1, 2, or 3 heteroatoms selected from one or more than one of N, O, and S".

In a preferred embodiment of the present disclosure, 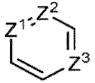 is phenyl or "a 6-membered heteroaromatic ring having 1, 2, or 3 heteroatoms selected from one or more than one of N, O, and S", such as a pyridine ring.

In a preferred embodiment of the present disclosure, 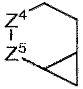 is "a 6-membered heteroalkyl ring substituted by cyclopropane having 1 heteroatom selected from N and O".

In a preferred embodiment of the present disclosure, 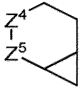 is "a 6-membered heteroalkyl ring substituted by cyclopropane having 1 heteroatom selected from N and O" or "a 6-membered heteroalkyl ring substituted by cyclopropane having 1 heteroatom selected from N", such as a pyridine ring.

In a preferred embodiment of the present disclosure, 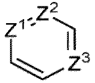 is

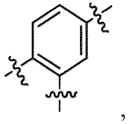

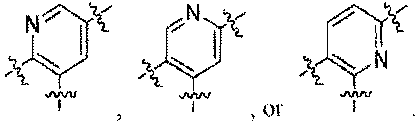

In a preferred embodiment of the present disclosure, the rings of 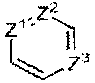 and 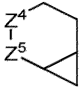 are connected to form 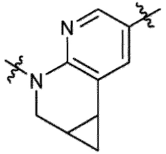 (*e.g.*, 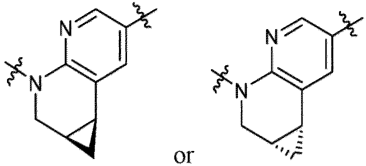 ) or 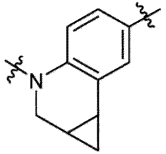 (*e.g.*, 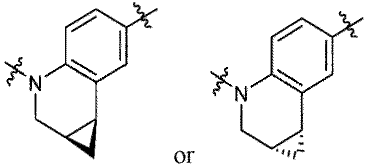 ).

In a preferred embodiment of the present disclosure, the heterocyclic compound of formula I has a structure of formula I-1 or formula I-2:

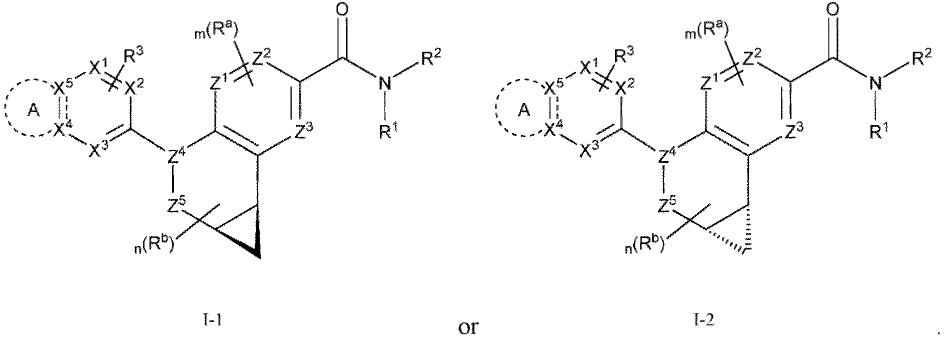

In a preferred embodiment of the present disclosure, the solvate is a hydrate.

In a preferred embodiment of the present disclosure, the R¹ and R² together with the N atom to which they are attached form a 3- to 11-membered heterocycloalkyl ring, in which the heterocycloalkyl ring is a monocyclic heterocycloalkyl ring, a bridged bicyclic heterocycloalkyl ring, or a spiro bicyclic heterocycloalkyl ring.

In a preferred embodiment of the present disclosure, the R¹ and R² together with the N atom to which they are attached form a 3- to 11-membered heterocycloalkyl ring, in which the heteroatom is one or more than one of N, O, or S, and the number of heteroatoms is 1, 2, 3, or 4.

In a preferred embodiment of the present disclosure, the R¹ and R² together with the N atom to which they are attached form a 3- to 11-membered heterocycloalkyl ring further substituted by one or more than one R¹⁻¹.

In a preferred embodiment of the present disclosure, the R¹ and R² together with the N atom to which they are attached form a 3- to 8-membered heterocycloalkyl ring.

Preferably, the 3- to 8-membered heterocycloalkyl ring is a monocyclic 3- to 8-membered heterocycloalkyl ring, a fused bicyclic 3- to 8-membered heterocycloalkyl ring, or a 3- to 8-membered heterocycloalkyl ring optionally comprising a bridged or spiro bicyclic ring.

Preferably, the 3- to 8-membered heterocycloalkyl ring further has 1 to 3 heteroatoms selected from one or more than one of N, O, and S.

Preferably, the 3- to 8-membered heterocycloalkyl ring is further substituted by halogen or halo-C₁-C₆ alkyl, or the 3- to 8-membered heterocycloalkyl ring is further substituted by halogen and/or halo-C₁-C₆ alkyl.

Further preferably, the 3- to 8-membered heterocycloalkyl ring is a monocyclic 3- to 6-membered heterocycloalkyl ring, a bridged bicyclic 6- to 8-membered heterocycloalkyl ring, or a spiro bicyclic 8-membered heterocycloalkyl ring, and the heterocycloalkyl ring has 1 or 2 heteroatoms being N and/or O, such as azetidinyl, pyrrolidinyl, piperidinyl, morpholinyl, pyrrolidinyl-fused cyclopropyl, oxazaspiro[2.5]octyl, azaspiro[2.5]octyl, or octahydrocyclopenta[c]pyrrolidinyl.

In a preferred embodiment of the present disclosure, the 3- to 8-membered heterocycloalkyl ring is further substituted by C₁-C₆ alkyl. Preferably, the substitutions are 0, 1, 2, or 3.

In a preferred embodiment of the present disclosure, in R¹⁻¹, the halogen and the halogen in the halo-C₁-C₆ alkyl are each independently F, Cl, or Br, such as F.

In a preferred embodiment of the present disclosure, in R¹⁻¹, the C₁-C₆ alkyl and the C₁-C₆ alkyl in the halo-C₁-C₆ alkyl are each independently methyl, ethyl, n-propyl, isopropyl, n-butyl, isobutyl, sec-butyl, or tert-butyl, such as methyl.

In a preferred embodiment of the present disclosure, in R¹⁻¹, the halo-C₁-C₆ alkyl is trifluoromethyl, difluoromethyl, or monofluoromethyl (*e.g.*, 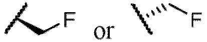 ).

In a preferred embodiment of the present disclosure, R¹ and R² together with the N atom to which they are attached form the following groups:

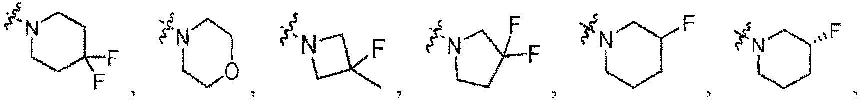

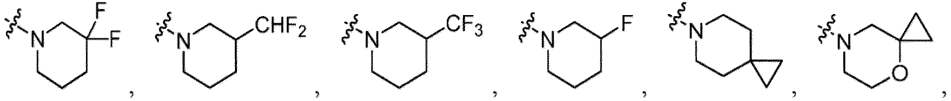

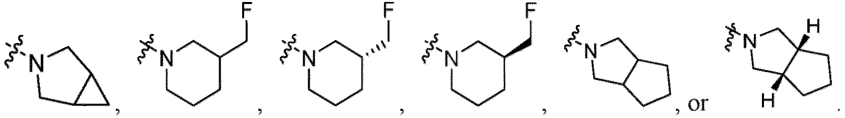

In a preferred embodiment of the present disclosure, R¹ and R² together with the N atom to which they are attached form the following groups:

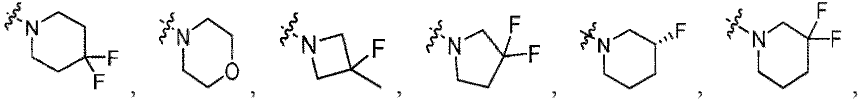

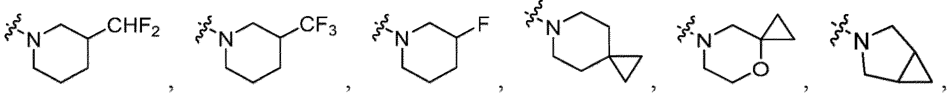

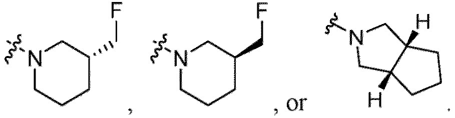

In a preferred embodiment of the present disclosure, in R⁴ and R⁵, each halogen is independently F, Cl, or Br, such as F.

In a preferred embodiment of the present disclosure, in R⁴ and R⁵, each C₁-C₆ alkyl in the aminocarbonyl optionally having 1 or 2 C₁-C₆ alkyl groups is independently methyl, ethyl, n-propyl, isopropyl, n-butyl, isobutyl, sec-butyl, or tert-butyl, such as methyl.

In a preferred embodiment of the present disclosure, in R⁴ and R⁵, each aminocarbonyl optionally having 1 or 2 C₁-C₆ alkyl groups is independently

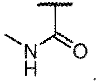

In a preferred embodiment of the present disclosure, the A together with the ring atoms X⁴ and X⁵ forms a 3- to 11-membered heterocycloalkyl ring, in which the heterocycloalkyl ring is a monocyclic cycloalkyl ring or a spiro bicyclic heterocycloalkyl ring.

In a preferred embodiment of the present disclosure, the A together with the ring atoms X⁴ and X⁵ forms a 3- to 11-membered heterocycloalkyl ring, in which the number of heteroatoms in the heterocycloalkyl ring is 1, 2, 3, or 4.

In a preferred embodiment of the present disclosure, the A together with the ring atoms X⁴ and X⁵ forms a 3- to 11-membered heterocycloalkyl ring, in which the 3- to 11-membered heterocycloalkyl ring is a 3- to 7-membered heterocycloalkyl ring.

In a preferred embodiment of the present disclosure, the A together with the ring atoms X⁴ and X⁵ forms a 3- to 11-membered heterocycloalkyl ring, in which the 3- to 11-membered heterocycloalkyl ring is a monocyclic 3- to 6-membered heterocycloalkyl ring or a spiro bicyclic 7-membered heterocycloalkyl ring, and the heterocycloalkyl ring has 1, 2, or 3 heteroatoms independently being N and/or O, such as pyrrolidinyl or azaspiro[2.4]heptyl.

In a preferred embodiment of the present disclosure, the A together with the ring atoms X⁴ and X⁵ forms a 5-membered heteroaromatic ring or 6-membered heteroaromatic ring, in which the number of heteroatoms is 1, 2, 3, or 4.

In a preferred embodiment of the present disclosure, the A together with the ring atoms X⁴ and X⁵ forms a 5-membered heteroaromatic ring or 6-membered heteroaromatic ring, and the heteroaromatic ring has 1, 2, or 3 heteroatoms being N, such as a pyridine ring, a pyrimidine ring, or a triazole ring.

In a preferred embodiment of the present disclosure, when A is present and the A is further substituted by R³⁻¹; the number of the R³⁻¹ is 2.

In a preferred embodiment of the present disclosure, in R³⁻¹, the halogen in the halo-C₁-C₆ alkyl is F, Cl, or Br, such as F.

In a preferred embodiment of the present disclosure, in R³⁻¹, the C₁-C₆ alkyl and the C₁-C₆ alkyl in the halo-C₁-C₆ alkyl and C₁-C₆ deuteroalkyl are each independently methyl, ethyl, *n*-propyl, isopropyl, *n*-butyl, isobutyl, *sec*-butyl, or *tert*-butyl, such as methyl or ethyl.

In a preferred embodiment of the present disclosure, in R³⁻¹, the halo-C₁-C₆ alkyl is

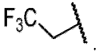

In a preferred embodiment of the present disclosure, in R³⁻¹, the C₁-C₆ deuteroalkyl is -CD₃.

In a preferred embodiment of the present disclosure, in R³⁻¹, the cycloalkyl in the C₃-C₈ cycloalkyl is monocyclic cycloalkyl.

In a preferred embodiment of the present disclosure, in R³⁻¹, the cycloalkyl in the C₃-C₈ cycloalkyl is cyclopropyl, cyclobutyl, cyclopentyl, or cyclohexyl.

In a preferred embodiment of the present disclosure, 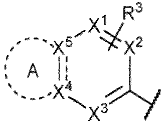 is

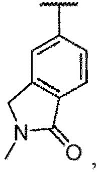

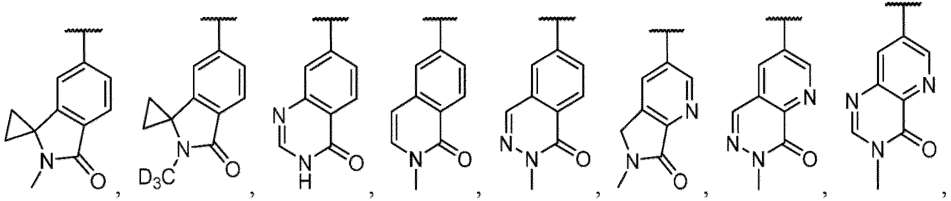

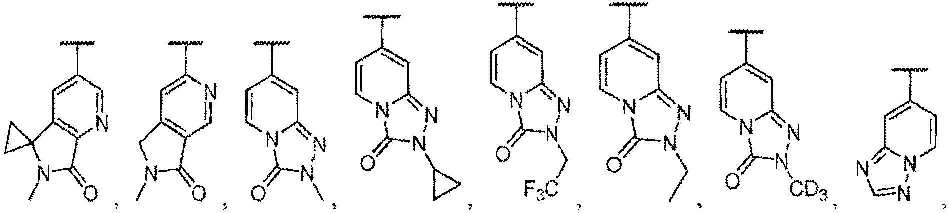

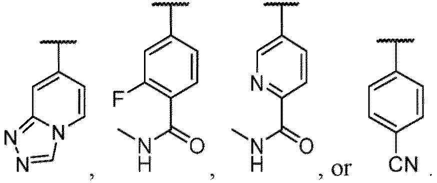

In a preferred embodiment of the present disclosure, Z¹, Z², and Z³ are each independently N, CH, or C.

In a preferred embodiment of the present disclosure, Z⁴ and Z⁵ are each independently N, NH, CH₂, or CH.

In a preferred embodiment of the present disclosure, R¹ and R² together with the N atom to which they are attached form a 3- to 11-membered heterocycloalkyl ring; wherein the 3- to 11-membered heterocycloalkyl ring is further substituted by 0, 1, or more than one R¹⁻¹.

In a preferred embodiment of the present disclosure, R¹⁻¹ is halogen, C₁-C₆ alkyl, or halo-C₁-C₆ alkyl.

In a preferred embodiment of the present disclosure, R⁴ and R⁵ are independently halogen, cyano, or aminocarbonyl optionally having 1 or 2 C₁-C₆ alkyl groups.

In a preferred embodiment of the present disclosure, R³⁻¹ is independently oxo (=O), C₁-C₆ alkyl, halo-C₁-C₆ alkyl, C₃-C₈ cycloalkyl, or C₁-C₆ deuteroalkyl.

In a preferred embodiment of the present disclosure, in the heterocyclic compound of formula I:
Z¹, Z², and Z³ are each independently N or C;
Z⁴ and Z⁵ are each independently N, NH, CH₂, or CH;
R¹ and R² together with the N atom to which they are attached form a 3- to 11-membered heterocycloalkyl ring; wherein the 3- to 11-membered heterocycloalkyl ring is further substituted by 0, 1, or more than one R¹⁻¹;
R¹⁻¹ is halogen, C₁-C₆ alkyl, or halo-C₁-C₆ alkyl;
m and n are 0;
R³ is hydrogen;
R⁴ and R⁵ are independently halogen, cyano, or aminocarbonyl optionally having 1 or 2 C₁-C₆ alkyl groups;
R³⁻¹ is independently oxo (=O), C₁-C₆ alkyl, halo-C₁-C₆ alkyl, C₃-C₈ cycloalkyl, or C₁-C₆ deuteroalkyl;
the group moiety 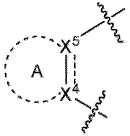 formed by the connection of X⁴ and X⁵ is as defined above.

It can be understood by those skilled in the art that, according to the conventions used in the art, in the structural formulas of the present disclosure, 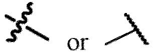 are used to depict a chemical bond, wherein the chemical bond is a point at which a moiety or a substituent is attached to a core structure or a backbone structure.

In a preferred embodiment of the present disclosure, the heterocyclic compound of formula I is selected from the following structure: 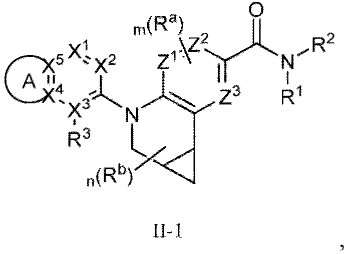 wherein X⁴ and X⁵ each independently represent a ring atom; X⁴ and X⁵ are each independently N, CH, or C; A together with X⁴ and X⁵ forms a 3- to 8-membered heterocycloalkyl ring, and the 3- to 8-membered heterocycloalkyl ring is further substituted by R³⁻¹; the R³⁻¹ substitution is one or more than one substitution, and when there is more than one substituent, the substituents are the same or different; each R³⁻¹ is independently hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups; Z¹, Z², Z³, X¹, X², and X³ are as defined in the first aspect of the present disclosure; R¹, R², R³, R^{a}, R^{b}, m, and n are as defined in the first aspect of the present disclosure.

In a preferred embodiment of the present disclosure, the heterocyclic compound of formula I is selected from the following structure: 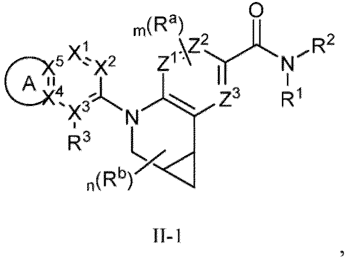 wherein X⁴ and X⁵ each independently represent a ring atom; X⁴ and X⁵ are each independently N or C; A together with X⁴ and X⁵ forms a 3- to 8-membered heterocycloalkyl ring, and the 3- to 8-membered heterocycloalkyl ring is further substituted by R³⁻¹; the R³⁻¹ substitution is one or more than one substitution, and when there is more than one substituent, the substituents are the same or different; each R³⁻¹ is independently hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups; Z¹, Z², Z³, X¹, X², and X³ are as defined in the first aspect of the present disclosure; R¹, R², R³, R^{a}, R^{b}, m, and n are as defined in the first aspect of the present disclosure.

In a preferred embodiment of the present disclosure, the heterocyclic compound of formula I is selected from the following structure: 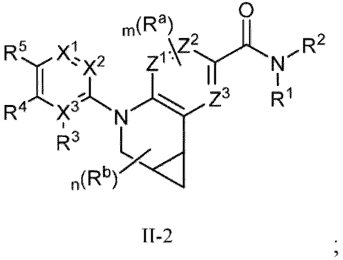 wherein R³, R⁴, and R⁵ are each independently hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups; Z¹, Z², Z³, X¹, X², and X³ are as defined in the first aspect of the present disclosure; R¹, R², R^{a}, R^{b}, m, and n are as defined in the first aspect of the present disclosure.

In a preferred embodiment of the present disclosure, the heterocyclic compound of formula I is selected from the following structure: 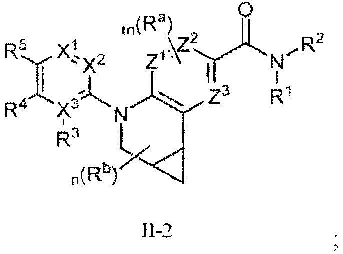 wherein R³ is halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, or halo-C₁-C₆ alkoxy; R⁴ and R⁵ are each independently hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups; Z¹, Z², Z³, X¹, X², and X³ are as defined in the first aspect of the present disclosure; R¹, R², R^{a}, R^{b}, m, and n are as defined in the first aspect of the present disclosure.

In a preferred embodiment of the present disclosure, the heterocyclic compound of formula I is selected from the following structure: 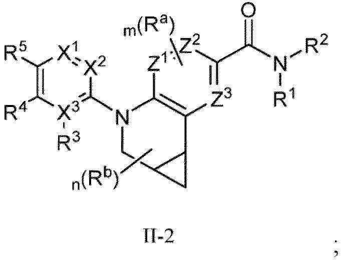 wherein R³ is H, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, or halo-C₁-C₆ alkoxy;
R⁴ and R⁵ are each independently hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
Z¹, Z², Z³, X¹, X², X³, R¹, R², R^{a}, R^{b}, m, and n are as defined in the first aspect of the present disclosure.

In a preferred embodiment of the present disclosure, the heterocyclic compound of formula I is selected from the following structure: 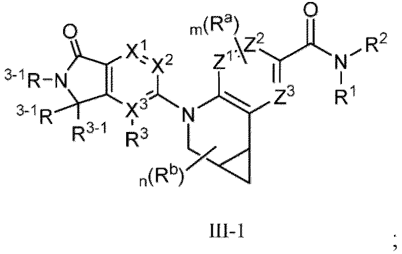 wherein Z¹, Z², Z³, X¹, X², X³, R¹, R², R³, R³⁻¹, R^{a}, R^{b}, m, and n are as defined in the first aspect of the present disclosure.

In a preferred embodiment of the present disclosure, the heterocyclic compound of formula I is selected from the following structure: 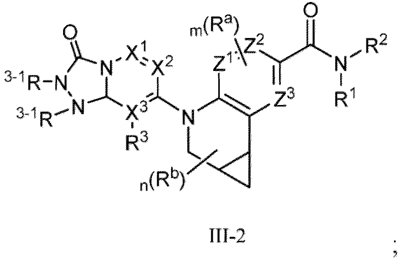 wherein Z¹, Z², Z³, X¹, X², X³, R¹, R², R³, R³⁻¹, R^{a}, R^{b}, m, and n are as defined in the first aspect of the present disclosure.

In a preferred embodiment of the present disclosure, the heterocyclic compound of formula I is selected from the following structure: 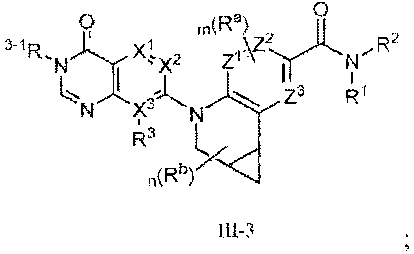 wherein Z¹, Z², Z³, X¹, X², X³, R¹, R², R³, R³⁻¹, R^{a}, R^{b}, m, and n are as defined in the first aspect of the present disclosure.

In a preferred embodiment of the present disclosure, the heterocyclic compound of formula I is selected from the following structure: 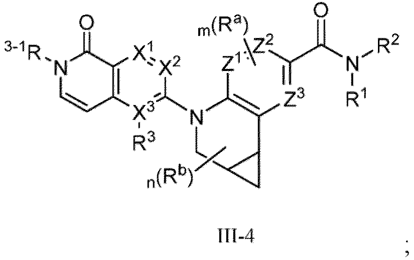 wherein Z¹, Z², Z³, X¹, X², X³, R¹, R², R³, R³⁻¹, R^{a}, R^{b}, m, and n are as defined in the first aspect of the present disclosure.

In a preferred embodiment of the present disclosure, the heterocyclic compound of formula I is selected from the following structure: 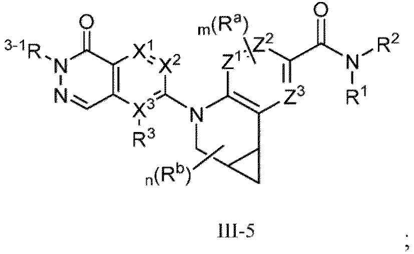 wherein Z¹, Z², Z³, X¹, X², X³, R¹, R², R³, R³⁻¹, R^{a}, R^{b}, m, and n are as defined in the first aspect of the present disclosure.

In a preferred embodiment of the present disclosure, the heterocyclic compound of formula I is selected from the following structure: 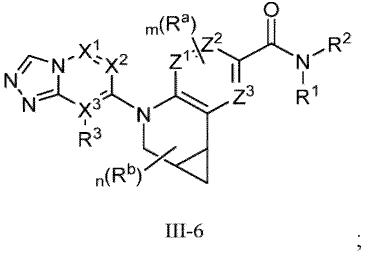 wherein Z¹, Z², Z³, X¹, X², X³, R¹, R², R³, R³⁻¹, R^{a}, R^{b}, m, and n are as defined in the first aspect of the present disclosure.

In a preferred embodiment of the present disclosure, the heterocyclic compound of formula I is selected from the following structure: 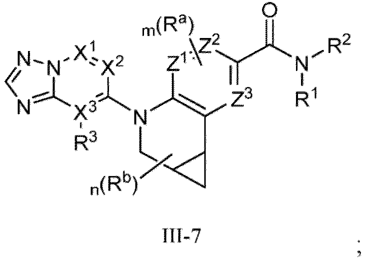 wherein Z¹, Z², Z³, X¹, X², X³, R¹, R², R³, R³⁻¹, R^{a}, R^{b}, m, and n are as defined in the first aspect of the present disclosure.

In a preferred embodiment of the present disclosure, the heterocyclic compound of formula I is selected from the following structure: 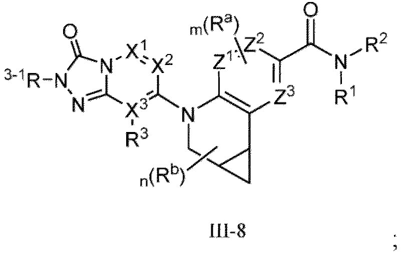 wherein Z¹, Z², Z³, X¹, X², X³, R¹, R², R³, R³⁻¹, R^{a}, R^{b}, m, and n are as defined in the first aspect of the present disclosure.

In a preferred embodiment of the present disclosure, the heterocyclic compound of formula I is selected from the following structure: 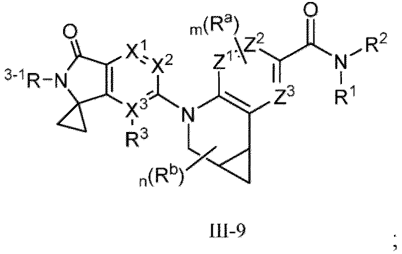 wherein Z¹, Z², Z³, X¹, X², X³, R¹, R², R³, R³⁻¹, R^{a}, R^{b}, m, and n are as defined in the first aspect of the present disclosure.

In a preferred embodiment of the present disclosure, the heterocyclic compound of formula I is selected from the following structure: 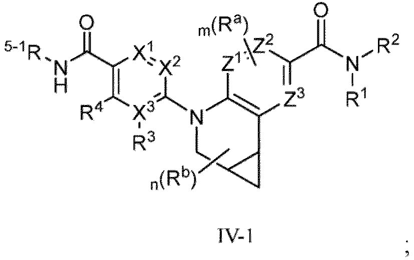 wherein R⁵⁻¹ is hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups; Z¹, Z², Z³, X¹, X², and X³ are as defined in the first aspect of the present disclosure; R¹, R², R³, R^{a}, R^{b}, m, and n are as defined in the first aspect of the present disclosure.

In a preferred embodiment of the present disclosure, the heterocyclic compound of formula I is selected from the following structure: 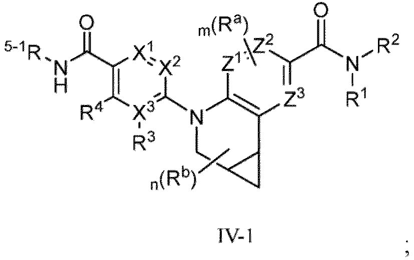 wherein R⁵⁻¹ is hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, C₁-C₆ alkylsulfonylamino, or amino optionally having 1 or 2 C₁-C₆ alkyl groups; Z¹, Z², Z³, X¹, X², and X³ are as defined in the first aspect of the present disclosure; R¹, R², R³, R^{a}, R^{b}, m, and n are as defined in the first aspect of the present disclosure.

In a preferred embodiment of the present disclosure, the heterocyclic compound of formula I is selected from the following structure: 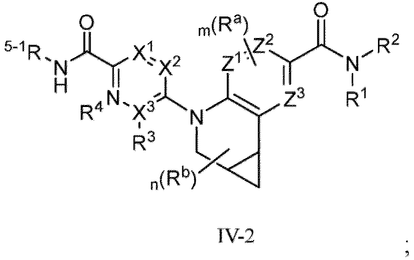 wherein R⁵⁻¹ is hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups; Z¹, Z², Z³, X¹, X², and X³ are as defined in the first aspect of the present disclosure; R¹, R², R³, R^{a}, R^{b}, m, and n are as defined in the first aspect of the present disclosure; R⁴ is absent.

In a preferred embodiment of the present disclosure, the heterocyclic compound of formula I is selected from the following structure: 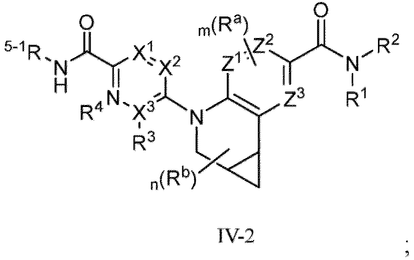 wherein R⁵⁻¹ is hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups; Z¹, Z², Z³, X¹, X², and X³ are as defined in the first aspect of the present disclosure; R¹, R², R³, R^{a}, R^{b}, m, and n are as defined in the first aspect of the present disclosure; R⁴ is absent.

In a preferred embodiment of the present disclosure, the heterocyclic compound of formula I is selected from any one of the following compounds:

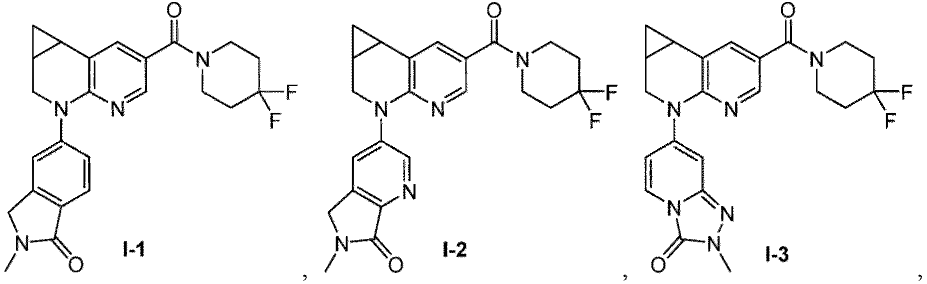

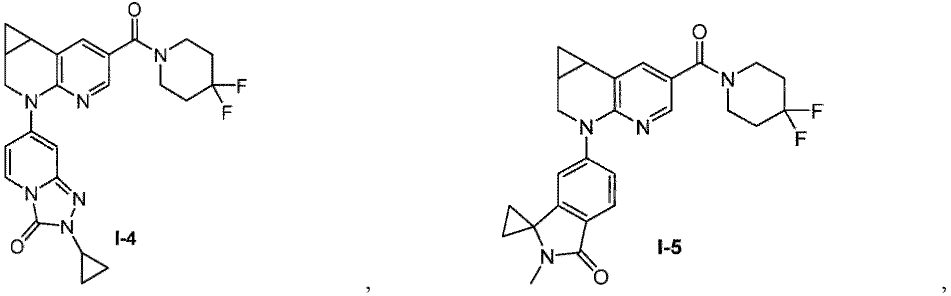

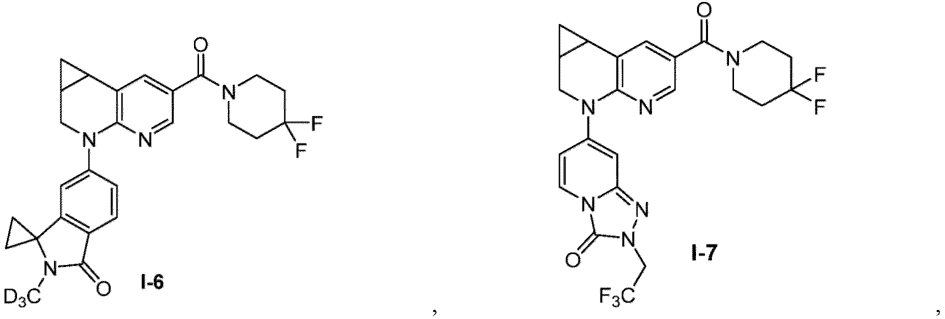

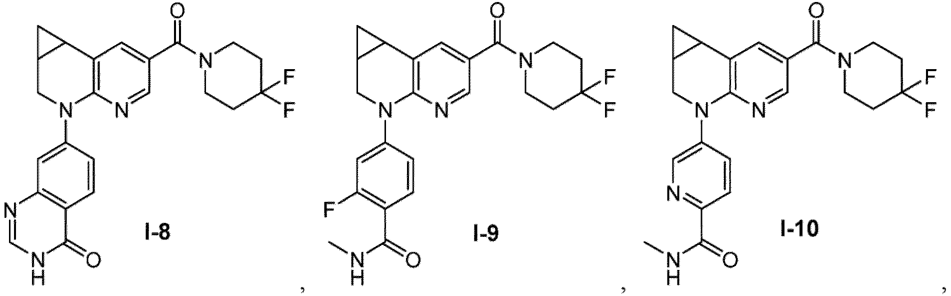

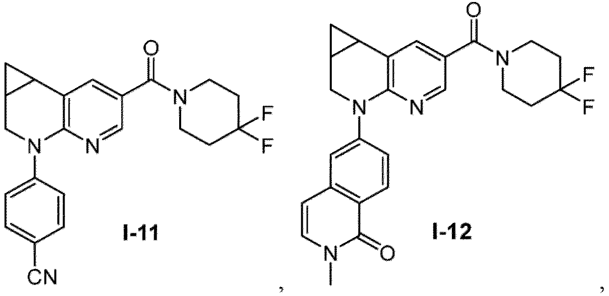

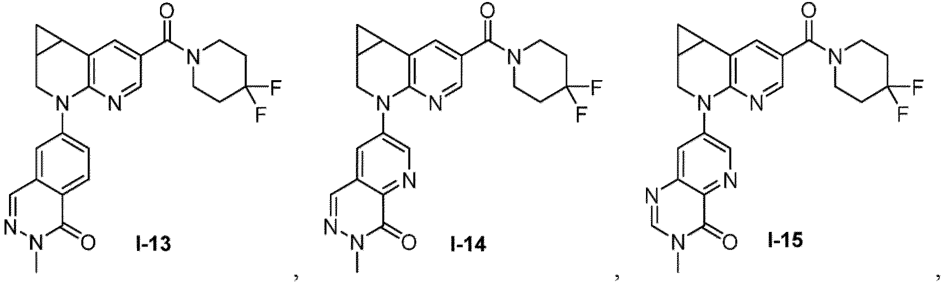

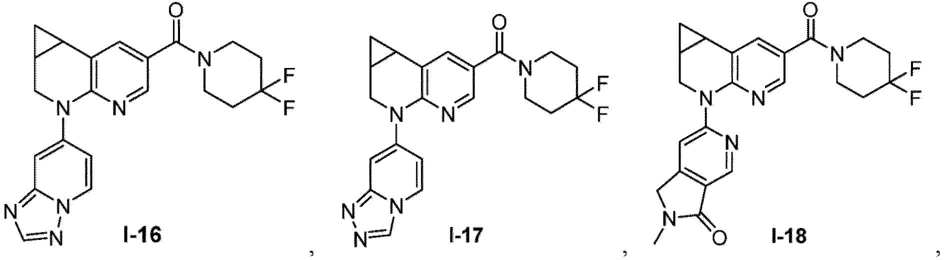

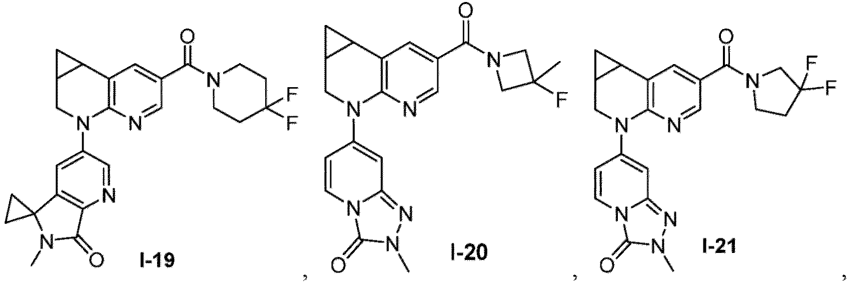

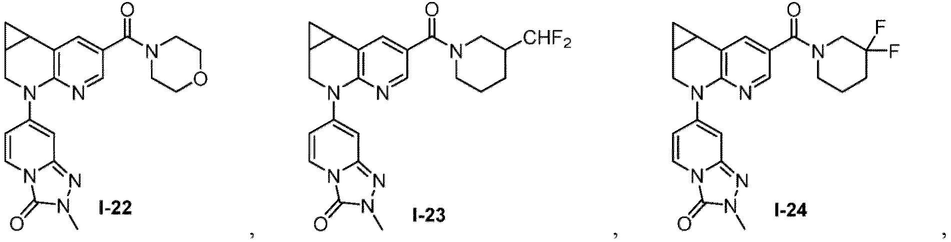

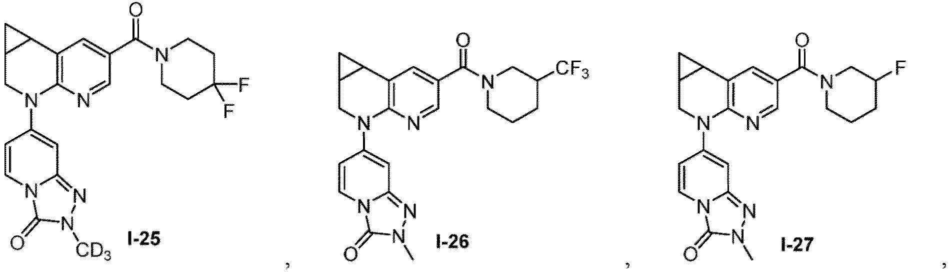

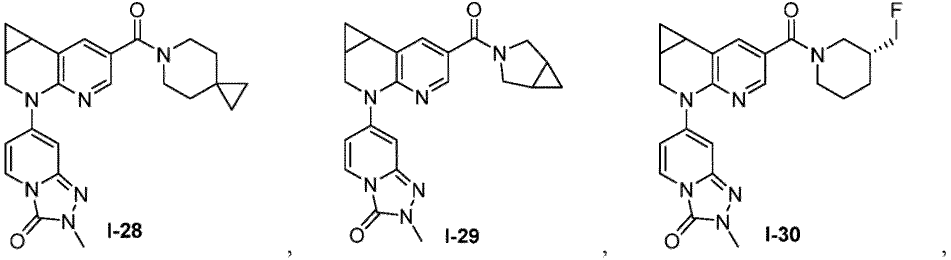

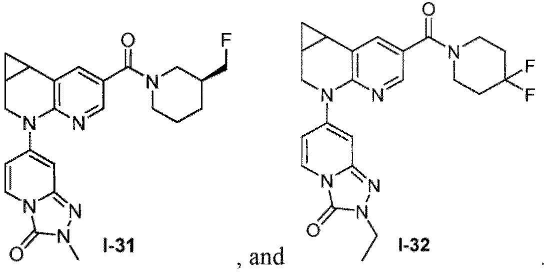

In a preferred embodiment of the present disclosure, the heterocyclic compound of formula I is selected from any one of the following compounds:

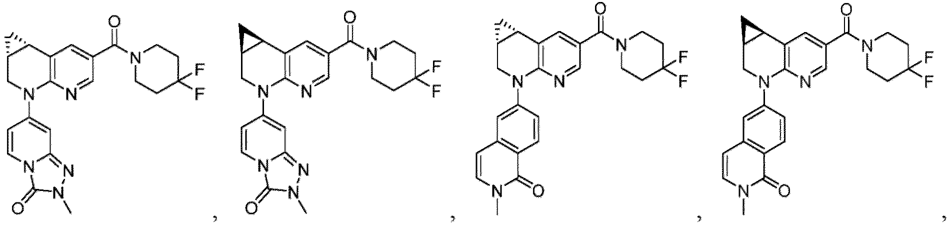

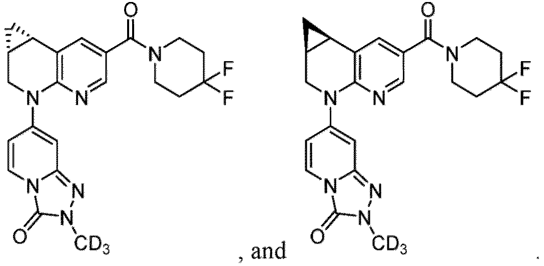

In a preferred embodiment of the present disclosure, the heterocyclic compound of formula I is selected from any one of the following compounds: 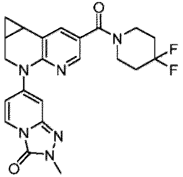
with a retention time of 0.743 min under the following SFC conditions:
chromatographic column: Chiralpak AD-3 50 × 4.6 mm I.D., 3 µm, mobile phase: mobile phase A: CO₂, mobile phase B: a solution of isopropanol and acetonitrile containing 0.05 vol% diethylamine;
isocratic elution: 50 vol% solution of isopropanol and acetonitrile containing 0.05 vol% diethylamine in CO₂, flow rate: 3 mL/min; detector: PDA, column temperature: 35°C; column pressure: 100 Bar; 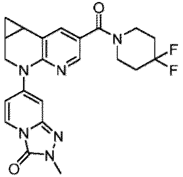
with a retention time of 1.670 min under the following SFC conditions:
chromatographic column: Chiralpak AD-3 50 × 4.6 mm I.D., 3 µm, mobile phase: mobile phase A: CO₂, mobile phase B: a solution of isopropanol and acetonitrile containing 0.05 vol% diethylamine;
isocratic elution: 50 vol% solution of isopropanol and acetonitrile containing 0.05 vol% diethylamine in CO₂, flow rate: 3 mL/min; detector: PDA, column temperature: 35°C; column pressure: 100 Bar; 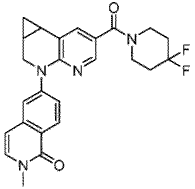
with a retention time of 0.816 min under the following SFC conditions:
chromatographic column: Chiralpak AS-3 50 × 4.6 mm I.D., 3 µm; mobile phase: mobile phase A: CO₂, mobile phase B: a solution of isopropanol and acetonitrile containing 0.05 vol% diethylamine; isocratic elution: 40 vol% solution of isopropanol and acetonitrile containing 0.05 vol% diethylamine in CO₂; flow rate: 3 mL/min; detector: PDA, column temperature: 35°C; column pressure: 100 Bar; 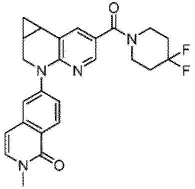
with a retention time of 1.477 min under the following SFC conditions:
chromatographic column: Chiralpak AS-3 50 × 4.6 mm I.D., 3 µm; mobile phase: mobile phase A: CO₂, mobile phase B: a solution of isopropanol and acetonitrile containing 0.05 vol% diethylamine; isocratic elution: 40 vol% solution of isopropanol and acetonitrile containing 0.05 vol% diethylamine in CO₂; flow rate: 3 mL/min; detector: PDA, column temperature: 35°C; column pressure: 100 Bar; 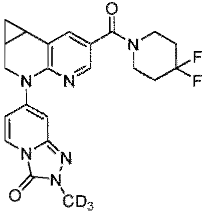
with a retention time of 0.764 min under the following SFC conditions:
chromatographic column: Chiralpak AD-3 50 × 4.6 mm I.D., 3 µm, mobile phase: mobile phase A: CO₂, mobile phase B: a solution of isopropanol and acetonitrile containing 0.05 vol% diethylamine; isocratic elution: 50 vol% solution of isopropanol and acetonitrile containing 0.05 vol% diethylamine in CO₂, flow rate: 3 mL/min; detector: PDA, column temperature: 35°C; column pressure: 100 Bar;
and 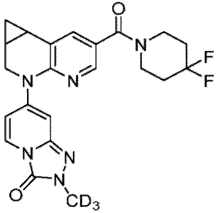 with a retention time of 1.702 min under the following SFC conditions:
chromatographic column: Chiralpak AD-3 50 × 4.6 mm I.D., 3 µm, mobile phase: mobile phase A: CO₂, mobile phase B: a solution of isopropanol and acetonitrile containing 0.05 vol% diethylamine; isocratic elution: 50 vol% solution of isopropanol and acetonitrile containing 0.05 vol% diethylamine in CO₂, flow rate: 3 mL/min; detector: PDA, column temperature: 35°C; column pressure: 100 Bar.

In a preferred embodiment of the present disclosure, the heterocyclic compound of formula I is selected from any one of the following compounds: 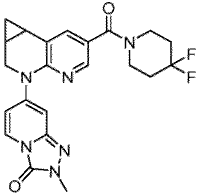
with an IC₅₀ of 12.16 nM under the conditions of test example 1; 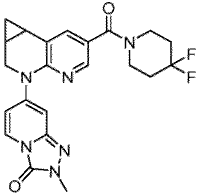
with an IC₅₀ of 3.58 nM under the conditions of test example 1; 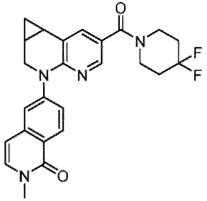
with an IC₅₀ of 1.52 nM under the conditions of test example 1;
and 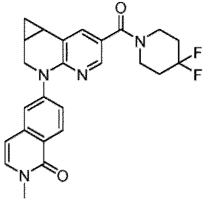 with an IC₅₀ of 7.42 nM under the conditions of test example 1. 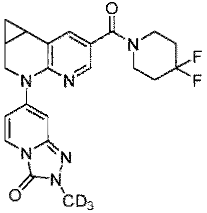
with an IC₅₀ of 15.8 nM under the conditions of test example 1;
and 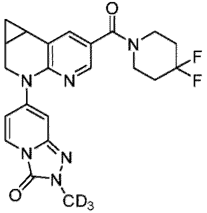 with an IC₅₀ of 3.78 nM under the conditions of test example 1.

In a preferred embodiment of the present disclosure, the heterocyclic compound of formula I is selected from any one of the following compounds: 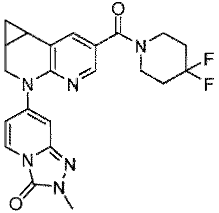
with an increased fold of 0.9 under the conditions of test example 2; 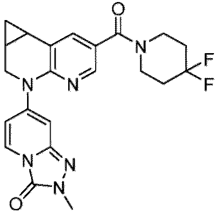
with an increased fold of 4.8 under the conditions of test example 2; 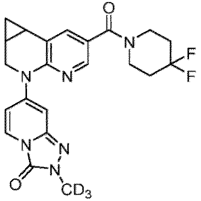
with an increased fold of 0.9 under the conditions of test example 2;
and 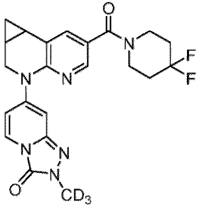 with an increased fold of 4.4 under the conditions of test example 2.

In a preferred embodiment of the present disclosure, the 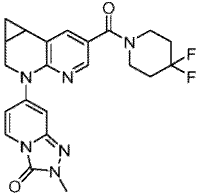 is 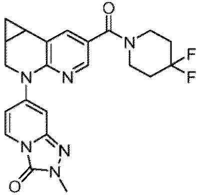 with a total pulmonary fibrosis score of 3.34 under the conditions of test example 3.

In a preferred embodiment of the present disclosure, the 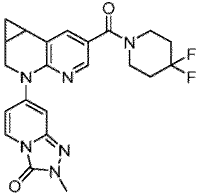 is 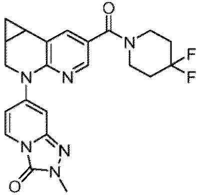 with a Cₘₐₓ of 4480 ng/mL under the conditions of test example 5.

In a preferred embodiment of the present disclosure, the 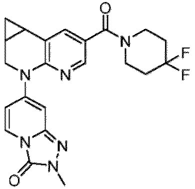 is 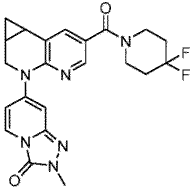 with an AUC₍₀₋ₜ₎ of 33173 h·ng/mL under the conditions of test example 5.

A third aspect of the present disclosure provides a pharmaceutical composition, comprising: the compound of formula I, the solvate, the pharmaceutically acceptable salt, the solvate of the pharmaceutically acceptable salt, or the prodrug thereof according to the first aspect of the present disclosure; and a pharmaceutically acceptable carrier.

A fourth aspect of the present disclosure provides a use of the compound of formula I, the solvate, the pharmaceutically acceptable salt, the solvate of the pharmaceutically acceptable salt, or the prodrug thereof according to the first aspect of the present disclosure, or a use of the pharmaceutical composition according to the fifth aspect of the present disclosure, comprising: inhibiting 15-PGDH; and/or, preventing and/or treating a disease related to 15-PGDH; and/or, acting as a 15-PGDH inhibitor; and/or, preparing a drug, pharmaceutical composition, or formulation for preventing and/or treating the disease related to 15-PGDH.

Preferably, the disease related to 15-PGDH includes, but is not limited to, one, two, or more of fibrotic disease, inflammatory disease, cardiovascular disease, trauma, autoimmune disease, graft-versus-host disease, hair growth, osteoporosis, ear disease, eye disease, neutropenia, diabetes, underactive bladder, implant promotion in stem cell or bone marrow transplantation or organ transplantation, neurogenesis and neuronal cell death, hematopoietic reconstruction, tissue injury, cervical disease, and kidney disease.

Preferably, the disease related to 15-PGDH includes, but is not limited to, one, two, or more of fibrotic disease (e.g., pulmonary fibrosis, including idiopathic pulmonary fibrosis, liver fibrosis, renal fibrosis, myocardial fibrosis, scleroderma, and myelofibrosis), inflammatory disease (e.g., chronic obstructive pulmonary disease (COPD), acute lung injury, sepsis, exacerbation of asthma and pulmonary disease, inflammatory bowel disease (IBD) (e.g., ulcerative colitis and Crohn's disease), peptic ulcer (e.g., NSAID-induced ulcer), autoinflammatory disease (e.g., Behcet's disease), vasculitis syndrome, acute liver injury, acute kidney injury, non-alcoholic fatty liver disease (NASH), atopic dermatitis, psoriasis, interstitial cystitis, prostatitis syndrome (e.g., chronic prostatitis/chronic pelvic pain syndrome)), cardiovascular disease (e.g., pulmonary hypertension, angina, myocardial infarction, heart failure, ischemic heart disease, stroke, and peripheral circulatory disorder), kidney disease (e.g., chronic kidney disease and renal failure), trauma (e.g., diabetic ulcer, burn, pressure ulcer, acute mucosal injury, including Stevens-Johnson syndrome, mucosal injury (e.g., mucositis or stomatitis), injury related to anticancer chemotherapeutic agents (particularly alkylating agents, DNA synthesis inhibitors, or DNA gyrase inhibitors) or injury related to antimetabolites, cellular or humoral immunotherapy or radiation), autoimmune disease (e.g., multiple sclerosis or rheumatoid arthritis), graft-versus-host disease (GVHD), hair growth, osteoporosis, ear disease (e.g., hearing loss, tinnitus, vertigo, and balance disorder), eye disease (e.g., glaucoma and dry eye), neutropenia, diabetes, underactive bladder, implant promotion in stem cell or bone marrow transplantation or organ transplantation, neurogenesis and neuronal cell death (e.g., neuropsychiatric disorder, neuropathy, neurotoxic disease, neuropathic pain, and neurodegenerative disease), liver regeneration, muscle regeneration (e.g., muscle atrophy, muscular dystrophy, and muscle injury), and cervical disease.

Preferably, the tissue injury is liver injury and/or muscle injury (e.g., muscle atrophy and muscular dystrophy).

Preferably, the disease related to 15-PGDH includes, but is not limited to, idiopathic pulmonary fibrosis (IPF).

Preferably, the prevention and/or treatment of the disease related to 15-PGDH includes, but is not limited to, liver regeneration.

Preferably, the disease related to 15-PGDH includes, but is not limited to, liver injury.

Preferably, the disease related to 15-PGDH includes, but is not limited to, IBD.

A fifth aspect of the present disclosure provides a use of the compound of formula I, the solvate, the pharmaceutically acceptable salt, the solvate of the pharmaceutically acceptable salt, or the prodrug thereof in the preparation of a drug for preventing or treating a disease as follows; the disease is one or more than one of fibrotic disease, inflammatory disease, or tissue injury.

The fibrotic disease, the inflammatory disease, and the tissue injury may be as described above.

A sixth aspect of the present disclosure provides a method for inhibiting 15-PGDH, or preventing and/or treating a disease related to 15-PGDH, comprising the steps of: administering to a subject in need the compound of formula I, the solvate, the pharmaceutically acceptable salt, the solvate of the pharmaceutically acceptable salt, or the prodrug thereof according to the first aspect of the present disclosure.

The disease related to 15-PGDH is as described above.

A seventh aspect of the present disclosure provides a method for preventing or treating a disease, comprising the steps of: administering to a subject in need the compound of formula I, the solvate, the pharmaceutically acceptable salt, the solvate of the pharmaceutically acceptable salt, or the prodrug thereof according to the first aspect of the present disclosure; the disease is one or more than one of fibrotic disease, inflammatory disease, or tissue injury.

The fibrotic disease and the inflammatory disease may be as described above.

The additional aspects and advantages of the present disclosure will be partly given in the following description, and part of them will become apparent from the following description, or can be understood through the implementation of the present disclosure.

### Term definitions and explanations

Unless otherwise specified, the definitions of groups and terms described in the description and claims include definitions thereof as examples, exemplary definitions, preferred definitions, definitions recorded in tables, and definitions of specific compounds in the examples, *etc.,* which can be arbitrarily combined and integrated with each other. Such combined and integrated definitions of groups and compound structures should fall within the scope of the description of the present disclosure.

Unless otherwise defined, all scientific and technological terms of the present disclosure have the same meanings as those commonly understood by those skilled in the art to which the subject matter of the claims belongs. Unless otherwise specified, all patents, patent applications, and published materials cited in the present disclosure are incorporated herein by reference in their entirety. If a term has multiple definitions in the present disclosure, the definitions in this section shall prevail.

It should be understood that the above brief description and the following detailed description are exemplary and for explanatory purposes only, and do not limit the subject matter of the present disclosure in any way. In the present disclosure, the use of the singular also includes the plural unless specifically stated otherwise. It must be noted that the singular form used in the present description and claims includes the plural form of the object referred to unless clearly stated otherwise. It should also be noted that the use of "or" and "alternatively" indicates "and/or" unless otherwise specified. Furthermore, the use of the term "comprising" as well as other forms, such as "comprises", "includes", and "contains", is not limiting.

Definitions of standard chemical terms can be found in references (including Carey and Sundberg "ADVANCED ORGANIC CHEMISTRY 4THED." Vols. A (2000) and B (2001), Plenum Press, New York). Unless otherwise specified, conventional methods in the technical scope of the art, such as mass spectrometry, NMR, IR, and UV/Vis spectroscopy, and pharmacological methods are used. Unless specific definitions are provided, the terms used herein in the relevant descriptions of analytical chemistry, synthetic organic chemistry, and pharmaceuticals and medicinal chemistry are known in the art. Standard techniques can be used in chemical synthesis, chemical analysis, drug preparation, formulation, and delivery, and treatment of patients. For example, reaction and purification can be carried out according to the manufacturer's instructions for use of the kit, or in a manner known in the art, or the description of the present disclosure. Generally, the above techniques and methods can be implemented according to the descriptions in a number of summary and more specific documents cited and discussed in the present disclosure according to conventional methods well-known in the art. In the present description, groups and substituents thereof can be selected by those skilled in the art to provide stable structural moieties and compounds.

When a substituent is described by a conventional chemical formula written from left to right, the substituent also includes the chemically equivalent substituent obtained when the structural formula is written from right to left. For example, CH₂O is equivalent to OCH₂. As used herein, 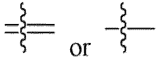 indicates the connection site of the group. As used herein, "R₁", "R1", and "R¹" have the same meaning and can be interchanged. For other symbols such as R², similar definitions have the same meaning.

The section headings used herein are for the purpose of organizing the article only and should not be construed as limiting the subject matter described. All documents, or portions of documents, cited in the present disclosure, including but not limited to patents, patent applications, articles, books, manuals, and treatises, are incorporated herein by reference in their entirety.

In addition to the foregoing, when used in the description and claims of the present disclosure, the following terms have the meanings shown below unless otherwise specified.

When the numerical range described in the description and claims of the present disclosure is understood as "integers", it should be understood as recording the two endpoints of the range and all integers in the range. For example, an "integer from 1 to 6" should be understood as recording every integer of 0, 1, 2, 3, 4, 5, and 6. When the numerical range is understood as "numbers", it should be understood as recording the two endpoints of the range as well as all integers in the range and all decimals in the range. For example, a "number from 1 to 10" should be understood as recording not only every integer of 1, 2, 3, 4, 5, 6, 7, 8, 9, and 10, but also at least the sum of each of these integers with 0.1, 0.2, 0.3, 0.4, 0.5, 0.6, 0.7, 0.8, and 0.9, respectively; for example, 1 to 3 should be understood as 1, 2, and 3.

In the present disclosure, the term "halogen" alone or as part of another substituent refers to fluorine, chlorine, bromine, or iodine; preferably fluorine or chlorine.

The term "alkyl" alone or as part of another substituent refers to a linear or branched hydrocarbon chain group consisting only of carbon atoms and hydrogen atoms, free of unsaturated bonds, having, for example, 1 to 6 carbon atoms, and connected to the rest of the molecule by a single bond. Examples of alkyl include, but are not limited to, methyl, ethyl, n-propyl, isopropyl, n-butyl, isobutyl, *tert-butyl,* pentyl, isopentyl, neopentyl, and hexyl. As used herein, the term "alkenyl" refers to an unbranched or branched monovalent hydrocarbon chain that contains one or more than one carbon-carbon double bond. As used herein, the term "alkynyl" refers to an unbranched or branched monovalent hydrocarbon chain that contains one or more than one carbon-carbon triple bond.

The term "C₁-C₆ alkyl", alone or as part of another substituent, should be understood to mean a linear or branched saturated monovalent hydrocarbon group having 1, 2, 3, 4, 5 or 6 carbon atoms. The alkyl is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof. In particular, the group has 1, 2, or 3 carbon atoms ("C₁-C₃ alkyl"), such as methyl, ethyl, n-propyl, or isopropyl.

The term "cycloalkyl" alone or as part of another substituent refers to a cyclic alkyl group. The term "m- to n-membered cycloalkyl" or "Cₘ-Cₙ cycloalkyl" should be understood to mean a saturated carbocyclic ring having m to n atoms. For example, "3- to 15-membered cycloalkyl" or "C₃-C₁₅ cycloalkyl" refers to a cyclic alkyl group containing 3 to 15, 3 to 9, 3 to 6, or 3 to 5 carbon atoms, which may contain 1 to 4 rings. "3- to 10-membered cycloalkyl" contains 3 to 10 carbon atoms. It includes a monocyclic, bicyclic, tricyclic, spiro, or bridged ring. Examples of cycloalkyl include, but are not limited to, cyclopropyl, cyclobutyl, cyclopentyl, cyclohexyl, and adamantyl, or bicyclic hydrocarbyl such as decahydronaphthalene ring. The terms "cycloalkyl" and "carbocyclyl" can be used interchangeably.

The term "heterocycloalkyl" alone or as part of another substituent refers to cycloalkyl in which one or more than one (in some embodiments, 1 to 3) carbon atom is replaced by heteroatoms such as, but not limited to, N, O, S, and P. The term "m- to n-membered heterocycloalkyl" or "Cₘ-Cₙ heterocycloalkyl" should be understood to mean a saturated ring having m to n atoms, wherein the heterocyclic atoms are selected from N, O, S, or P, preferably selected from N, O, or S. For example, the term "4- to 8-membered heterocycloalkyl" or "C₄-C₈ heterocycloalkyl" should be understood to mean a saturated ring having 4 to 8 ring atoms, wherein 1, 2, 3, or 4 ring atoms are selected from N, O, S, or P, preferably selected from N, O, or S. "4- to 10-membered heterocyclyl" refers to a saturated ring having 4 to 10 ring atoms. When a prefix such as 4- to 8-membered or 4- to 10-membered is used to indicate heterocycloalkyl, the number of carbons is also meant to include heteroatoms. It includes a monocyclic, bicyclic, tricyclic, spiro, or bridged ring. Examples of heterocycloalkyl are: pyrrolidinyl, tetrahydrofuranyl, tetrahydropyranyl, tetrahydrothienyl, tetrahydropyridyl, tetrahydropyrrolyl, azetidinyl, thiazolidinyl, oxazolidinyl, piperidinyl, morpholinyl, thiomorpholinyl, piperazinyl, azepanyl, diazepanyl, oxazepanyl, *etc.* The terms "heterocycloalkyl" and "heteroalkyl ring" can be used interchangeably.

The term "alkenyl" alone or as part of another substituent refers to a linear or branched monovalent hydrocarbon group (for example, C₂-C₆ alkenyl; for another example, C₂-C₄ alkenyl) of 2 to 40 carbon atoms having at least one carbon-carbon sp2 double bond, and includes groups having *"cis"* and *"trans"* orientations or "*E*' and "*Z*" orientations. Examples of alkenyl include, but are not limited to, vinyl and allyl.

The term "alkynyl" alone or as part of another substituent refers to a linear or branched monovalent hydrocarbon group (for example, C₂-C₆ alkynyl; for another example, C₂-C₄ alkynyl) of 2 to 40 carbon atoms having at least one carbon-carbon sp triple bond. Examples of alkynyl include, but are not limited to, ethynyl and propynyl.

The term "alkoxy" alone or as part of another substituent refers to the group -O-R^{X}, wherein R^{X} is the "alkyl" as defined above.

The term "oxo" alone or as part of another substituent means that two H on methylene are substituted by O, that is, methylene is substituted by carbonyl.

The term "aryl" alone or as part of another substituent refers to a monocyclic or polycyclic carbocyclic ring having 6 to 20 carbon atoms, wherein at least one of the rings is an aromatic ring. When one of the rings is a non-aromatic ring, the group can be attached by an aromatic ring or by a non-aromatic ring. Examples of aryl include, but are not limited to, phenyl, naphthyl, tetrahydronaphthyl, 2,3-dihydroindenyl, biphenyl, phenanthryl, anthracenyl, and acenaphthyl.

The term "heteroaromatic ring" alone or as part of another substituent refers to a monocyclic or polycyclic carbocyclic ring, wherein at least one ring atom is a heteroatom independently selected from O, S, and N, and the remaining ring atoms are C, and wherein at least one ring is an aromatic ring. The group may be a carbon group or a heteroatom group *(i.e.,* it may be C-linked or N-linked, insofar as this is possible). When one of the rings is a non-aromatic ring, the group can be attached by an aromatic ring or by a non-aromatic ring. Examples of heteroaryl include, but are not limited to, imidazolyl, acridinyl, carbazolyl, cinnolinyl, quinoxalinyl, pyrazolyl, indolyl, benzotriazolyl, furyl, thienyl, benzothienyl, benzofuryl, quinolyl, isoquinolyl, oxazolyl, isoxazolyl, indolyl, pyrazinyl, pyridazinyl, pyridyl, pyrimidinyl, pyrrolyl, *N-*methylpyrrolyl, and tetrahydroquinolyl. The terms "heteroaromatic ring", "heteroaryl", or "heteroaromatic ring group" can be used interchangeably.

The term "heteroalkenyl ring" alone or as part of another substituent refers to a monocyclic group having a heteroatom (the monocyclic group has a double bond but is not aromatic), preferably a monocyclic ring containing 1, 2, or 3 ring heteroatoms independently selected from N, O, and S. Examples of heterocycloalkenyl are: dihydrofuryl, dihydrothienyl, dihydropyrrolyl, dioxolyl, dihydroimidazolyl, dihydropyrazolyl, dihydrothiazolyl, dihydroisothiazolyl, dihydrooxadiazolyl, dihydrothiadiazolyl, dihydrotriazolyl, dihydrotetrazolyl, tetrahydropyridyl, 3,4-dihydro-2H-pyran, pyranyl, thiopyranyl, dihydropyridyl, dihydropyrazinyl, dihydropyrimidinyl, oxazinyl, dihydrotetrazolyl, *etc.* The term "heteroalkenyl ring" and the term "heterocycloalkenyl" can be used interchangeably.

The term "spiro ring" alone or as part of another substituent refers to a polycyclic group that shares one carbon atom (called spiro atom) between monocyclic rings, which may contain one or more than one double bond, but none of the rings has a fully conjugated π-electron system. Spirocycloalkyl can be divided into monospirocycloalkyl, bispirocycloalkyl, or multispirocycloalkyl according to the number of spiro atoms shared between rings, preferably monospirocycloalkyl and bispirocycloalkyl. Non-limiting examples of spirocycloalkyl include:

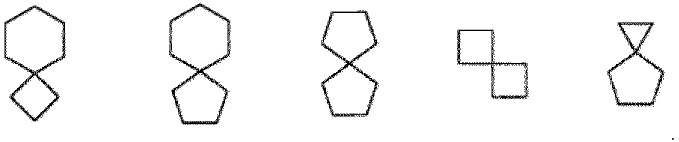

Spirocycloalkyl in which monospirocycloalkyl shares a spiro atom with heterocycloalkyl is also included. Non-limiting examples include: 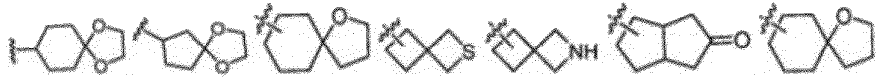 and

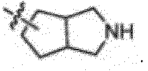

The term "bridged ring" refers to a cyclic hydrocarbon in which any two rings in a compound share two carbon atoms that are not directly connected, and can be divided into bicyclic hydrocarbon, tricyclic hydrocarbon, tetracyclic hydrocarbon, *etc.* according to the number of constituent rings. Non-limiting examples include:

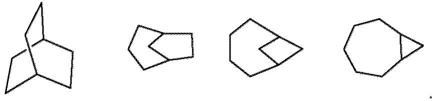

"Haloalkyl" alone or as part of another substituent refers to a branched and linear saturated aliphatic hydrocarbon group having a specific number of carbon atoms and substituted by one or more than one halogen (e.g., -CvFw, where v = 1 to 3 and w = 1 to (2v + 1)). Examples of haloalkyl include, but are not limited to, trifluoromethyl, trichloromethyl, pentafluoroethyl, pentachloroethyl, 2,2,2-trifluoroethyl, heptafluoropropyl, and heptachloropropyl.

"Deuteroalkyl" alone or as part of another substituent refers to alkyl substituted by one or more than one deuterium atom, wherein alkyl is as defined above.

In the present disclosure, "optional" or "optionally" means that the subsequently described event or circumstance may or may not occur, and the description includes both the occurrence and non-occurrence of the event or circumstance. For example, "optionally substituted aryl" means that the aryl is substituted or unsubstituted, and the description includes both substituted and unsubstituted aryl.

In the present disclosure, the term "salt" or "pharmaceutically acceptable salt" includes a pharmaceutically acceptable acid addition salt and a pharmaceutically acceptable base addition salt. The term "pharmaceutically acceptable" refers to that for those compounds, materials, compositions and/or dosage forms, they are suitable for use in contact with the tissues of human and animal without excess toxicity, irritation, allergic reactions, or other problems or complications within the scope of reliable medical judgment, and are commensurate with a reasonable benefit/risk ratio.

"Pharmaceutically acceptable acid addition salt" refers to a salt which retains the biological effectiveness of the free base without other side effects and is formed with an inorganic or organic acid. "Pharmaceutically acceptable base addition salt" refers to a salt which retains the biological effectiveness of the free acid without other side effects and is formed with an inorganic or organic base. In addition to the pharmaceutically acceptable salts, other salts may be adopted in the present disclosure. The other salts can serve as intermediates in the purification of compounds or in the preparation of other pharmaceutically acceptable salts or can be used for identifying, characterizing, or purifying the compounds of the present disclosure.

The term "solvate" refers to the compound of the present disclosure or a salt thereof including a stoichiometric or non-stoichiometric solvent bonded through an intermolecular non-covalent force. When the solvent is water, the solvate is a hydrate.

The term "solvate of pharmaceutically acceptable salt" means a substance formed by combining a compound with a pharmaceutically acceptable acid or base, and a solvent, which includes, but is not limited to, water, methanol, ethanol, *etc.* The amount of solvent can be stoichiometric or non-stoichiometric. The solvate of the pharmaceutically acceptable salt includes, but is not limited to, monohydrochloride monohydrate.

The term "prodrug" can be converted into the compound of the present disclosure having biological activity under physiological conditions or through solvolysis. The prodrug of the present disclosure is prepared by modifying the functional groups in the compound, and the modification can be removed by conventional operations or *in vivo,* so as to obtain the parent compound. The prodrug includes a compound formed by attaching a hydroxyl or amino group in the compound of the present disclosure to any group. When the prodrug of the compound of the present disclosure is administered to a mammal individual, the prodrug is dissociated to form a free hydroxyl group and a free amino group, respectively.

The term "pharmaceutical composition" of the present disclosure refers to a formulation of the compound of the present disclosure with a medium generally accepted in the art for delivering a biologically active compound to a mammal (*e*.*g*., a human). The medium includes a pharmaceutically acceptable carrier. The purpose of the pharmaceutical composition is to promote the administration to an organism, facilitate the absorption of the active ingredient and thus exert its biological activity.

In the present disclosure, "pharmaceutically acceptable carrier" includes, but is not limited to, any acceptable adjuvants, carriers, excipients, glidants, sweeteners, diluents, preservatives, dyes/coloring agents, flavoring agents, surfactants, wetting agents, dispersants, suspending agents, stabilizers, isotonic agents, solvents, or emulsifiers for humans or livestocks as licensed by relevant governmental administrations.

The term "excipient" refers to a pharmaceutically acceptable inert ingredient. Examples of categories of the term "excipient" include, but are not limited to, binders, disintegrants, lubricants, glidants, stabilizers, fillers, diluents, *etc.* Excipients can enhance operation properties of the pharmaceutical formulation, *i*.e., allowing the formulation to be more suitable for direct compression by increasing fluidity and/or adhesion.

The term "treatment" refers to therapeutic therapy. When referring to a specific disorder, treatment refers to: (1) ameliorating one or more than one biological manifestation of the disease or disorder, (2) interfering with (a) one or more than one point in the biological cascade leading to or causing the disorder or (b) one or more than one biological manifestation of the disorder, (3) ameliorating one or more than one symptom, effect, or side effect associated with the disorder, or one or more than one symptom, effect, or side effect associated with the disorder or its treatment, or (4) slowing the progression of the disorder or one or more than one biological manifestation of the disorder.

The term "prevention" refers to the reduction of the risk of acquiring or developing a disease or disorder.

The term "patient" refers to any animal that will or has received the administration of the compound or composition according to the example of the present disclosure, preferably a mammal. The term "mammal" includes any mammal. Examples of mammals include, but are not limited to, cows, horses, sheep, pigs, cats, dogs, mice, rats, rabbits, guinea pigs, monkeys, humans, *etc.,* preferably humans.

The term "therapeutically effective amount" refers to the amount of a compound that is sufficient to effectively treat the disease or disorder described herein when administered to a patient. The "therapeutically effective amount" will vary according to the compound, the disease and its severity, and the age of the patient to be treated, but it can be adjusted by those skilled in the art as needed.

The term "inflammatory bowel disease" refers to IBD and is used to describe diseases involving chronic inflammation of the digestive tract. It mainly includes: ulcerative colitis and Crohn's disease. Ulcerative colitis causes inflammation and ulcers in the superficial lining of the large intestine (colon) and rectum. Crohn's disease is characterized by inflammation of the lining of the digestive tract, and the inflammation usually affects the deeper layers of the digestive tract.

For the reaction of each step, the reaction temperature can be appropriately selected according to the solvent, starting material, reagent, *etc.,* and the reaction time can also be appropriately selected according to the reaction temperature, solvent, starting material, reagent, *etc.* After the reaction of each step, the target compound can be separated and purified from the reaction system by common methods, such as filtration, extraction, recrystallization, washing, silica gel column chromatography, and other methods. Without affecting the next reaction step, the target compound can also be directly used in the next reaction step without separation and purification.

The above preferred conditions can be combined arbitrarily to obtain preferred examples of the present disclosure without violating common knowledge in the art.

The positive and progressive effect of the present disclosure lies in the fact that after extensive and in-depth research, the present inventors unexpectedly developed a heterocyclic compound or a pharmaceutically acceptable salt thereof, a preparation method therefor, and a use thereof. The compound of formula I of the present disclosure has at least one of the following effect advantages:
(1) The present disclosure provides a compound of formula I, a solvate, a pharmaceutically acceptable salt, a solvate of the pharmaceutically acceptable salt, or a prodrug thereof, and the compound of formula I has a significant inhibitory effect on 15-PGDH.
(2) It can significantly increase the production of PGE₂ in a dose-dependent manner at 2.5 nM to 2500 nM, and has a significant effect on IPF and liver regeneration.
(3) Combined with the pharmacokinetic data in mice, it can be seen that the compounds of the present disclosure exhibit excellent pharmacokinetic properties in mice and have high safety and druggability.
(4) The present disclosure provides a method for preparing the compound of formula I, the solvate, the pharmaceutically acceptable salt, the solvate of the pharmaceutically acceptable salt, or the prodrug thereof, and the intermediate thereof. The method is simple in operation, high in yield and high in purity, and can be used in the industrialized production of medicines.

The above and/or additional aspects and advantages of the present disclosure will become apparent and readily understood from the description of the examples in conjunction with the following drawings.

### BRIEF DESCRIPTION OF THE DRAWINGS

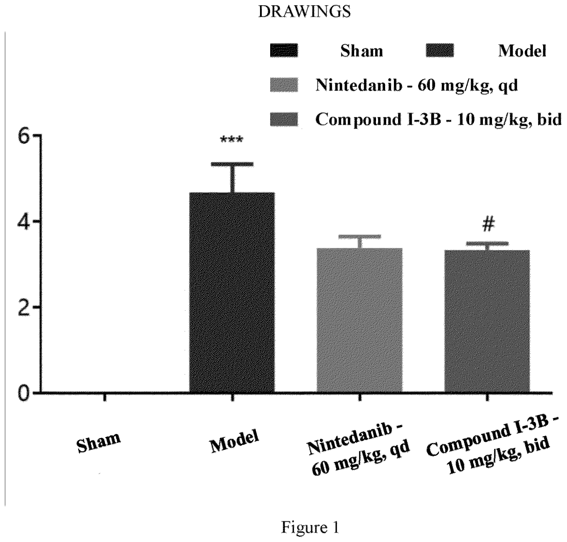

Figure 1 shows a graph of pulmonary fibrosis scores of animals in each group in the efficacy test on IPF prevention model of test example 3 of the present disclosure; wherein one-way ANOVA is used: "***" indicates *p*<0.001 vs. Sham, "#" indicates *p*<0.05 vs. G2 model group.

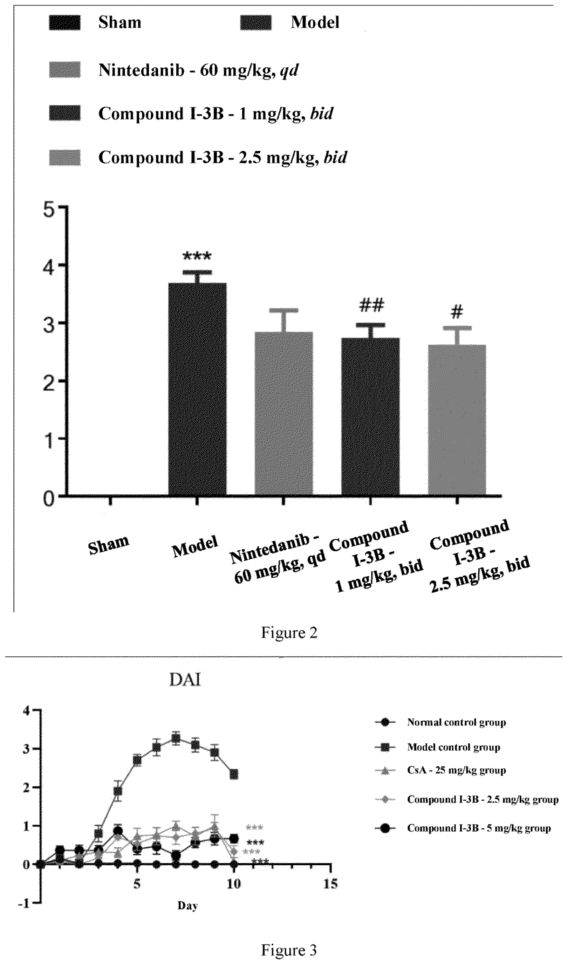

Figure 2 shows a graph of pulmonary fibrosis scores of animals in each group in the efficacy test on IPF treatment model of test example 4 of the present disclosure; wherein "***" indicates *p*<0.001 vs. G1-Sham; T-test: "#" indicates *p*<0.05 vs. model group; "##" indicates *p*<0.01 vs. model group.
Figure 3 shows a graph of DAI scores for each group of test example 7 of the present disclosure; wherein "***" indicates *p*<0.001 vs. G2 model control group.

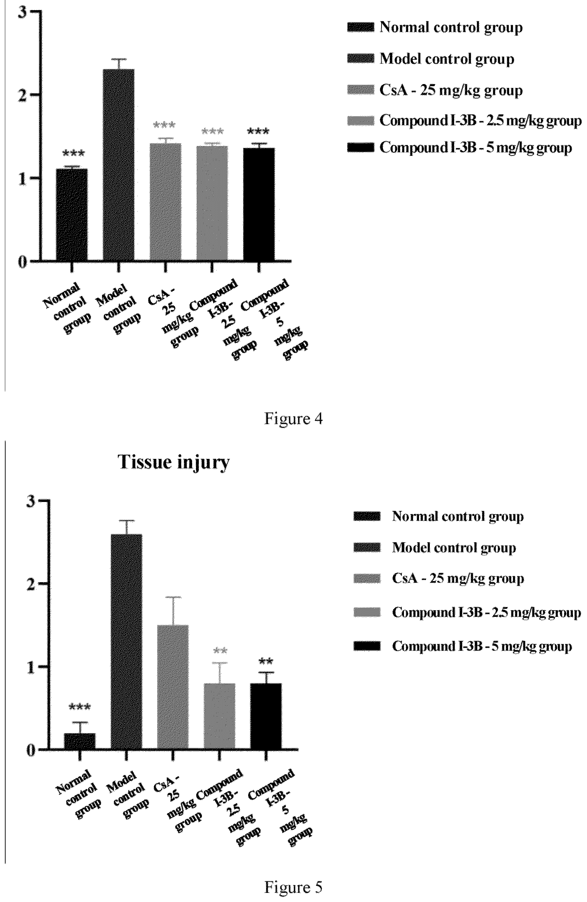

Figure 4 shows a graph of colon weight/colon length/body weight index for each group of test example 7 of the present disclosure; wherein one-way ANOVA is used: "***" indicates *p*<0.001 vs. G2 model control group.
Figure 5 shows a graph of colon injury scores for each group of test example 7 of the present disclosure; wherein "***" indicates *p*<0.001 vs. G2 model control group, and "**" indicates *p*<0.01 vs. G2 model control group.

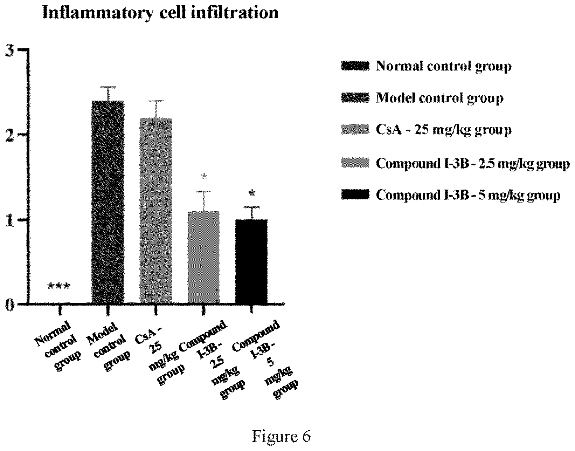

Figure 6 shows a graph of colon inflammatory cell infiltration scores for each group of test example 7 of the present disclosure; wherein "***" indicates *p*<0.001 vs. G2 model control group, and "*" indicates *p*<0.05 vs. G2 model control group.

### DETAILED DESCRIPTION OF THE PREFERRED EMBODIMENT

The present disclosure is further illustrated below by the way of examples, but the present disclosure is not thereby limited to the scope of the described examples.

The present disclosure is further illustrated below in conjunction with specific examples. It should be understood that the following description is only the most preferred embodiment of the present disclosure and should not be construed as limiting the scope of protection of the present disclosure. On the basis of a full understanding of the present disclosure, the experimental methods without indication of specific conditions in the following examples shall be implemented usually in accordance with conventional conditions or the conditions suggested by the manufacturer. Those skilled in the art can make non-essential modifications to the technical solutions of the present disclosure, and such modifications should be considered to be included in the scope of protection of the present disclosure.

**The abbreviations in the present disclosure are defined as follows:**
Symbols or units:
   IC₅₀: half inhibitory concentration, which refers to a concentration at which half of the maximum inhibitory effect is reached
   M: mol/L, for example, n-butyllithium (14.56 mL, 29.1 mmol, 2.5 M n-hexane) means a solution of n-butyllithium in n-hexane with a molar concentration of 2.5 mol/L
   N: equivalent concentration, for example, 2 N hydrochloric acid means 2 mol/L hydrochloric acid solution
Reagents:
   NBS: *N*-bromosuccinimide
   DMF: *N,N*-dimethylformamide
   IPA: isopropyl alcohol
   DEA: diethylamine

### Intermediate A1: Preparation of 6'-bromo-2'-methylspiro[cyclopropane-1,1'-isoindolin]-3'-one

The synthetic route of **intermediate A1** was as follows:

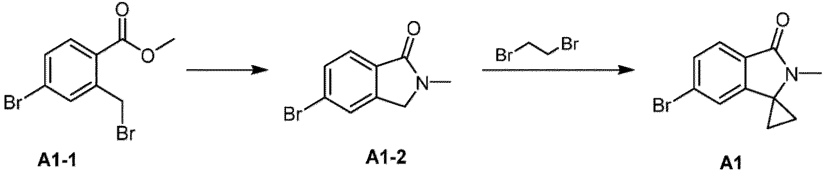

### Step 1: Synthesis of 5-bromo-2-methylisoindolin-1-one (A1-2)

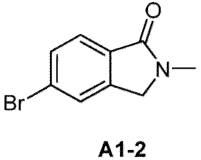

To a mixture of methylamine (1.95 mL, 3.9 mmol) and triethylamine (0.9 mL, 6.52 mmol) in 2 M tetrahydrofuran was added methyl 4-bromo-2-(bromomethyl)benzoate (l g, 3.26 mmol) in a sealed tube, and the reaction mixture was heated at 100°C for 12 hours. After the reaction was completed, the mixture was concentrated under reduced pressure. The resulting residue was diluted with ethyl acetate and washed with water. The organic layer was dried over anhydrous sodium sulfate, filtered, and concentrated under reduced pressure. The residue was triturated with hexane to obtain 5-bromo-2-methylisoindolin-1-one (500 mg, yield: 68%).

LC-MS, M/Z (ESI): 225.9 [M+H]⁺.

### Step 2: Synthesis of 6'-bromo-2'-methylspiro[cyclopropane-1,1'-isoindolin]-3'-one (A1)

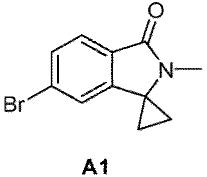

5-Bromo-2-methylisoindolin-1-one (500 mg, 2.21 mmol) was dissolved in DMF (20 mL), then 1,2-dibromoethane (499 mg, 2.65 mmol) and cesium carbonate (l.44 g, 4.42 mmol) were added thereto, and the reaction mixture was heated at 100°C for 12 hours. After the reaction was completed, the mixture was concentrated under reduced pressure. The resulting residue was diluted with ethyl acetate and washed with water. The organic layer was dried over anhydrous sodium sulfate, filtered, and concentrated under reduced pressure. The residue was purified by silica gel column chromatography (petroleum ether: ethyl acetate (V/V) = 20:1 to 6:1) to obtain 6'-bromo-2'-methylspiro[cyclopropane-1,1'-isoindolin]-3'-one (250 mg, yield: 44.8%).

LC-MS, M/Z (ESI): 251.9 [M+H]⁺.

### Intermediate A2: Preparation of 6'-bromo-2'-(deuteromethyl)-spiro[cyclopropane-1,1'-isoindolin]-3'-one

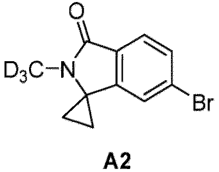

Intermediate A2 was synthesized with reference to the synthesis of intermediate A1 by replacing methylamine with deuteromethylamine. LC-MS, M/Z (ESI): 255.1 [M+H]⁺.

### Intermediate A3: Preparation of 3'-bromo-6'-methylspiro[cyclopropane-1,5'-pyrrolo[3,4-b]pyridin]-7'(6'H)-one

The synthetic route of intermediate A3 was as follows:

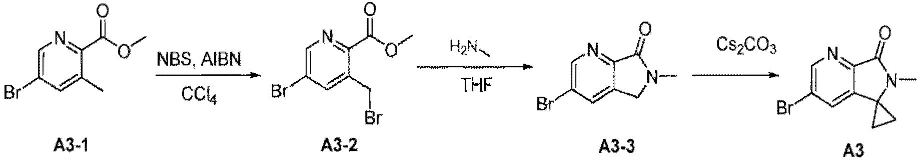

### Step 1: Synthesis of methyl 5-bromo-3-(bromomethyl)pyridine-2-carboxylate (A3-2)

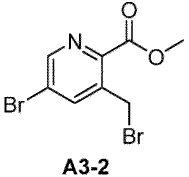

Methyl 5-bromo-3-methylpyridine-2-carboxylate (A3-1) (5 g, 21.73 mmol) was dissolved in carbon tetrachloride (50 mL), then azobisisobutyronitrile (0.71 g, 4.35 mmol) and NBS (4.64 g, 26.1 mmol) were added thereto, and the reaction mixture was reacted at 80°C for 5 hours. The reaction mixture was diluted with water (100 mL), and extracted with ethyl acetate (100 mL). The organic phases were combined, washed with saturated brine (100 mL), dried over anhydrous sodium sulfate, filtered, and concentrated under reduced pressure to obtain a crude product. The crude product was purified by silica gel column chromatography (petroleum ether: ethyl acetate (V/V) = 5:1 to 1:1) to obtain compound 5-bromo-3-(bromomethyl)pyridine-2-carboxylate (**A3-2**) (4.5 g, yield: 67%).

LC-MS, M/Z (ESI): 307.8 [M+H]⁺.

### Step 2: Synthesis of 3-bromo-6-methyl-5,6-dihydro-7H-pyrrolo[3,4-b]pyridin-7-one (A3-3)

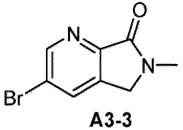

To a mixture of methylamine (1.95 mL, 3.9 mmol) and triethylamine (0.9 mL, 6.52 mmol) in 2 M tetrahydrofuran was added 5-bromo-3-(bromomethyl)pyridine-2-carboxylate (l g, 3.24 mmol) in a sealed tube, and the reaction mixture was heated at 100°C for 12 hours. After the reaction was completed, the mixture was concentrated under reduced pressure. The resulting residue was diluted with ethyl acetate and washed with water. The organic layer was dried over anhydrous sodium sulfate, filtered, and concentrated under reduced pressure. The residue was triturated with hexane to obtain 3-bromo-6-methyl-5,6-dihydro-7*H*-pyrrolo[3,4-*b*]pyridin-7-one (500 mg, yield: 68%).

LC-MS, M/Z (ESI): 226.9 [M+H]⁺.

### Step 3: Synthesis of 3'-bromo-6'-methylspiro[cyclopropane-1,5'-pyrrolo[3,4-b]pyridin]-7'(6'H)-one (A3)

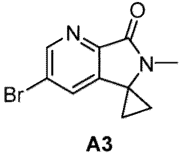

3-Bromo-6-methyl-5,6-dihydro-7*H*-pyrrolo[3,4-*b*]pyridin-7-one (500 mg, 2.20 mmol) was dissolved in DMF (20 mL), then 1,2-dibromoethane (496 mg, 2.64 mmol) and cesium carbonate (l.43 g, 4.40 mmol) were added thereto, and the reaction mixture was heated at 100°C for 12 hours. After the reaction was completed, the mixture was concentrated under reduced pressure. The resulting residue was diluted with ethyl acetate and washed with water. The organic layer was dried over anhydrous sodium sulfate, filtered, and concentrated under reduced pressure. The residue was purified by silica gel column chromatography (petroleum ether: ethyl acetate (V/V) = 10:1 to 3:1) to obtain 3'-bromo-6'-methylspiro[cyclopropane-1,5'-pyrrolo[3,4-*b*]pyridin]-7'(6'*H*)-one (250 mg, yield: 44.9%).

LC-MS, M/Z (ESI): 253.0 [M+H]⁺.

### Example 1: Preparation of 5-(6-(4,4-difluoropiperidine-1-carbonyl)-1,1a,2,7b-tetrahydro-3H-cyclopropa[c][1,8]naphthyridin-3-yl)-2-methylisoindolin-1-one (I-1)

The synthetic route of target **compound 1-1** was as follows:

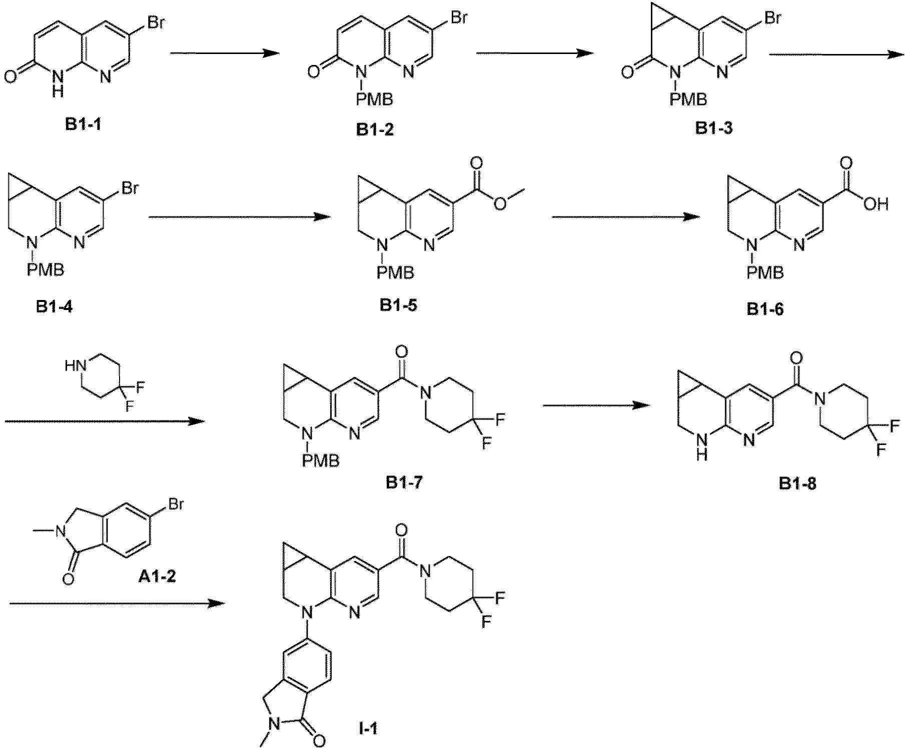

### Step 1: Synthesis of 6-bromo-1-(4-methoxybenzyl)-1,8-naphthyridin-2(1H)-one (B1-2)

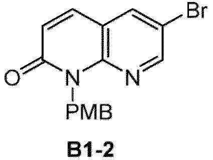

6-Bromo-1,8-naphthyridin-2(1*H*)-one (500 mg, 2.22 mmol) was dissolved in tetrahydrofuran (5 mL), then sodium hydride (133 mg, 3.33 mmol, content: 60%) was added thereto at 0 to 5°C under nitrogen atmosphere, and the reaction mixture was reacted at 0 to 5°C for 1 hour. 1-(Chloromethyl)-4-methoxybenzene (522 mg, 3.33 mmol) was added thereto at 0 to 10°C, and then the reaction mixture was reacted at 25°C for 5 hours. The reaction mixture was added with saturated ammonium chloride (50 mL) to quench at 0 to 10°C under nitrogen atmosphere, and then extracted with ethyl acetate (50 mL * 2). The organic phases were combined, washed with saturated brine (50 mL), dried over anhydrous sodium sulfate, filtered, and concentrated to obtain 6-bromo-1-(4-methoxybenzyl)-1,8-naphthyridin-2(1*H*)-one (B1-2) (700 mg, yellow crude product), which was directly used in the next step.

LC-MS, M/Z (ESI): 345.0 [M+H]⁺.

### Step 2: Synthesis of 6-bromo-3-(4-methoxybenzyl)-1,1a,3,7b-tetrahydro-2H-cyclopropa[c][1,8]naphthyridin-2-one (B1-3)

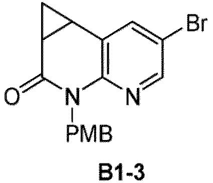

Sodium hydride (118 mg, 2.95 mmol, purity: 60%) was dispersed in tetrahydrofuran (5 mL) under nitrogen atmosphere, then trimethylsulfoxonium iodide (574 mg, 2.61 mmol) was added thereto in batches at 0 to 10°C, and then the reaction mixture was reacted at 0 to 10°C for 1 hour. 6-Bromo-1-(4-methoxybenzyl)-1,8-naphthyridin-2(1*H*)-one (600 mg, 1.74 mmol) was added thereto in batches at 25°C under nitrogen atmosphere, and then the reaction mixture was reacted at 90°C for 2 hours. The reaction mixture was added with saturated ammonium chloride (30 mL) to quench at 0 to 10°C under nitrogen atmosphere, and then extracted with ethyl acetate (50 mL * 2). The organic phases were combined, washed with saturated brine (50 mL), dried over anhydrous sodium sulfate, filtered, and concentrated to obtain a crude product, which was then purified by silica gel column chromatography (petroleum ether: ethyl acetate (V/V) = 20:1 to 6:1) to obtain compound 6-bromo-3-(4-methoxybenzyl)-1,1a,3,7b-tetrahydro-2*H*-cyclopropa[*c*][1,8]naphthyridin-2-one (**B1-3**) (yellow solid, 350 mg, yield: 56.1%).

LC-MS, M/Z (ESI): 359.0 [M+H]⁺.

### Step 3: Synthesis of 6-bromo-3-(4-methoxybenzyl)-1a,2,3,7b-tetrahydro-1H-cyclopropa[c][1,8]naphthyridine (B1-4)

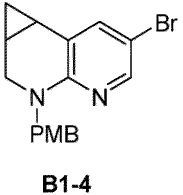

To tetrahydrofuran (4 mL) was added 6-bromo-3-(4-methoxybenzyl)-1,1a,3,7b-tetrahydro-2H-cyclopropa[c][1,8]naphthyridin-2-one (500 mg, 1.39 mmol) under nitrogen atmosphere, then borane dimethyl sulfide solution (10 M, 1.39 mL, 13.9 mmol) was added dropwise thereto at 0 to 10°C, and then the reaction mixture was stirred and reacted at 60°C for 1 hour. The reaction mixture was added with water (10 mL) to quench at 0 to 5°C under nitrogen atmosphere, and then extracted with dichloromethane (50 mL * 2). The organic phases were combined, washed with saturated brine (50 mL), dried over anhydrous sodium sulfate, filtered, and concentrated to obtain a crude product, which was then purified by silica gel column chromatography (petroleum ether: ethyl acetate (V/V) = 200:1 to 20:1) to obtain compound 6-bromo-3-(4-methoxybenzyl)-1a,2,3,7b-tetrahydro-1*H-*cyclopropa[*c*][1,8]naphthyridine (**B1-4**) (400 mg, yield: 69.4%) as a colorless liquid.

LC-MS, M/Z (ESI): 345.0 [M+H]⁺.

### Step 4: Synthesis of methyl 3-(4-methoxybenzyl)-1a,2,3,7b-tetrahydro-1H-cyclopropa[c][1,8]naphthyridine-6-carboxylate (B1-5)

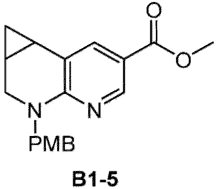

To a high pressure reactor were added 6-bromo-3-(4-methoxybenzyl)-1a,2,3,7b-tetrahydro-1*H*-cyclopropa[*c*][1,8]naphthyridine (400 mg, 1.16 mmol), [1,1'-bis(diphenylphosphino)ferrocene]dichloropalladium(II) (169 mg, 232 µmol), triethylamine (352 mg, 3.48 mmol), and methanol (10 mL) at 25°C under nitrogen atmosphere, then the reaction mixture was replaced with nitrogen, and CO was introduced thereto. The reaction mixture was stirred and reacted at 120°C, 4 Mpa pressure for 48 hours, filtered, and concentrated to obtain a crude product, which was then purified by silica gel column chromatography (petroleum ether: ethyl acetate (V/V) = 200:1 to 20:1) to obtain compound methyl 3-(4-methoxybenzyl)-1a,2,3,7b-tetrahydro-1*H*-cyclopropa[*c*][1,8]naphthyridine-6-carboxylate (**B1-5**) (350 mg, yield: 93.1%) as a white solid.

LC-MS, M/Z (ESI): 325.1 [M+H]⁺.

### Step 5: Synthesis of 3-(4-methoxybenzyl)-1a,2,3,7b-tetrahydro-1H-cyclopropa[c][1,8]naphthyridine-6-carboxylic acid (B1-6)

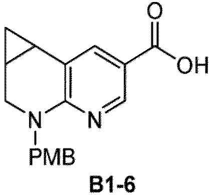

To tetrahydrofuran (3 mL) and water (1 mL) were added methyl 3-(4-methoxybenzyl)-1a,2,3,7b-tetrahydro-1*H*-cyclopropa[*c*][1,8]naphthyridine-6-carboxylate (340 mg, 1.05 mmol) and lithium hydroxide monohydrate (176 mg, 4.19 mmol) under nitrogen atmosphere, and the reaction mixture was reacted at 60°C for 1 hour. The reaction mixture was added with water (10 mL), then added with 1 M dilute hydrochloric acid to adjust the pH to 3 to 4 at 0 to 10°C, and extracted with dichloromethane (50 mL * 2). The organic phases were combined, washed with saturated brine (50 mL), dried over anhydrous sodium sulfate, filtered, and concentrated to obtain 3-(4-methoxybenzyl)-1a,2,3,7b-tetrahydro-1*H*-cyclopropa[*c*][1,8]naphthyridine-6-carboxylic acid (**B1-6**) (320 mg, crude product as a yellow solid).

LC-MS, M/Z (ESI): 310.9 [M+H]⁺.

### Step 6: Synthesis of (4,4-difluoropiperidin-1-yl)(3-(4-methoxybenzyl)-1a,2,3,7b-tetrahydro-1H-cyclopropa[c][1,8]naphthyridin-6-yl)methanone (B1-7)

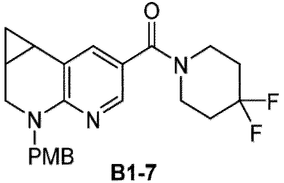

To *N,N-*dimethylformamide (4 mL) were added 3-(4-methoxybenzyl)-1a,2,3,7b-tetrahydro-1*H*-cyclopropa[*c*][1,8]naphthyridine-6-carboxylic acid (320 mg, 1.03 mmol), 4,4-difluoropiperidine (249 mg, 2.06 mmol), *N,N*-diisopropylethylamine (399 mg, 3.09 mmol), and 2-(7-azabenzotriazol-1-yl)-*N,N,N',N'*-tetramethyluronium hexafluorophosphate (784 mg, 2.06 mmol) under nitrogen atmosphere, and then the reaction mixture was stirred and reacted at 25°C for 16 hours. The reaction mixture was added with water (30 mL), and then extracted with ethyl acetate (50 mL * 2). The organic phases were combined, washed with saturated brine (50 mL), dried over anhydrous sodium sulfate, filtered, and concentrated to obtain a crude product, which was then purified by silica gel column chromatography (petroleum ether: ethyl acetate (V/V) = 50:1 to 6:1) to obtain compound (4,4-difluoropiperidin-1-yl)(3-(4-methoxybenzyl)-1a,2,3,7b-tetrahydro-1*H*-cyclopropa[*c*][1,8]naphthyridin-6-yl)methanone (**B1-7**) (350 mg, two-step yield: 82.1%) as a white solid.

LC-MS, M/Z (ESI): 414.3 [M+H]⁺.

### Step 7: Synthesis of (4,4-difluoropiperidin-1-yl)(1a,2,3,7b-tetrahydro-1H-cyclopropa[c][1,8]naphthyridin-6-yl)methanone (B1-8)

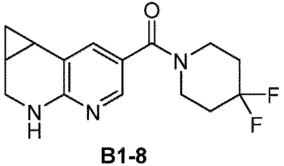

To trifluoroacetic acid (0.2 mL) was added (4,4-difluoropiperidin-1-yl)(3-(4-methoxybenzyl)-1a,2,3,7b-tetrahydro-1*H*-cyclopropa[*c*][1,8]naphthyridin-6-yl)methanone (50 mg, 121 µmol) under nitrogen atmosphere, and then the reaction mixture was stirred and reacted at 60°C for 4 hours, and concentrated under reduced pressure to obtain the crude product of (4,4-difluoropiperidin-1-yl)(1a,2,3,7b-tetrahydro-1*H-*cyclopropa[c][1,8]naphthyridin-6-yl)methanone (**B1-8**) (35.0 mg) as a yellow solid.

LC-MS, M/Z (ESI): 294.2 [M+H]⁺.

### Step 8: 5-(6-(4,4-Difluoropiperidine-1-carbonyl)-1,1a,2,7b-tetrahydro-3H-cyclopropa[c][1,8]naphthyridin-3-yl)-2-methylisoindolin-1-one (I-1)

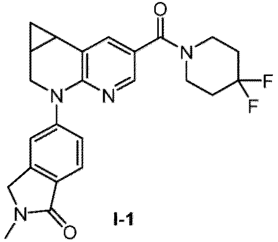

To dioxane (1 mL) were added (4,4-difluoropiperidin-1-yl)(1a,2,3,7b-tetrahydro-1*H-*cyclopropa[*c*][1,8]naphthyridin-6-yl)methanone (35.0 mg, 119 µmol), cesium carbonate (155 mg, 477 µmol), 2-methyl-5-bromoisoindolin-1-one (40.4 mg, 179 µmol), bis(dibenzylideneacetone)palladium (6.86 mg, 11.9 µmol), and 4,5-bis(diphenylphosphino)-9,9-dimethylxanthene (69.0 mg, 119 µmol) under nitrogen atmosphere. The reaction mixture was stirred and reacted at 100°C for 12 hours, and then concentrated to obtain a crude product. The crude product was then subjected to preparative high performance liquid chromatography (column: Phenomenex Synergi C₁₈ 150 * 25 mm * 5 µm; solvent: A = water + 0.1 vol% trifluoroformic acid (99%), B = acetonitrile; gradient: 23% to 53% B, 9 min) to obtain target compound 5-(6-(4,4-difluoropiperidine-1-carbonyl)-1,1a,2,7b-tetrahydro-3*H-*cyclopropa[*c*][1,8]naphthyridin-3-yl)-2-methylisoindolin-1-one (**I-1**) (13.0 mg, yield: 23.5%).

¹H NMR (400 MHz, CDCl₃) δ 7.98 (d, 1H), 7.80 (d, 1H), 7.65 (d, 1H), 7.26 (s, 1H), 7.25 (s, 1H), 4.34 (d, 2H), 3.84-3.92 (m, 2H), 3.74 (br, 4H), 3.18 (s, 3H), 2.00-2.06 (m, 6H), 1.24-1.26 (m, 1H), 1.07-1.10 (m, 1H).

LC-MS, M/Z (ESI): 439.3 [M+H]⁺.

### Example 2: Preparation of 3-(6-(4,4-difluoropiperidine-1-carbonyl)-1,1a,2,7b-tetrahydro-3H-cyclopropa[c][1,8]naphthyridin-3-yl)-6-methyl-5,6-dihydro-7H-pyrrolo[3,4-b]pyridin-7-one (1-2)

The synthetic route of target **compound 1-2** was as follows:

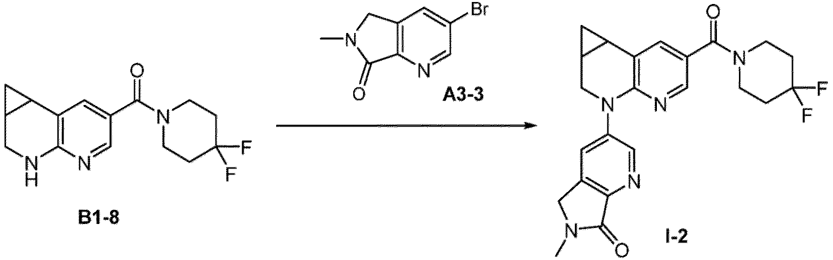

To dioxane (1 mL) were added (4,4-difluoropiperidin-1-yl)(la,2,3,7b-tetrahydro-1*H-*cyclopropa[*c*][1,8]naphthyridin-6-yl)methanone (35.0 mg, 119 µmol), cesium carbonate (155 mg, 477 µmol), 3-bromo-6-methyl-5,6-dihydro-7*H*-pyrrolo[3,4-*b*]pyridin-7-one (40.6 mg, 179 µmol), bis(dibenzylideneacetone)palladium (6.86 mg, 11.9 µmol), and 4,5-bis(diphenylphosphino)-9,9-dimethylxanthene (69.0 mg, 119 µmol) under nitrogen atmosphere. The reaction mixture was stirred and reacted at 100°C for 12 hours, and then concentrated to obtain a crude product. The crude product was then purified by silica gel column chromatography (petroleum ether: ethyl acetate (V/V) = 5:1 to 1:1) to obtain target compound 3-(6-(4,4-difluoropiperidine-1-carbonyl)-1,1a,2,7b-tetrahydro-3*H-*cyclopropa[c][1,8]naphthyridin-3-yl)-6-methyl-5,6-dihydro-7*H*-pyrrolo[3,4-b]pyridin-7-one (**I-2**) (15.0 mg, yield: 28.6%).

¹H NMR (400 MHz, DMSO) δ 8.60 (d, 1H), 7.93 (d, 1H), 7.84 (d, 1H), 7.80 (d, 1H), 4.45 (s, 2H), 3.87 (s, 2H), 3.60 (br, 4H), 3.10 (s, 3H), 2.18-2.50 (m, 1H), 2.00-2.09 (m, 3H), 1.22-1.23 (m, 2H), 1.06-1.08 (m, 1H), 0.83-0.86 (m, 1H).

LC-MS, M/Z (ESI): 440.2 [M+H]⁺.

### Example 3: Preparation of 7-(6-(4,4-difluoropiperidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (I-3)

The synthetic route of target compound **I-3** was as follows:

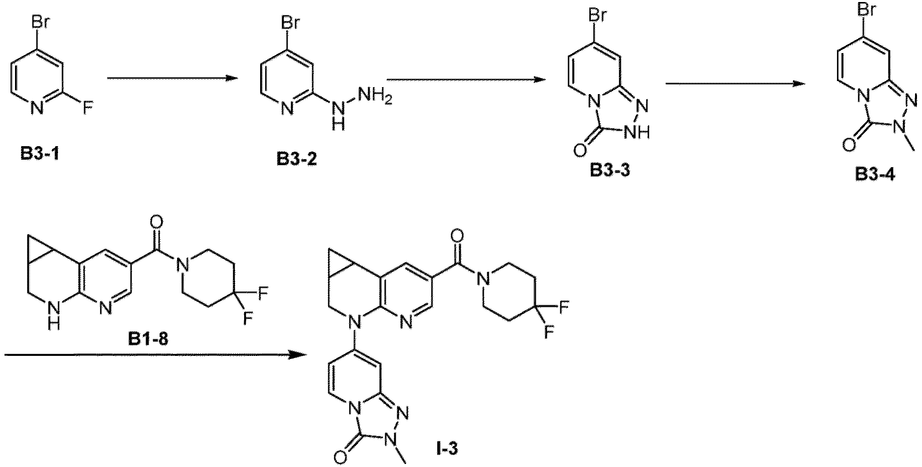

### Step 1: 4-Bromo-2-hydrazinopyridine (B3-2)

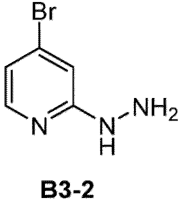

To a solution of 4-bromo-2-fluoropyridine (5 g, 28.4 mmol) in ethanol (50 mL) was added 80% hydrazine hydrate solution (17 mL). The reaction mixture was stirred at room temperature for 12 hours and concentrated. The residue was diluted with water (50 mL), and extracted with ethyl acetate (50 mL * 3). The organic phases were combined, dried over anhydrous sodium sulfate, filtered, and subjected to rotary evaporation until dryness to obtain 4-bromo-2-hydrazinopyridine (3.7 g, yield: 69.3%).

LC-MS, M/Z (ESI): 187.9 [M+H]⁺.

### Step 2: 7-Bromo-[1,2,4]triazolo[4,3-a]pyridin-3(2H)-one (B3-3)

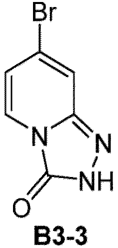

4-Bromo-2-hydrazinopyridine (3.2 g, 17.02 mmol) was dissolved in tetrahydrofuran (30 mL), and carbonyldiimidazole (5.52 g, 34.0 mmol) was added thereto. The reaction mixture was stirred at room temperature for 12 hours, diluted with water (50 mL), and extracted with ethyl acetate (50 mL * 3). The organic phases were combined, dried over anhydrous sodium sulfate, filtered, subjected to rotary evaporation until dryness, and slurried with ethyl acetate (10 mL) to obtain 7-bromo-[1,2,4]triazolo[4,3-*a*]pyridin-3(2*H*)-one (2.2 g, yield: 60.4%).

LC-MS, M/Z (ESI): 213.9 [M+H]⁺.

### Step 3: 7-Bromo-2-methyl-[1,2,4]triazolo[4,3-a]pyridin-3(2H)-one (B3-4)

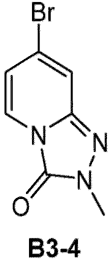

To a mixture of 7-bromo-[1,2,4]triazolo[4,3-*a*]pyridin-3(2*H*)-one (500 mg, 2.33 mmol) and cesium carbonate (1.14 g, 3.50 mmol) in DMF (5 mL) was added dropwise iodomethane (995 mg, 7.01 mmol) at room temperature. The reaction mixture was stirred at room temperature for 3 hours, diluted with water (20 mL), and extracted with ethyl acetate (20 mL * 3). The organic phases were combined, dried over anhydrous sodium sulfate, filtered, subjected to rotary evaporation until dryness, and the crude product was purified by silica gel column chromatography (petroleum ether: ethyl acetate (V/V) = 5:1 to 1:1) to obtain 7-bromo-2-methyl-[1,2,4]triazolo[4,3-*a*]pyridin-3(2*H*)-one (400 mg, yield: 75%).

LC-MS, M/Z (ESI): 227.9 [M+H]⁺.

### Step 4: 7-(6-(4,4-Difluoropiperidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (I-3)

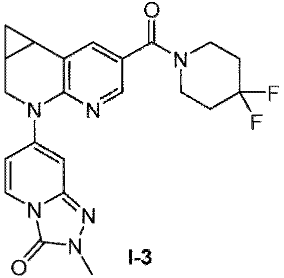

To dioxane (1 mL) were added (4,4-difluoropiperidin-1-yl)(1a,2,3,7b-tetrahydro-1*H-*cyclopropa[*c*][1,8]naphthyridin-6-yl)methanone (35.0 mg, 119 µmol), cesium carbonate (155 mg, 477 µmol), 7-bromo-2-methyl-[1,2,4]triazolo[4,3-*a*]pyridin-3(2*H*)-one (40.8 mg, 179 µmol), bis(dibenzylideneacetone)palladium (6.86 mg, 11.9 µmol), and 4,5-bis(diphenylphosphino)-9,9-dimethylxanthene (69.0 mg, 119 µmol) under nitrogen atmosphere. The reaction mixture was stirred and reacted at 100°C for 12 hours, and then concentrated to obtain a crude product. The crude product was then purified by silica gel column chromatography (petroleum ether: ethyl acetate (V/V) = 5:1 to 1:1) to obtain target compound 7-(6-(4,4-difluoropiperidine-1-carbonyl)-1,1a,2,7b-tetrahydro-3*H-*cyclopropa[*c*][1,8]naphthyridin-3-yl)-2-methyl-[1,2,4]triazolo[4,3-*a*]pyridin-3(2*H*)-one **(I-3)** (20.0 mg, yield: 38.1%).

The resulting product was separated by SFC (chromatographic column: Chiralpak AD-3 50 × 4.6 mm I.D., 3 µm; mobile phase: mobile phase A: CO₂, mobile phase B: IPA + ACN (0.05 vol% DEA); isocratic elution: 50 vol% IPA + ACN (0.05 vol% DEA) in CO₂; flow rate: 3 mL/min; detector: PDA, column temperature: 35°C; column pressure: 100 Bar).

Isomer **I-3A** (RT = 0.743 min) and isomer **I-3B** (RT = 1.670 min) were obtained.

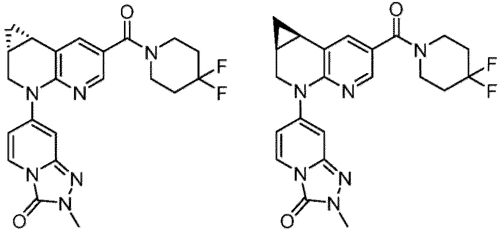

¹H NMR (400 MHz, DMSO) δ 8.04 (d, 1H), 7.81 (d, 1H), 7.61 (d, 1H), 6.68 (d, 1H), 6.58 (dd, 2.0 Hz, 1H), 3.90 (d, 1H), 3.70-3.52 (m, 5H), 3.48 (s, 3H), 2.18 (dt, 1H), 2.13-1.93 (m, 5H), 1.06 (dd, 2H).

LC-MS, M/Z (ESI): 441.1 [M+H]⁺.

### Example 4: Preparation of 2-cyclopropyl-7-(6-(4,4-difluoropiperidine-1-carbonyl)-1,1a,2,7b-tetrahydro-3H-cyclopropa[c][1,8]naphthyridin-3-yl)-[1,2,4]triazolo[4,3-a]pyridin-3(2H)-one (I-4)

The synthetic route of target compound **1-4** was as follows:

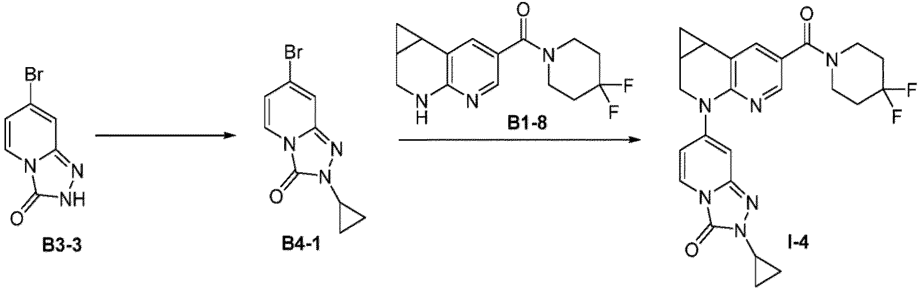

### Step 1: 7-Bromo-2-cyclopropyl-[1,2,4]triazolo[4,3-a]pyridin-3(2H)-one (B4-1)

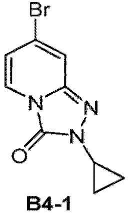

To a mixture of 7-bromo-[1,2,4]triazolo[4,3-*a*]pyridin-3(2*H*)-one (500 mg, 2.33 mmol) and cesium carbonate (1.14 g, 3.50 mmol) in DMF (5 mL) was added dropwise cyclopropyl bromide (848 mg, 7.01 mmol). The reaction mixture was stirred at room temperature for 3 hours, diluted with water (20 mL), and extracted with ethyl acetate (20 mL * 3). The organic phases were combined, dried over anhydrous sodium sulfate, filtered, subjected to rotary evaporation until dryness, and the crude product was purified by silica gel column chromatography (petroleum ether: ethyl acetate (V/V) = 5:1 to 1:1) to obtain 7-bromo-2-cyclopropyl-[1,2,4]triazolo[4,3-*a*]pyridin-3(2*H*)-one **(B4-1)** (200 mg, yield: 37.5%).

LC-MS, M/Z (ESI): 253.9 [M+H]⁺.

### Step 4: 2-Cyclopropyl-7-(6-(4,4-difluoropiperidine-1-carbonyl)-1,1a,2,7b-tetrahydro-3H-cyclopropa[c][1,8]naphthyridin-3-yl)-[1,2,4]triazolo[4,3-a]pyridin-3(2H)-one (I-4)

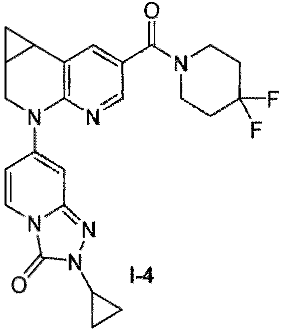

To dioxane (1 mL) were added (4,4-difluoropiperidin-1-yl)(1a,2,3,7b-tetrahydro-1*H-*cyclopropa[*c*][1,8]naphthyridin-6-yl)methanone (35.0 mg, 119 µmol), cesium carbonate (155 mg, 477 µmol), 7-bromo-2-cyclopropyl-[1,2,4]triazolo[4,3-*a*]pyridin-3(2*H*)-one (45.5 mg, 179 µmol), bis(dibenzylideneacetone)palladium (6.86 mg, 11.9 µmol), and 4,5-bis(diphenylphosphino)-9,9-dimethylxanthene (69.0 mg, 119 µmol) under nitrogen atmosphere. The reaction mixture was stirred and reacted at 100°C for 12 hours, and then concentrated to obtain a crude product. The crude product was purified by silica gel column chromatography (petroleum ether: ethyl acetate (V/V) = 5:1 to 1:1) to obtain target compound 2-cyclopropyl-7-(6-(4,4-difluoropiperidine-1-carbonyl)-1,1a,2,7b-tetrahydro-3*H-*cyclopropa[*c*][1,8]naphthyridin-3-yl)-[1,2,4]triazolo[4,3-*a*]pyridin-3(2*H*)-one **(I-4)** (20.0 mg, yield: 55.7%).

LC-MS, M/Z (ESI): 467.1 [M+H]⁺.

### Example 5: Preparation of 6'-(6-(4,4-difluoropiperidine-1-carbonyl)-1,1a,2,7b-tetrahydro-3H-cyclopropa[c][1,8]naphthyridin-3-yl)-2'-methylspiro[cyclopropane-1,1'-isoindolin]-3'-one (I-5)

The synthetic route of target compound **1-5** was as follows:

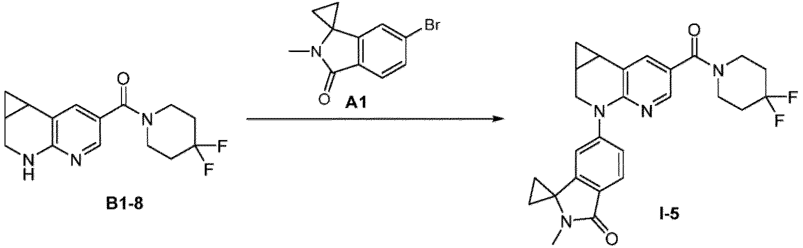

To dioxane (1 mL) were added (4,4-difluoropiperidin-1-yl)(1a,2,3,7b-tetrahydro-1*H-*cyclopropa[*c*][1,8]naphthyridin-6-yl)methanone (35.0 mg, 119 µmol), cesium carbonate (155 mg, 477 µmol), 6'-bromo-2'-methylspiro[cyclopropane-1,1'-isoindolin]-3'-one (45.1 mg, 179 µmol), bis(dibenzylideneacetone)palladium (6.86 mg, 11.9 µmol), and 4,5-bis(diphenylphosphino)-9,9-dimethylxanthene (69.0 mg, 119 µmol) under nitrogen atmosphere. The reaction mixture was stirred and reacted at 100°C for 12 hours, and then concentrated to obtain a crude product. The crude product was then purified by silica gel column chromatography (petroleum ether: ethyl acetate (V/V) = 5:1 to 1:1) to obtain target compound 6'-(6-(4,4-difluoropiperidine-1-carbonyl)-1,1a,2,7b-tetrahydro-3*H-*cyclopropa[*c*][1,8]naphthyridin-3-yl)-2'-methylspiro[cyclopropane-1,1'-isoindolin]-3'-one **(I-5)** (25.0 mg, yield: 45.1%).

LC-MS, M/Z (ESI): 465.2 [M+H]⁺.

### Example 6: Preparation of 6'-(6-(4,4-difluoropiperidine-1-carbonyl)-1,1a,2,7b-tetrahydro-3H-cyclopropa[c][1,8]naphthyridin-3-yl)-2'-(deuteromethyl)spiro[cyclopropane-1,1'-isoindolin]-3'-one (I-6)

The synthetic route of target compound **1-6** was as follows:

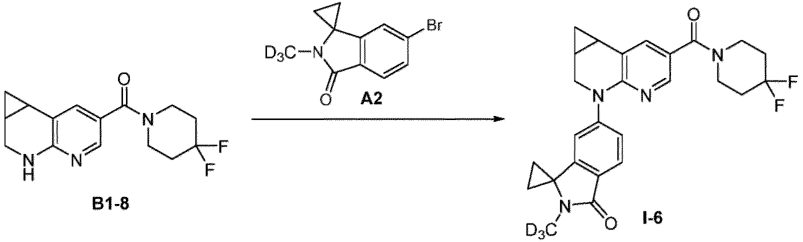

To dioxane (1 mL) were added (4,4-difluoropiperidin-1-yl)(1a,2,3,7b-tetrahydro-1*H-*cyclopropa[*c*][1,8]naphthyridin-6-yl)methanone (35.0 mg, 119 µmol), cesium carbonate (155 mg, 477 µmol), 6'-bromo-2'-(deuteromethyl)spiro[cyclopropane-1,1'-isoindolin]-3'-one (45.7 mg, 179 µmol), bis(dibenzylideneacetone)palladium (6.86 mg, 11.9 µmol), and 4,5-bis(diphenylphosphino)-9,9-dimethylxanthene (69.0 mg, 119 µmol) under nitrogen atmosphere. The reaction mixture was stirred and reacted at 100°C for 12 hours, and then concentrated to obtain a crude product. The crude product was then purified by silica gel column chromatography (petroleum ether: ethyl acetate (V/V) = 5:1 to 1:1) to obtain target compound 6'-(6-(4,4-difluoropiperidine-1-carbonyl)-1,1a,2,7b-tetrahydro-3*H-*cyclopropa[*c*][1,8]naphthyridin-3-yl)-2'-(deuteromethyl)spiro[cyclopropane-1,1'-isoindolin]-3'-one **(I-6)** (26.0 mg, yield: 46.6%).

LC-MS, M/Z (ESI): 468.2 [M+H]⁺.

### Example 7: Preparation of 7-(6-(4,4-difluoropiperidine-1-carbonyl)-1,1a,2,7b-tetrahydro-3H-cyclopropa[c][1,8]naphthyridin-3-yl)-2-(2,2,2-trifluoroethyl)-[1,2,4]triazolo[4,3-a]pyridin-3(2H)-one (I-7)

The synthetic route of target compound **1-7** was as follows:

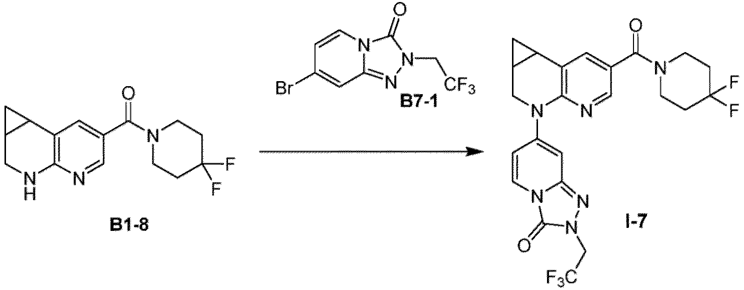

To dioxane (1 mL) were added (4,4-difluoropiperidin-1-yl)(1a,2,3,7b-tetrahydro-1*H-*cyclopropa[*c*][1,8]naphthyridin-6-yl)methanone (50.0 mg, 170 µmol), cesium carbonate (166 mg, 510 µmol), 7-bromo-2-(2,2,2-trifluoroethyl)-[1,2,4]triazolo[4,3-*a*]pyridin-3(2*H*)-one (B7-1 was synthesized with reference to WO2020145250A1) (60.6 mg, 205 µmol), bis(dibenzylideneacetone)palladium (9.8 mg, 1.70 µmol), and 4,5-bis(diphenylphosphino)-9,9-dimethylxanthene (98.6 mg, 1.70 µmol) under nitrogen atmosphere. The reaction mixture was stirred and reacted at 100°C for 12 hours, and then concentrated to obtain a crude product. The crude product was then subjected to preparative high performance liquid chromatography (column: Phenomenex Synergi C₁₈ 150 * 25 mm * 5 µm; solvent: A = water + 0.1 vol% trifluoroformic acid (99%), B = acetonitrile; gradient: 25% to 56% B, 8 min) to obtain target compound 7-(6-(4,4-difluoropiperidine-1-carbonyl)-1,1a,2,7b-tetrahydro-3*H-*cyclopropa[*c*][1,8]naphthyridin-3-yl)-2-(2,2,2-trifluoroethyl)-[1,2,4]triazolo[4,3-*a*]pyridin-3(2*H*)-one **(I-7)** (18.8 mg, yield: 21.6%).

LC-MS, M/Z (ESI): 509.2 [M+H]⁺.

### Example 8: Preparation of 7-(6-(4,4-difluoropiperidine-1-carbonyl)-1,1a,2,7b-tetrahydro-3H-cyclopropa[c][1,8]naphthyridin-3-yl)quinazolin-4(3H)-one (I-8)

The synthetic route of target compound **1-8** was as follows:

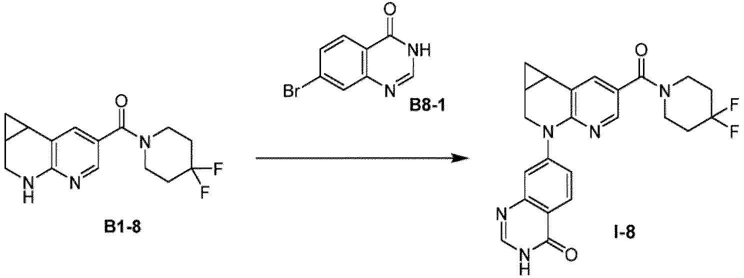

To dioxane (1 mL) were added (4,4-difluoropiperidin-1-yl)(1a,2,3,7b-tetrahydro-1*H-*cyclopropa[*c*][1,8]naphthyridin-6-yl)methanone (50.0 mg, 170 µmol), cesium carbonate (166 mg, 510 µmol), 7-bromoquinazolin-4(3*H*)-one (46.0 mg, 205 µmol), bis(dibenzylideneacetone)palladium (9.8 mg, 1.70 µmol), and 4,5-bis(diphenylphosphino)-9,9-dimethylxanthene (98.6 mg, 1.70 µmol) under nitrogen atmosphere. The reaction mixture was stirred and reacted at 100°C for 12 hours, and then concentrated to obtain a crude product. The crude product was then subjected to preparative high performance liquid chromatography (column: Phenomenex Synergi C₁₈ 150 * 25 mm * 5 µm; solvent: A = water + 0.1 vol% trifluoroformic acid (99%), B = acetonitrile; gradient: 29% to 60% B, 7 min) to obtain target compound 7-(6-(4,4-difluoropiperidine-1-carbonyl)-1,1a,2,7b-tetrahydro-3*H-*cyclopropa[*c*][1,8]naphthyridin-3-yl)quinazolin-4(3*H*)-one **(I-8)** (25.0 mg, yield: 33.0%).

LC-MS, M/Z (ESI): 438.2 [M+H]⁺.

### Example 9: Preparation of 4-(6-(4,4-difluoropiperidine-1-carbonyl)-1,1a,2,7b-tetrahydro-3H-cyclopropa[c][1,8]naphthyridin-3-yl)-2-fluoro-N methylbenzamide (I-9)

The synthetic route of target compound **1-9** was as follows:

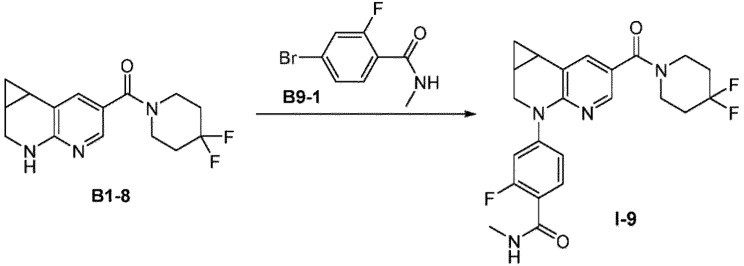

To dioxane (1 mL) were added (4,4-difluoropiperidin-1-yl)(1a,2,3,7b-tetrahydro-1*H-*cyclopropa[c][1,8]naphthyridin-6-yl)methanone (50.0 mg, 170 µmol), cesium carbonate (166 mg, 510 µmol), 4-bromo-2-fluoro-N-methylbenzamide (47.5 mg, 205 µmol), bis(dibenzylideneacetone)palladium (9.8 mg, 1.70 µmol), and 4,5-bis(diphenylphosphino)-9,9-dimethylxanthene (98.6 mg, 1.70 µmol) under nitrogen atmosphere. The reaction mixture was stirred and reacted at 100°C for 12 hours, and then concentrated to obtain a crude product. The crude product was then subjected to preparative high performance liquid chromatography (column: Phenomenex Synergi C₁₈ 150 * 25 mm * 5 µm; solvent: A = water + 0.1 vol% trifluoroformic acid (99%), B = acetonitrile; gradient: 20% to 70% B, 9 min) to obtain target compound 4-(6-(4,4-difluoropiperidine-1-carbonyl)-1,1a,2,7b-tetrahydro-3*H-*cyclopropa[*c*][1,8]naphthyridin-3-yl)-2-fluoro-N methylbenzamide **(I-9)** (30.5 mg, yield: 40.3%).

LC-MS, M/Z (ESI): 445.2 [M+H]⁺.

### Example 10: Preparation of 5-(6-(4,4-difluoropiperidine-1-carbonyl)-1,1a,2,7b-tetrahydro-3H-cyclopropa[c][1,8]naphthyridin-methylpicolinamide (I-10)

The synthetic route of target compound **1-10** was as follows:

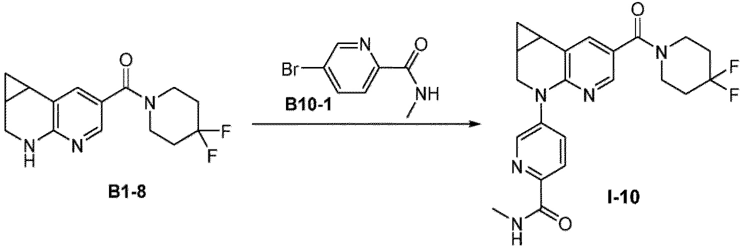

To dioxane (1 mL) were added (4,4-difluoropiperidin-1-yl)(1a,2,3,7b-tetrahydro-1*H-*cyclopropa[*c*][1,8]naphthyridin-6-yl)methanone (50.0 mg, 170 µmol), cesium carbonate (166 mg, 510 µmol), 5-bromo-*N*-methylpicolinamide (44.0 mg, 205 µmol), bis(dibenzylideneacetone)palladium (9.8 mg, 1.70 µmol), and 4,5-bis(diphenylphosphino)-9,9-dimethylxanthene (98.6 mg, 1.70 µmol) under nitrogen atmosphere. The reaction mixture was stirred and reacted at 100°C for 12 hours, and then concentrated to obtain a crude product. The crude product was then subjected to preparative high performance liquid chromatography (column: Phenomenex Synergi C₁₈ 150 * 25 mm * 5 µm; solvent: A = water + 0.1 vol% trifluoroformic acid (99%), B = acetonitrile; gradient: 18% to 75% B, 10 min) to obtain target compound 5-(6-(4,4-difluoropiperidine-1-carbonyl)-1,1a,2,7b-tetrahydro-3*H-*cyclopropa[*c*][1,8]naphthyridin-3-yl)-N-methylpicolinamide **(I-10)** (26.5 mg, yield: 36.4%).

LC-MS, M/Z (ESI): 428.2 [M+H]⁺.

### Example 11: Preparation of 4-(6-(4,4-difluoropiperidine-1-carbonyl)-1,1a,2,7b-tetrahydro-3H-cyclopropa[c][1,8]naphthyridin-3-yl)benzonitrile (I-11)

The synthetic route of target compound **1-11** was as follows:

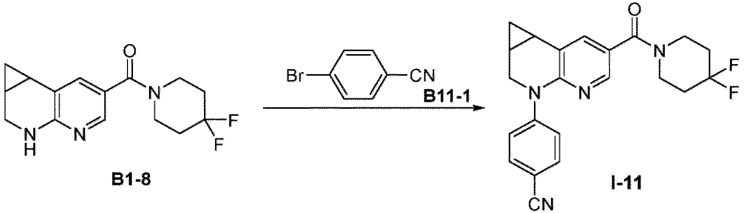

To dioxane (1 mL) were added (4,4-difluoropiperidin-1-yl)(1a,2,3,7b-tetrahydro-1*H-*cyclopropa[*c*][1,8]naphthyridin-6-yl)methanone (50.0 mg, 170 µmol), cesium carbonate (166 mg, 510 µmol), 4-bromobenzonitrile (37.2 mg, 205 µmol), bis(dibenzylideneacetone)palladium (9.8 mg, 1.70 µmol), and 4,5-bis(diphenylphosphino)-9,9-dimethylxanthene (98.6 mg, 1.70 µmol) under nitrogen atmosphere. The reaction mixture was stirred and reacted at 100°C for 12 hours, and then concentrated to obtain a crude product. The crude product was then subjected to preparative high performance liquid chromatography (column: Phenomenex Synergi C₁₈ 150 * 25 mm * 5 µm; solvent: A = water + 0.1 vol% trifluoroformic acid (99%), B = acetonitrile; gradient: 30% to 80% B, 12 min) to obtain target compound 4-(6-(4,4-difluoropiperidine-1-carbonyl)-1,1a,2,7b-tetrahydro-3*H-*cyclopropa[c][1,8]naphthyridin-3-yl)benzonitrile (28.8 mg, yield: 42.8%).

¹H NMR (400 MHz, CDCl₃) δ 8.01 (d, 1H), 7.68 (d, 1H), 7.61 (s, 1H), 7.59 (s, 1H), 7.29 (s, 1H), 7.26 (s, 1H), 3.94 (d, 1H), 3.85-3.63 (m, 5H), 2.04 (ddd, 6H), 1.18 (q, 1H), 1.11 (td, 1H).

LC-MS, M/Z (ESI): 395.2 [M+H]⁺.

### Example 12: Preparation of 6-(6-(4,4-difluoropiperidine-1-carbonyl)-1,1a,2,7b-tetrahydro-3H-cyclopropa[c][1,8]naphthyridin-3-yl)-2-methylisoquinolin-1(2H)-one (I-12)

The synthetic route of target compound **1-12** was as follows:

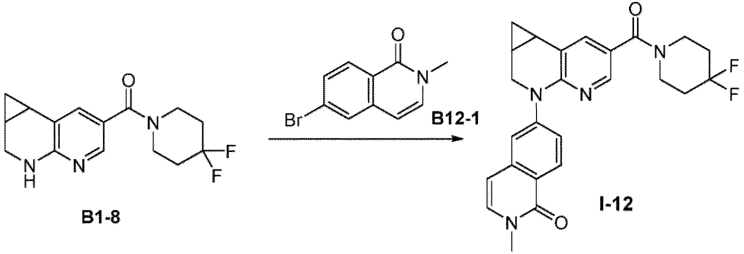

To dioxane (1 mL) were added (4,4-difluoropiperidin-1-yl)(1a,2,3,7b-tetrahydro-1*H-*cyclopropa[*c*][1,8]naphthyridin-6-yl)methanone (50.0 mg, 170 µmol), cesium carbonate (166 mg, 510 µmol), 6-bromo-2-methylisoquinolin-1(2*H*)-one (48.7 mg, 205 µmol), bis(dibenzylideneacetone)palladium (9.8 mg, 1.70 µmol), and 4,5-bis(diphenylphosphino)-9,9-dimethylxanthene (98.6 mg, 1.70 µmol) under nitrogen atmosphere. The reaction mixture was stirred and reacted at 100°C for 12 hours, and then concentrated to obtain a crude product. The crude product was then subjected to preparative high performance liquid chromatography (column: Phenomenex Synergi C₁₈ 150 * 25 mm * 5 µm; solvent: A = water + 0.1 vol% trifluoroformic acid (99%), B = acetonitrile; gradient: 30% to 70% B, 10 min) to obtain target compound 6-(6-(4,4-difluoropiperidine-1-carbonyl)-1,1a,2,7b-tetrahydro-3*H-*cyclopropa[*c*][1,8]naphthyridin-3-yl)-2-methylisoquinolin-1(2*H*)-one **(I-12)** (21.1 mg, yield: 27.5%).

The resulting product was separated by SFC (chromatographic column: Chiralpak AS-3 50 × 4.6 mm I.D., 3 µm; mobile phase: mobile phase A: CO₂, mobile phase B: IPA + ACN (0.05 vol% DEA); isocratic elution: 40 vol% IPA (0.05 vol% DEA) in CO₂; flow rate: 3 mL/min; detector: PDA, column temperature: 35°C; column pressure: 100 Bar). Isomer **1-12A** (RT = 0.816 min) and isomer **1-12B** (RT = 1.477 min) were obtained.

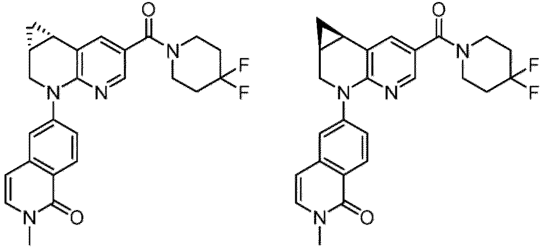

¹H NMR (400 MHz, CDCl₃) δ 8.33 (d, 1H), 8.00 (s, 1H), 7.66 (s, 1H), 7.35 (d, 1H), 7.20 (s, 1H), 7.03 (d, 1H), 6.40 (d, 1H), 3.91 (dd, 2H), 3.81-3.62 (m, 4H), 3.58 (s, 3H), 2.09-1.95 (m, 6H), 1.25 (s, 1H), 1.09 (s, 1H).

LC-MS, M/Z (ESI): 451.2 [M+H]⁺.

### Example 13: Preparation of 6-(6-(4,4-difluoropiperidine-1-carbonyl)-1,1a,2,7b-tetrahydro-3H-cyclopropa[c]quinolin-3-yl)-2-methylphthalazin-1(2H)-one (I-13)

The synthetic route of target compound **1-13** was as follows:

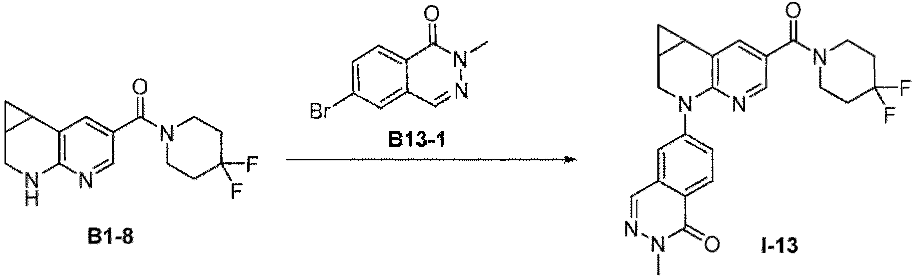

**Compound 1-13** was synthesized with reference to **compound 1-1** by replacing **A1-2** with intermediate **B13-1,** which was synthesized with reference to patent WO2021016333.

¹H NMR (400 MHz, CDCl₃) δ 8.33 (d, 1H), 8.00 (s, 1H), 7.66 (s, 1H), 7.35 (d, 1H), 7.20 (s, 1H), 7.03 (d, 1H), 6.40 (d, 1H), 3.91 (dd, 2H), 3.81-3.62 (m, 4H), 3.58 (s, 3H), 2.09-1.95 (m, 6H), 1.25 (s, 1H), 1.09 (s, 1H).

LC-MS, M/Z (ESI): 451.2 [M+H]⁺.

### Example 14: Preparation of 3-(6-(4,4-difluoropiperidine-1-carbonyl)-1,1a,2,7b-tetrahydro-3H-cyclopropa[c]quinolin-3-yl)-7-methylpyrido[2,3-d]pyridazin-8(7H)-one (I-14)

The synthetic route of target compound **1-14** was as follows:

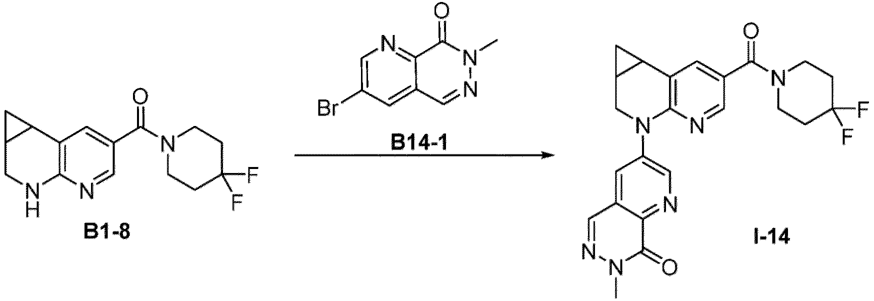

**Compound 1-14** was synthesized with reference to **compound 1-1** by replacing **A1-2** with intermediate **B14-1,** which was synthesized with reference to patent WO2021016333.

¹H NMR (400 MHz, CDCl₃) δ 9.07 (s, 1H), 8.08 (s, 1H), 7.98 (s, 1H), 7.74 (s, 1H), 7.60 (s, 1H), 3.75-4.04 (m, 9H), 2.03-2.13 (m, 5H), 1.18-1.25 (m, 3H).

LC-MS, M/Z (ESI): 452.2 [M+H]⁺.

### Example 15: Preparation of 7-(6-(4,4-difluoropiperidine-1-carbonyl)-1,1a,2,7b-tetrahydro-3H-cyclopropa[c]quinolin-3-yl)-3-methylpyrido[3,2-d]pyrimidin-4(3H)-one (I-15)

The synthetic route of target **compound 1-15** was as follows:

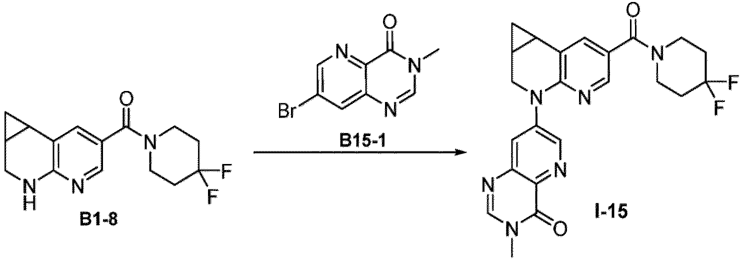

**Compound 1-15** was synthesized with reference to **compound 1-1** by replacing **A1-2** with intermediate **B15-1,** which was synthesized with reference to patent WO2021016333.

¹H NMR (400 MHz, CDCl₃) δ 8.89 (d, 1H), 8.08 (s, 1H), 7.99 (d, 1H), 7.73 (d, 1H), 7.61 (d, 1H), 4.02 (d, 1H), 3.66-3.85 (m, 8H), 2.03-2.13 (m, 5H), 1.25-1.31 (m, 3H).

LC-MS, M/Z (ESI): 452.2 [M+H]⁺.

### Example 16: Preparation of (3-([1,2,4]triazolo[1,5-a]pyridin-7-yl)-1a,2,3,7b-tetrahydro-1H-cyclopropa[c][1,8]naphthyridin-6-yl)(4,4-difluoropiperidin-1-yl)methanone (I-16)

The synthetic route of target compound **1-16** was as follows:

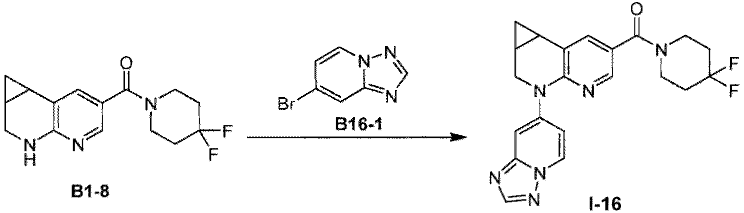

**Compound 1-16** was synthesized with reference to **compound 1-1** by replacing **A1-2** with intermediate **B16-1.**

¹H NMR (400 MHz, CDCl₃) δ 8.37 (d, 1H), 8.24 (s, 1H), 8.05 (d, 1H), 7.72 (d, 1H), 7.28 (d, 1H), 7.07 (dd, 1H), 4.03 (d, 1H), 3.82 (d, 2H), 3.75 (s, 3H), 2.10 (dd, 2H), 2.02 (s, 4H), 1.22 (dd, 1H), 1.15 (td, 1H).

LC-MS, M/Z (ESI): 411.2 [M+H]⁺.

### Example 17: Preparation of (3-([1,2,4]triazolo[4,3-a]pyridin-7-yl)-1a,2,3,7b-tetrahydro-1H-cyclopropa[c][1,8]naphthyridin-6-yl)(4,4-difluoropiperidin-1-yl)methanone(I-17)

The synthetic route of target compound **1-17** was as follows:

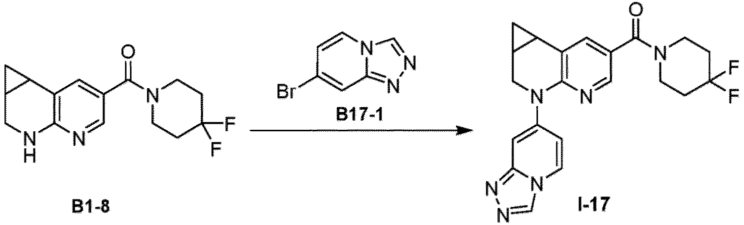

**Compound 1-17** was synthesized with reference to **compound 1-1** by replacing **A1-2** with intermediate **B17-1.**

¹H NMR (400 MHz, CDCl₃) δ 8.67 (s, 1H), 8.05 (d, 1H), 7.91 (d, 1H), 7.70 (d, 1H), 7.24 (s, 1H), 6.94 (d, 1H), 4.03 (d, 1H), 3.81 (d, 2H), 3.75 (s, 3H), 2.11 (dd, 2H), 2.02 (s, 4H), 1.18 (ddd, 2H).

LC-MS, M/Z (ESI): 411.2 [M+H]⁺.

### Example 18: Preparation of 6-(6-(4,4-difluoropiperidine-1-carbonyl)-1,1a,2,7b-tetrahydro-3H-cyclopropa[c][1,8]naphthyridin-3-yl)-2-methyl-1,2-dihydro-3H-pyrrolo[3,4-c]pyridin-3-one (I-18)

The synthetic route of target compound **1-18** was as follows:

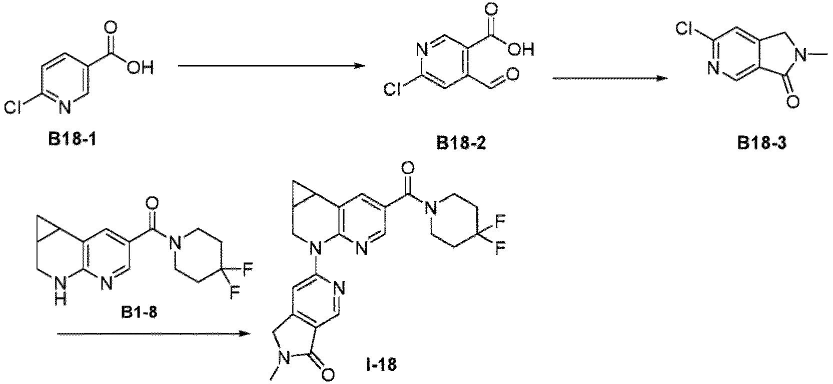

### Step 1: Synthesis of 6-chloro-4-formylnicotinic acid (B18-2)

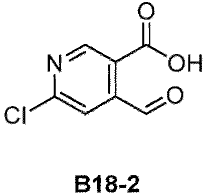

To a solution of 2,2,6,6-tetramethylpiperidine in tetrahydrofuran (50 mL) was added dropwise n-butyllithium (40.6 mL, 102 mmol) at -78°C. After the dropwise addition was completed, the reaction mixture was stirred at -78°C for 30 minutes, and then stirred at -50°C for 30 minutes. A solution of 6-chloronicotinic acid (4 g, 25.4 mmol) in tetrahydrofuran (25 mL) was then added dropwise thereto at -78°C. After the dropwise addition was completed, the reaction mixture was stirred at -78°C for 30 minutes, and then stirred at -50°C for 30 minutes. *N,N-*Dimethylformamide (12 mL, 155.2 mmol) was then added dropwise thereto at -78°C, and the reaction mixture was slowly warmed to room temperature and stirred overnight. The reaction mixture was added with 2 N HCl to quench at 0°C and to adjust the pH to 7, subjected to rotary evaporation to remove most of the tetrahydrofuran solvent, then added with 2 N HCl to adjust the pH to 3 and to precipitate a large amount of solid, and filtered. The filter cake was washed with water (40 mL), dried under reduced pressure, and then dried to obtain 1.61 g of compound 6-chloro-4-formylnicotinic acid **(B18-2)** (1.61 g, yield: 34.2%) as a light gray solid.

LC-MS, M/Z (ESI): 183.8 [M-H]⁻.

### Step 2: Synthesis of 6-chloro-2-methyl-1,2-dihydro-3H-pyrrolo[3,4-c]pyridin-3-one (B18-3)

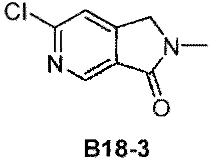

To a suspension of 6-chloro-4-formylnicotinic acid (520 mg, 2.8 mmol) in glacial acetic acid (1.12 mL, 19.6 mmol) were added 40% methylamine aqueous solution (0.36 mL, 3.22 mmol), a solution of HCl in dioxane (4 M, 0.77 mL, 3.08 mmol), and sodium triacetoxyborohydride (891 mg, 4.2 mmol) under nitrogen atmosphere. The reaction mixture was stirred at room temperature for 18 hours, and then stirred at 60°C for 5 hours. 40% methylamine aqueous solution (0.25 mL, 2.24 mmol) and sodium triacetoxyborohydride (475 mg, 2.24 mmol) were then added thereto at room temperature, and the reaction mixture was reacted at room temperature for another 18 hours. The reaction mixture was added with ethyl acetate (30 mL), washed with 1 M sodium carbonate solution (30 mL), washed with water (30 mL), washed with saturated brine (30 mL), dried over anhydrous sodium sulfate, and concentrated to obtain compound 6-chloro-2-methyl-1,2-dihydro-3*H*-pyrrolo[3,4-*c*]pyridin-3-one **(B18-3)** (light yellow solid, 312.2 mg, yield: 61%).

LC-MS, M/Z (ESI): 183.0 [M+H]⁺.

### Step 3: Synthesis of 6-(6-(4,4-difluoropiperidine-1-carbonyl)-1,1a,2,7b-tetrahydro-3H-cyclopropa[c][1,8]naphthyridin-3-yl)-2-methyl-1,2-dihydro-3H-pyrrolo[3,4-c]pyridin-3-one (I-18)

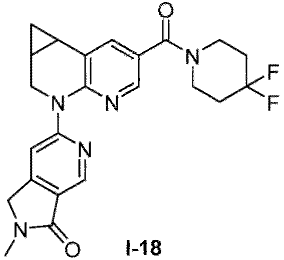

**Compound 1-18** was synthesized with reference to the synthesis of **compound 1-1** by replacing **A1-2** with intermediate **B18-3.**

¹H NMR (400 MHz, DMSO) δ 8.58 (d, 1H), 8.12 (d, 1H), 7.90 (d, 1H), 7.58 (s, 1H), 4.88 (d, 1H), 4.41 (dd, 2H), 3.63 (s, 4H), 3.34 (s, 1H), 3.01 (s, 3H), 2.19 (td, 1H), 2.15-1.99 (m, 5H), 1.09-1.01 (m, 1H), 0.90 (d, 1H).

LC-MS, M/Z (ESI): 440.2 [M+H]⁺.

### Example 19: Preparation of 3'-(6-(4,4-difluoropiperidine-1-carbonyl)-1,1a,2,7b-tetrahydro-3H-cyclopropa[c][1,8]naphthyridin-3-yl)-6'-methylspiro[cyclopropane-1,5'-pyrrolo[3,4-b]pyridin]-7'(6'H)-one (I-19)

The synthetic route of target compound **1-19** was as follows:

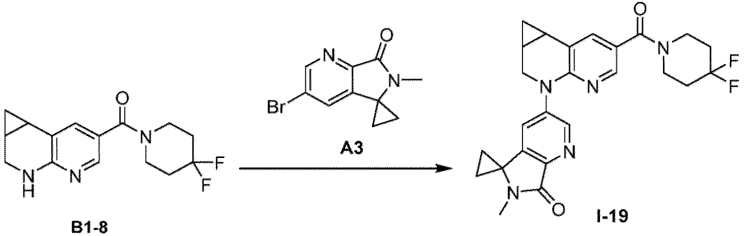

**Compound 1-19** was synthesized with reference to **compound 1-1** by replacing **A1-2** with intermediate **A3.**

¹H NMR (400 MHz, CDCl₃) δ 8.64 (s, 1H), 7.98 (d, 1H), 7.72 (s, 1H), 7.22 (s, 1H), 3.94-3.91 (m, 2H), 3.81-3.76 (m, 2H), 3.25 (s, 3H), 2.07-2.04 (m, 3H), 1.73-1.62 (m, 6H), 1.37 (s, 1H), 1.28-1.23 (m, 3H), 1.19-1.17(m, 1H).

LC-MS, M/Z (ESI): 466.2 [M+H]⁺.

### Example 20: Preparation of 7-(6-(3-fluoro-3-methylazetidine-1-carbonyl)-1a,2-dihydro-1H-cyclopropa[c][1,8]naphthyridin-3(7bH)-yl)-2-methyl-[1,2,4]triazolo[4,3-a]pyridin-3(2H)-one (I-20)

The synthetic route of target compound **1-20** was as follows:

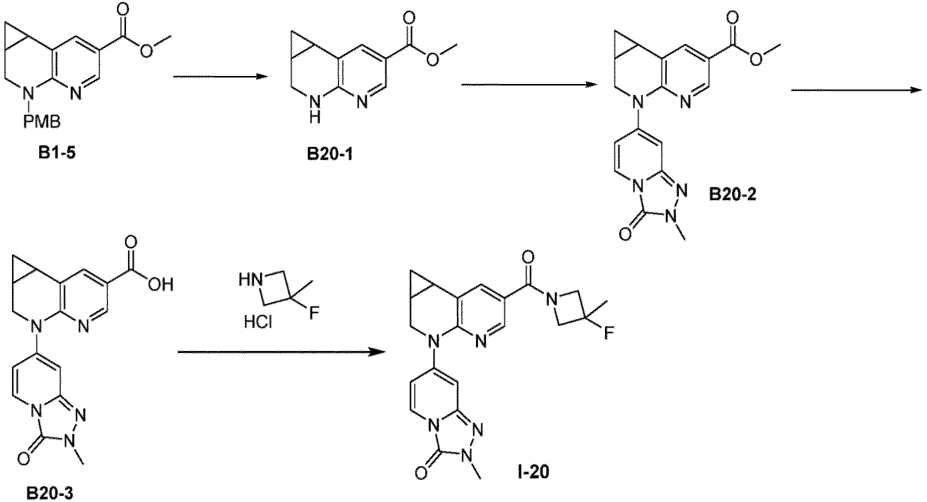

### Step 1: Methyl 1a,2,3,7b-tetrahydro-1H-cyclopropa[c][1,8]naphthyridine-6-carboxylate (B20-1)

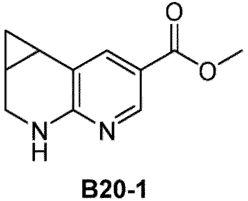

Methyl 3-(4-methoxybenzyl)-1a,2,3,7b-tetrahydro-1*H-*cyclopropa[*c*][1,8]naphthyridine-6-carboxylate (35 g, 108 mmol) was placed in a single-necked flask (500 mL), then trifluoroacetic acid (83 mL) was added thereto, and the reaction mixture was stirred at 60°C for 4 hours. After the reaction was completed, the reaction mixture was subjected to rotary evaporation to remove trifluoroacetic acid. The residue was slowly added with water (500 mL), then added with concentrated hydrochloric acid to adjust the pH to 1, and extracted with ethyl acetate (300 mL * 3). The aqueous phase was then added with sodium carbonate powder to adjust the pH to 9, and extracted with dichloromethane (500 mL * 3). The organic phases were combined, dried over anhydrous sodium sulfate, filtered, and concentrated to obtain methyl 1a,2,3,7b-tetrahydro-1*H*-cyclopropa[*c*][1,8]naphthyridine-6-carboxylate (20.3 g, yield: 92%).

LC-MS, M/Z (ESI): 205.1 [M+H]⁺.

### Step 2: Methyl 3-(2-methyl-3-oxo-2,3-dihydro-[1,2,4]triazolo[4,3-a]pyridin-7-yl)-1a,2,3,7b-tetrahydro-1H-cyclopropa[c][1,8]naphthyridine-6-carboxylate (B20-2)

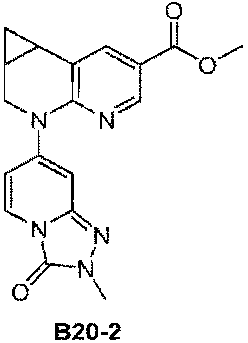

To dioxane (200 mL) were added methyl 1a,2,3,7b-tetrahydro-1*H-*cyclopropa[*c*][1,8]naphthyridine-6-carboxylate (19.4 g, 95 mmol), cesium carbonate (77 g, 237 mmol), 7-bromo-2-methyl-[1,2,4]triazolo[4,3-a]pyridin-3(2*H*)-one (23.83 g, 104 mmol), bis(dibenzylideneacetone)palladium (8.7 g, 9.5 mmol), and 4,5-bis(diphenylphosphino)-9,9-dimethylxanthene (5.5 g, 19.5 mmol) under nitrogen atmosphere. The reaction mixture was stirred and reacted at 85°C for 12 hours, filtered, and the filtrate was concentrated to obtain a crude product. The crude product was added with ethyl acetate (90 mL) and petroleum ether (180 mL), stirred and slurried for 2 hours, and filtered to obtain methyl 3-(2-methyl-3-oxo-2,3-dihydro-[1,2,4]triazolo[4,3-*a*]pyridin-7-yl)-1a,2,3,7b-tetrahydro-1*H-*cyclopropa[*c*][1,8]naphthyridine-6-carboxylate (44 g, crude product).

LC-MS, M/Z (ESI): 352.1 [M+H]⁺.

### Step 3: 3-(2-Methyl-3-oxo-2,3-dihydro-[1,2,4]triazolo[4,3-a]pyridin-7-yl)-1a,2,3,7b-tetrahydro-1H-cyclopropa[c][1,8]naphthyridine-6-carboxylic acid (B20-3)

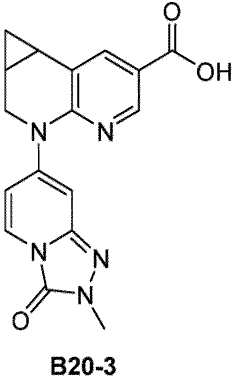

The crude product methyl 3-(2-methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylate (41.3 g, 118 mmol) was dissolved in a mixed solution of tetrahydrofuran (300 mL), methanol (60 mL), and water (60 mL), then lithium hydroxide monohydrate (24.66 g, 588 mmol) was added thereto, and the reaction mixture was stirred at room temperature for 12 hours. After the reaction was completed, the reaction mixture was concentrated to obtain a residue, which was dissolved in water (300 mL) and extracted with ethyl acetate (300 mL * 3). The aqueous phase was then added dropwise with concentrated hydrochloric acid with stirring to adjust the pH to 1, and filtered to obtain 3-(2-methyl-3-oxo-2,3-dihydro-[1,2,4]triazolo[4,3-*a*]pyridin-7-yl)-1a,2,3,7b-tetrahydro-1*H*-cyclopropa[c][1,8]naphthyridine-6-carboxylic acid (27 g, two-step yield: 87%).

LC-MS, M/Z (ESI): 338.1 [M+H]⁺.

### Step 4: 7-(6-(3-Fluoro-3-methylazetidine-1-carbonyl)-1a,2-dihydro-1H-cyclopropa[c][1,8]naphthyridin-3(7bH)-yl)-2-methyl-[1,2,4]triazolo[4,3-a]pyridin-3(2H)-one (I-20)

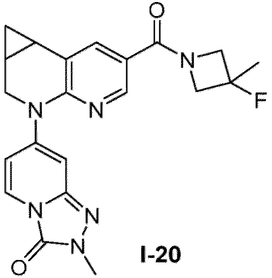

To *N,N-*dimethylformamide (5 mL) were added 3-(2-methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylic acid (280 mg, 830 µmol), 3-fluoro-3-methyl-azetidine hydrochloride (148 mg, 1.18 mmol), 2-(7-azabenzotriazol-1-yl)-*N,N,N',N*'-tetramethyluronium hexafluorophosphate (316 mg, 830 µmol), and *N,N-*diisopropylethylamine (107 mg, 830 µmol) under nitrogen atmosphere, and the reaction mixture was reacted at 25°C for 8 hours under nitrogen atmosphere. After the reaction was completed, the reaction mixture was added with water (50 mL), and then extracted with ethyl acetate (60 mL * 2). The organic phases were combined, dried over anhydrous sodium sulfate, filtered, and concentrated to obtain a crude product. The crude product was purified by high performance liquid chromatography, and the separation method was (column: Phenomenex Synergi C₁₈ 150 * 50 mm * 10 µm; solvent: A = water + 0.1 vol% ammonium bicarbonate (99%), B = acetonitrile; gradient: 16% to 46%, 10 min) to obtain target compound 7-(6-(3-fluoro-3-methylazetidine-1-carbonyl)-1a,2-dihydro-1*H-*cyclopropa[*c*][1,8]naphthyridin-3(7b*H*)-yl)-2-methyl-[1,2,4]triazolo[4,3-*a*]pyridin-3(2*H*)-one **(I-20)** (218 mg, yield: 62.9%).

¹H NMR (400 MHz, CDCl₃) *δ* 8.23 (d, *J* = 2.0 Hz, 1H), 7.96 (d, *J* = 2.0 Hz, 1H), 7.59 (d, *J* = 8.4 Hz, 1H), 6.52-6.54 (m, 2H), 4.39-4.46 (m, 2H), 4.21-4.25 (m, 2H), 3.92-3.95 (m, 1H), 3.71-3.73 (m, 1H), 3.63 (s, 3H), 2.05-2.14 (m, 2H), 1.63-1.68 (m, 3H). 1.11-1.16 (m, 2H).

LC-MS, M/Z (ESI): 409.1 [M+H]⁺.

### Example 21: Preparation of 7-(6-(3,3-difluoropyrrolidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (I-21)

The synthetic route of target compound **1-21** was as follows:

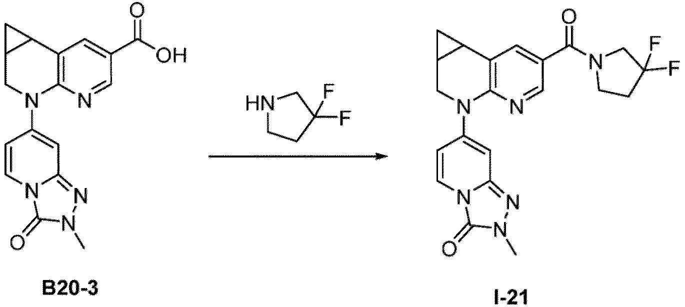

3-(2-Methyl-3-oxo-2,3-dihydro-[1,2,4]triazolo[4,3-a]pyridin-7-yl)-1a,2,3,7b-tetrahydro-1*H*-cyclopropa[*c*][1,8]naphthyridine-6-carboxylic acid (200 mg, 0.6 mmol), 3,3-difluoropyrrolidine (62 mg, 0.7 mmol), and *N,N-*diisopropylethylamine (155 mg, 1.2 mmol) were dissolved in *N,N-*dimethylformamide (10 mL), then 2-(7-azabenzotriazol-1-yl)-*N,N,N',N*'-tetramethyluronium hexafluorophosphate (270 mg, 0.7 mmol) was added thereto, and the reaction mixture was reacted at 25°C for 1 hour. After the reaction was completed, the reaction mixture was diluted with water (20 mL), and extracted three times with ethyl acetate (90 mL). The organic phases were combined, washed with saturated brine (30 mL), dried over anhydrous sodium sulfate, filtered, and concentrated to obtain a crude product. The crude product was separated by reversed-phase high performance liquid chromatography, and the separation method was (column: Phenomenex C₁₈ 75 * 30 mm * 3 µm; solvent: A = water + 1 vol% formic acid (99%) + water, B = acetonitrile; gradient: 28% to 58%, 7 min) to obtain 7-(6-(3,3-difluoropyrrolidine-1-carbonyl)-1,1a,2,7b-tetrahydro-3*H-*cyclopropa[*c*][1,8]naphthyridin-3-yl)-2-methyl-[1,2,4]triazolo[4,3-*a*]pyridin-3(2*H*)-one **(I-21)** (170 mg, yield: 50.4%).

¹H NMR (400 MHz, CDCl₃) *δ* 8.14 (d, 1H), 7.91 (s, 1H), 7.62 (d, 1H), 6.70 (s, 1H), 6.57 (d, 1H), 3.88-3.91 (m, 3H), 3.65-3.68 (m, 3H), 3.48 (s, 3H), 2.40-2.45 (m, 2H), 2.20-2.23 (m, 1H), 2.06-2.08 (m, 1H), 1.04-1.09 (m, 2H).

LC-MS, M/Z (ESI): 427.2 [M+H]⁺.

### Example 22: Preparation of 2-methyl-7-(6-(morpholine-4-carbonyl)-1,1a,2,7b-tetrahydro-3H-cyclopropa[c][1,8]naphthyridin-3-yl)-[1,2,4]triazolo[4,3-a]pyridin-3(2H)-one (I-22)

The synthetic route of target compound **1-22** was as follows:

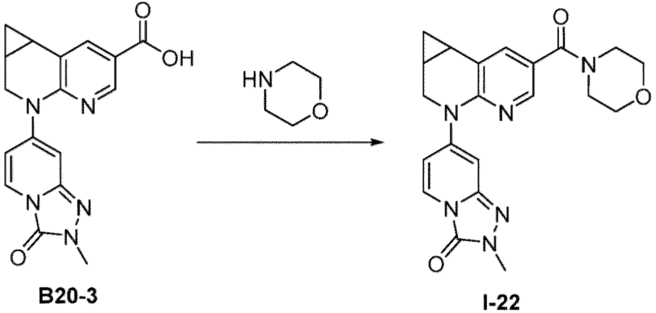

3-(2-Methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylic acid (200 mg, 0.6 mmol), morpholine (62 mg, 0.7 mmol), and *N,N-*diisopropylethylamine (155 mg, 1.2 mmol) were dissolved in *N,N-*dimethylformamide (10 mL), then 2-(7-azabenzotriazol-1-yl)-*N,N,N'N'-*tetramethyluronium hexafluorophosphate (270 mg, 0.7 mmol) was added thereto, and the reaction mixture was reacted at 25°C for 1 hour. After the reaction was completed, the reaction mixture was diluted with water (20 mL), and extracted three times with ethyl acetate (90 mL). The organic phases were combined, washed with saturated brine (30 mL), dried over anhydrous sodium sulfate, filtered, and concentrated to obtain a crude product. The crude product was separated by reversed-phase high performance liquid chromatography, and the separation method was (column: Phenomenex C₁₈ 75 * 30 mm * 3 µm; solvent: A = water + 1 vol% formic acid (99%) + water, B = acetonitrile; gradient: 28% to 58%, 7 min) and lyophilized to obtain 2-methyl-7-(6-(morpholine-4-carbonyl)-1,1a,2,7b-tetrahydro-3*H-*cyclopropa[*c*][1,8]naphthyridin-3-yl)-[1,2,4]triazolo[4,3-*a*]pyridin-3(2*H*)-one **(I-22)** (170 mg, yield: 50.4%).

¹H NMR (400 MHz, CDCl₃) δ 8.08 (d, 1H), 7.71 (d, 1H), 7.58 (dd, 1H), 6.55-6.50 (d, 2H), 3.95 (d, 1H), 3.71-3.63 (m, 12H), 2.01-2.06 (m, 2H), 1.18-1.13 (m, 2H).

LC-MS, M/Z (ESI): 407.2 [M+H]⁺.

### Example 23: Preparation of 7-(6-(3-(difluoromethyl)piperidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (I-23)

The synthetic route of target compound **1-23** was as follows:

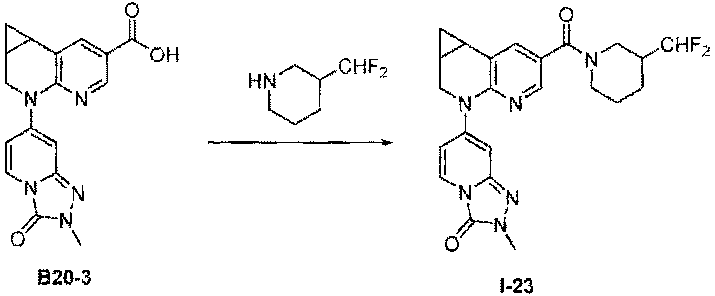

3-(2-Methyl-3-oxo-2,3-dihydro-[1,2,4]triazolo[4,3-a]pyridin-7-yl)-1a,2,3,7b-tetrahydro-1*H*-cyclopropa[*c*][1,8]naphthyridine-6-carboxylic acid (200 mg, 0.6 mmol), 3-(difluoromethyl)piperidine (96 mg, 0.7 mmol), and *N,N-*diisopropylethylamine (155 mg, 1.2 mmol) were dissolved in *N,N-*dimethylformamide (10 mL), then 2-(7-azabenzotriazol-1-yl)-*N,N,N',N'*-tetramethyluronium hexafluorophosphate (270 mg, 0.7 mmol) was added thereto, and the reaction mixture was reacted at 25°C for 1 hour. After the reaction was completed, the reaction mixture was diluted with water (20 mL), and extracted three times with ethyl acetate (90 mL). The organic phases were combined, washed with saturated brine (30 mL), dried over anhydrous sodium sulfate, filtered, and concentrated to obtain a crude product. The crude product was separated by reversed-phase high performance liquid chromatography, and the separation method was (column: Phenomenex C₁₈ 75 * 30 mm * 3 µm; solvent: A = water + 1 vol% formic acid (99%) + water, B = acetonitrile; gradient: 28% to 58%, 7 min) to obtain 7-(6-(3-(difluoromethyl)piperidine-1-carbonyl)-1,1a,2,7b-tetrahydro-3*H-*cyclopropa[*c*][1,8]naphthyridin-3-yl)-2-methyl-[1,2,4]triazolo[4,3-*a*]pyridin-3(2*H*)-one **(I-23)** (144 mg, yield: 53.5%).

¹H NMR (400 MHz, CDCl₃) δ 8.07 (t, 1H), 7.69 (t, 1H), 7.58 (d, 1H), 6.55-6.50 (m, 2H), 5.69 (t, 1H), 3.95 (d, 1H), 3.70 (d, 1H), 3.63 (s, 3H), 3.01 (b, 2H), 2.09-2.01 (m, 4H), 1.60 (b, 1H), 1.58-1.49 (m, 4H), 1.18-1.12 (m, 2H).

LC-MS, M/Z (ESI): 455.2 [M+H]⁺.

### Example 24: Preparation of 7-(6-(3,3-difluoropiperidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (I-24)

The synthetic route of target compound **1-24** was as follows:

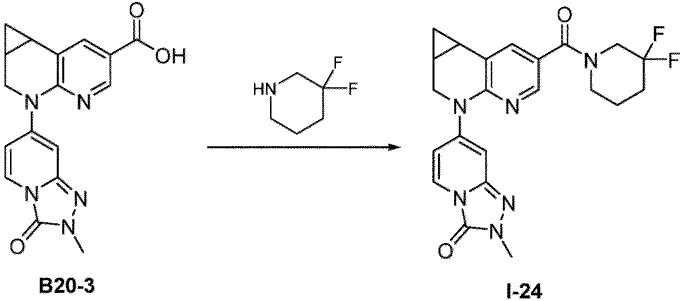

3-(2-Methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylic acid (200 mg, 593 µmol), 3,3-difluoropiperidine (108 mg, 889 µmol), and *N,N-*diisopropylethylamine (153 mg, 1.18 mmol) were dissolved in *N,N-*dimethylformamide (10 mL), then 2-(7-azabenzotriazol-1-yl)-*N,N,N',N*'-tetramethyluronium hexafluorophosphate (271 mg, 711 µmol) was added thereto, and the reaction mixture was reacted at 25°C for 1 hour. After the reaction was completed, the reaction mixture was diluted with water (20 mL), and extracted three times with ethyl acetate (10 mL). The organic phases were combined, washed with saturated brine (30 mL), dried over anhydrous sodium sulfate, filtered, and concentrated to obtain a crude product. The crude product was subjected to column chromatography (DCM/MeOH (v/v) = 15/1) to obtain target compound 7-(6-(3,3 -difluoropiperidine-1-carbonyl)-1,1a,2,7b-tetrahydro-3*H-*cyclopropa[*c*][1,8]naphthyridin-3-yl)-2-methyl-[1,2,4]triazolo[4,3-*a*]pyridin-3(2*H*)-one **(I-24)** (120 mg, yield: 46%).

¹H NMR (400 MHz, CDCl₃) δ 8.09 (d, *J* = 2.2 Hz, 1H), 7.70 (d, *J* = 2.2 Hz, 1H), 7.59 (d, *J* = 7.6 Hz, 1H), 6.55 (dd, *J* = 7.6, 2.0 Hz, 1H), 6.51 (d, *J* = 1.5 Hz, 1H), 3.94 (d, *J* = 11.1 Hz, 1H), 3.79 (d, *J* = 9.2 Hz, 2H), 3.70 (dd, *J* = 10.9, 1.4 Hz, 2H), 3.63 (s, 4H), 2.14-2.04 (m, 4H), 1.89-1.81 (m, 2H), 1.19-1.10 (m, 2H).

LC-MS, M/Z (ESI): 441.1 [M+H]⁺.

### Example 25: Preparation of 7-(6-(4,4-difluoropiperidine-1-carbonyl)-1,1a,2,7b-tetrahydro-3H-cyclopropa[c][1,8]naphthyridin-3-yl)-2-(methyl-d₃)-[1,2,4]triazolo[4,3-a]pyridin-3(2H)-one (I-25)

The synthetic route of target compound **1-25** was as follows:

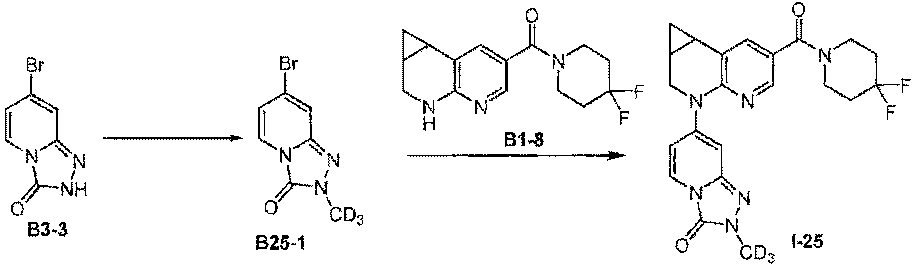

**Compound 1-25** was synthesized with reference to **compound 1-3** by replacing iodomethane with deuterated iodomethane, LC-MS, M/Z (ESI): 444.2 [M+H]⁺.

¹H NMR (400 MHz, DMSO) δ 8.03 (d, 1H), 7.80 (s, 1H), 7.61 (d, 1H), 6.68 (d, 1H), 6.58 (dd, 1H), 3.90 (d, 1H), 3.66-3.59 (m, 5H), 2.18-2.16 (m, 1H), 2.08-1.99 (m, 5H), 1.06 (t, 2H).

The product **1-25** was separated by SFC (chromatographic column: Chiralpak AD-3 50 × 4.6 mm I.D., 3 µm; mobile phase: mobile phase A: CO₂, mobile phase B: IPA + ACN (0.05 vol% DEA); isocratic elution: 50 vol% IPA + ACN (0.05 vol% DEA) (in CO₂); flow rate: 3 mL/min; detector: PDA, column temperature: 35°C; column pressure: 100 Bar). Isomer **I-25A** (RT = 0.764 min) and isomer **I-25B** (RT = 1.702 min) were obtained.

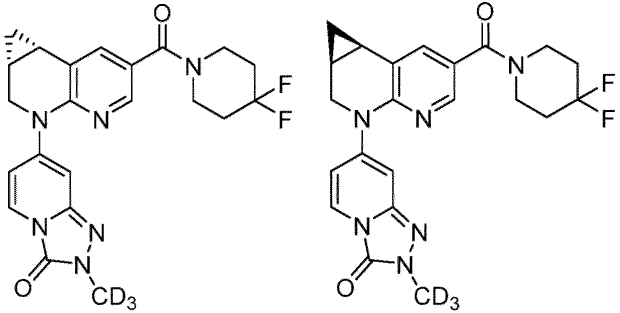

### Example 26: Preparation of 2-methyl-7-(6-(3-(trifluoromethyl)piperidine-1-carbonyl)-1,1a,2,7b-tetrahydro-3H-cyclopropa[c][1,8]naphthyridin-3-yl)-[1,2,4]triazolo[4,3-a]pyridin-3(2H)-one (I-26)

The synthetic route of target compound 1-26 was as follows:

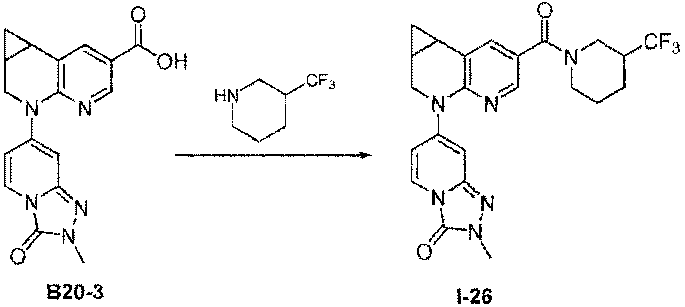

3-(2-Methyl-3-oxo-2,3-dihydro-[1,2,4]triazolo[4,3-a]pyridin-7-yl)-1a,2,3,7b-tetrahydro-1*H*-cyclopropa[*c*][1,8]naphthyridine-6-carboxylic acid (200 mg, 593 µmol), 3-(trifluoromethyl)piperidine (136 mg, 889 µmol), and *N,N-*disopropylethylamine (153 mg, 1.18 mmol) were dissolved in *N,N-*dimethylformamide (10 mL), then 2-(7-azabenzotriazol-1-yl)-*N,N,N',N*'-tetramethyluronium hexafluorophosphate (271 mg, 711 µmol) was added thereto, and the reaction mixture was reacted at 25°C for 1 hour. After the reaction was completed, the reaction mixture was diluted with water (20 mL), and extracted three times with ethyl acetate (10 mL). The organic phases were combined, washed with saturated brine (30 mL), dried over anhydrous sodium sulfate, filtered, and concentrated to obtain a crude product. The crude product was subjected to column chromatography (DCM/MeOH (v/v) = 15/1) to obtain the target compound **(I-26)** (130 mg, yield: 50%).

¹H NMR (400 MHz, CDCl₃) δ 8.06 (t, *J* = 2.0 Hz, 1H), 7.68 (t, *J* = 1.9 Hz, 1H), 7.58 (dd, *J* = 7.6, 0.5 Hz, 1H), 6.54 (dd, *J* = 7.6, 2.0 Hz, 1H), 6.51 (d, *J* = 1.4 Hz, 1H), 3.94 (d, *J* = 11.0 Hz, 1H), 3.70 (d, *J* = 10.4 Hz, 1H), 3.63 (s, 3H), 2.98 (s, 2H), 2.32 (s, 1H), 2.09 (ddd, *J* = 11.7, 10.3, 7.3 Hz, 3H), 1.84 (s, 1H), 1.68 (d, *J* = 10.3 Hz, 1H), 1.66-1.45 (m, 3H), 1.19-1.10 (m, 2H).

LC-MS, M/Z (ESI): 473.1 [M+H]⁺.

### Example 27: Preparation of 7-(6-(3-fluoropiperidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (I-27)

The synthetic route of target compound **1-27** was as follows:

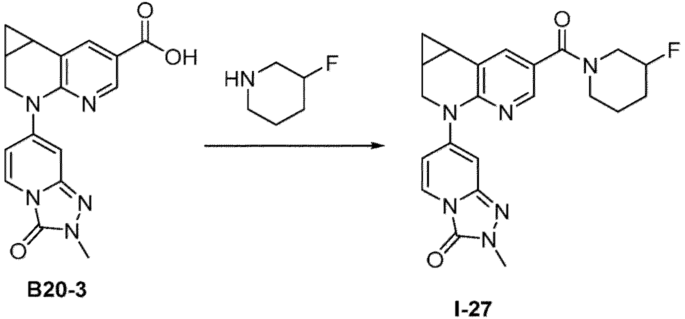

To *N,N-*dimethylformamide (6 mL) were added 3-(2-methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylic acid (200 mg, 0.59 mmol), 3-fluoropiperidine hydrochloride (83 mg, 0.59 mmol), *N,N-*diisopropylethylamine (307 mg, 2.37 mmol), and 2-(7-azabenzotriazol-1-yl)-*N,N,N',N*'-tetramethyluronium hexafluorophosphate (271 mg, 0.71 mmol). The reaction mixture was reacted at room temperature for 2 hours, added with water (30 mL), and then extracted with dichloromethane (50 mL * 2). The organic phases were combined, washed with saturated brine (30 mL), dried over anhydrous sodium sulfate, filtered, and concentrated to obtain a crude product, which was then purified by silica gel column chromatography (ethyl acetate: methanol (V/V) = 20:1) to obtain compound 7-(6-(3-fluoropiperidine-1-carbonyl)-1,1a,2,7b-tetrahydro-3*H*-cyclopropa[*c*][1,8]naphthyridin-3-yl)-2-methyl-[1,2,4]triazolo[4,3-*a*]pyridin-3(2*H*)-one (I-27) (196 mg, yield: 78.4%).

¹H NMR (400 MHz, DMSO) δ 7.96 (d, 1H), 7.75 (d, 1H), 7.62 (d, 1H), 6.68 (d, 1H), 6.59 (ddd, 1H), 4.77 (d, 1H), 3.91 (d, 2H), 3.65 (dd, 1H), 3.49 (s, 5H), 3.15 (s, 1H), 2.19 (dt, 1H), 2.08 (dd, 1H), 1.90 (d, 2H), 1.77-1.66 (m, 1H), 1.54 (s, 1H), 1.12-1.03 (m, 2H).

LC-MS, M/Z (ESI): 423.3 [M+H]⁺.

### Example 28: Preparation of 7-(6-(6-azaspiro[2.5]octane-6-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (I-28)

The synthetic route of target compound **1-28** was as follows:

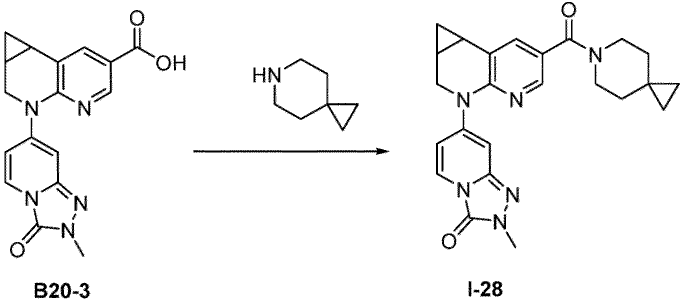

To *N,N-*dimethylformamide (5 mL) were added 3-(2-methyl-3-oxo-2,3-dihydro-[1,2,4]triazolo[4,3-*a*]pyridin-7-yl)-1a,2,3,7b-tetrahydro- 1*H*-cyclopropa[*c*][1,8]naphthyridine-6-carboxylic acid (120 mg, 0.36 mmol), 6-azaspiro[2.5]octane (40 mg, 0.36 mmol), *N,N-*diisopropylethylamine (184 mg, 1.42 mmol), and 2-(7-azabenzotriazol-1-yl)-*N,N,N',N'-*tetramethyluronium hexafluorophosphate (162 mg, 0.43 mmol). The reaction mixture was reacted at room temperature for 2 hours, added with water (30 mL), and then extracted with dichloromethane (50 mL * 2). The organic phases were combined, washed with saturated brine (30 mL), dried over anhydrous sodium sulfate, filtered, and concentrated to obtain a crude product, which was then purified by silica gel column chromatography (dichloromethane: methanol (V/V) = 30:1) to obtain compound 7-(6-(6-azaspiro[2.5]octane-6-carbonyl)-1,1a,2,7b-tetrahydro-3*H*-cyclopropa[*c*][1,8]naphthyridin-3-yl)-2-methyl-[1,2,4]triazolo[4,3-*a*]pyridin-3(2*H*)-one **(I-28)** (82 mg, yield: 53.6%).

¹H NMR (400 MHz, DMSO) δ 7.98 (d, 1H), 7.77 (d, 1H), 7.61 (d, 1H), 6.67 (d, 1H), 6.59 (dd, 1H), 3.90 (d, 1H), 3.64 (dd, 2H), 3.48 (s, 5H), 2.19 (dd, 1H), 2.07 (dd, 1H), 1.35 (s, 4H), 1.25 (dd, 1H), 1.06 (t, 2H), 0.34 (s, 4H).

LC-MS, M/Z (ESI): 431.2 [M+H]⁺.

### Example 29: Preparation of 7-(6-(3-azabicyclo[3.1.0]hexane-3-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (I-29)

The synthetic route of target compound **1-29** was as follows:

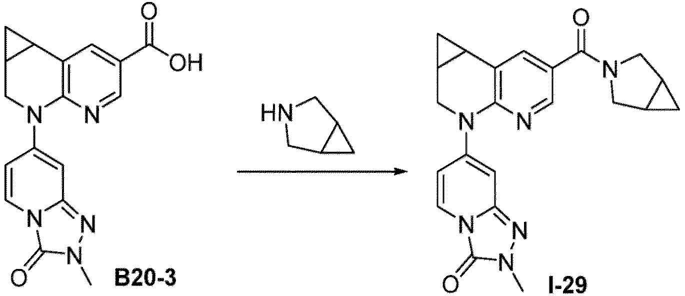

To *N,N-*dimethylformamide (5 mL) were added 3-(2-methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylic acid (200 mg, 0.59 mmol), 3-azabicyclo[3.1.0]hexane (49 mg, 0.59 mmol), *N,N-*diisopropylethylamine (307 mg, 2.37 mmol), and 2-(7-azabenzotriazol-1-yl)-*N,N,N,N'-*tetramethyluronium hexafluorophosphate. The reaction mixture was reacted at room temperature for 2 hours, added with water (30 mL), and then extracted with dichloromethane (50 mL * 2). The organic phases were combined, washed with saturated brine (30 mL), dried over anhydrous sodium sulfate, filtered, and concentrated to obtain a crude product, which was then purified by silica gel column chromatography (ethyl acetate: methanol (V/V) = 25:1) to obtain compound 7-(6-(3-azabicyclo[3.1.0]hexane-3-carbonyl)-1,1a,2,7b-tetrahydro-3*H-*cyclopropa[*c*][1,8]naphthyridin-3-yl)-2-methyl-[1,2,4]triazolo[4,3-*a*]pyridin-3(2*H*)-one **(I-29)** (158 mg, yield: 66.1%).

¹H NMR (400 MHz, DMSO) δ 8.06 (d, 1H), 7.82 (d, 1H), 7.61 (d, 1H), 6.68 (d, 1H), 6.57 (dd, 1H), 3.93 (dd, 2H), 3.80-3.61 (m, 2H), 3.48 (s, 3H), 3.43 (d, 2H), 2.18 (dd, 1H), 2.11-2.03 (m, 1H), 1.54 (d, 2H), 1.11-0.99 (m, 2H), 0.64 (dd, 1H), 0.07 (s, 1H).

LC-MS, M/Z (ESI): 403.2 [M+H]⁺.

### Example 30: Preparation of 7-(6-((R)-3-(fluoromethyl)piperidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (I-30)

The synthetic route of target compound **1-30** was as follows:

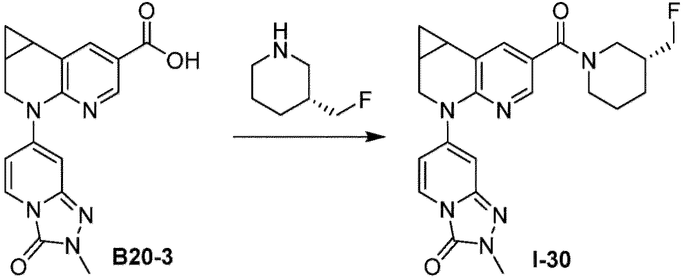

3-(2-Methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylic acid (200 mg, 0.6 mmol), (*R*)-3-(fluoromethyl)piperidine (70 mg, 0.6 mmol), and *N,N-*diisopropylethylamine (155 mg, 1.2 mmol) were dissolved in *N,N-*dimethylformamide (10 mL), then 2-(7-azabenzotriazol-1-yl)-*N,N,N',N'*-tetramethyluronium hexafluorophosphate (270 mg, 0.7 mmol) was added thereto, and the reaction mixture was reacted at 25°C for 1 hour. After the reaction was completed, the reaction mixture was diluted with water (20 mL), and extracted three times with ethyl acetate (90 mL). The organic phases were combined, washed with saturated brine (30 mL), dried over anhydrous sodium sulfate, filtered, and concentrated to obtain a crude product. The crude product was separated by reversed-phase high performance liquid chromatography, and the separation method was (column: Phenomenex C₁₈ 75 * 30 mm * 3 µm; solvent: A = water + 1 vol% formic acid (99%) + water, B = acetonitrile; gradient: 28% to 58%, 7 min) to obtain 7-(6-((*R*)-3-(fluoromethyl)piperidine-1-carbonyl)-1,1a,2,7b-tetrahydro-3*H-*cyclopropa[*c*][1,8]naphthyridin-3-yl)-2-methyl-[1,2,4]triazolo[4,3-*a*]pyridin-3(2*H*)-one **(I-30)** (183 mg, yield: 70.7%).

¹H NMR (400 MHz, CDCl₃) δ 8.07 (t, 1H), 7.69 (s, 1H), 7.58 (d, 1H), 6.56-6.54 (m, 1H), 6.45 (t, 1H), 4.36 (d, 3H), 3.95 (d, 1H), 3.69 (d, 1H), 3.63 (s, 3H), 3.01 (b, 2H), 2.09-1.78 (m, 6H), 1.60 (b, 1H), 1.40 (b, 1H), 1.19-1.10 (m, 2H).

LC-MS, M/Z (ESI): 437.2 [M+H]⁺.

### Example 31: Preparation of 7-(6-((S)-3-(fluoromethyl)piperidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (I-31)

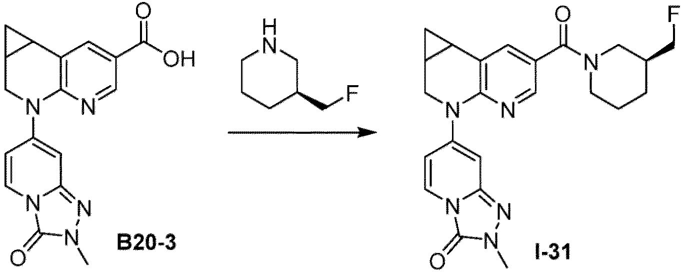

3-(2-Methyl-3-oxo-2,3-dihydro-[1,2,4]triazolo[4,3-*a*]pyridin-7-yl)-1a,2,3,7b-tetrahydro-1*H*-cyclopropa[c][1,8]naphthyridine-6-carboxylic acid (200 mg, 0.6 mmol), (*S*)-3-(fluoromethyl)piperidine (70 mg, 0.6 mmol), and *N,N-*diisopropylethylamine (155 mg, 1.2 mmol) were dissolved in *N,N-*dimethylformamide (10 mL), then 2-(7-azabenzotriazol-1-yl)-*N,N,N',N'*-tetramethyluronium hexafluorophosphate (270 mg, 0.7 mmol) was added thereto, and the reaction mixture was reacted at 25°C for 1 hour. After the reaction was completed, the reaction mixture was diluted with water (20 mL), and extracted three times with ethyl acetate (90 mL). The organic phases were combined, washed with saturated brine (30 mL), dried over anhydrous sodium sulfate, filtered, and concentrated to obtain a crude product. The crude product was separated by reversed-phase high performance liquid chromatography, and the separation method was (column: Phenomenex C₁₈ 75 * 30 mm * 3 µm; solvent: A = water + 1 vol% formic acid (99%) + water, B = acetonitrile; gradient: 28% to 58%, 7 min) to obtain 7-(6-((*S*)-3-(fluoromethyl)piperidine-1-carbonyl)-1,1a,2,7b-tetrahydro-3*H-*cyclopropa[*c*][1,8]naphthyridin-3-yl)-2-methyl-[1,2,4]triazolo[4,3-*a*]pyridin-3(2*H*)-one **(I-31)** (white solid, 206 mg, yield: 79.6%).

¹H NMR (400 MHz, CDCl₃) δ 8.07 (t, 1H), 7.69 (s, 1H), 7.58 (d, 1H), 6.56-6.54 (m, 1H), 6.45 (t, 1H), 4.36 (d, 3H), 3.95 (d, 1H), 3.69 (d, 1H), 3.63 (s, 3H), 3.01 (b, 2H), 2.09-1.78 (m, 6H), 1.60 (b, 1H), 1.40 (b, 1H), 1.19-1.10 (m, 2H).

LC-MS, M/Z (ESI): 437.2 [M+H]⁺.

### Example 32: Preparation of 7-(6-(4,4-difluoropiperidine-1-carbonyl)-1,1a,2,7b-tetrahydro-3H-cyclopropa[c][1,8]naphthyridin-3-yl)-2-ethyl-[1,2,4]triazolo[4,3-a]pyridin-3(2H)-one (I-32)

The synthetic route of target **compound 1-32** was as follows:

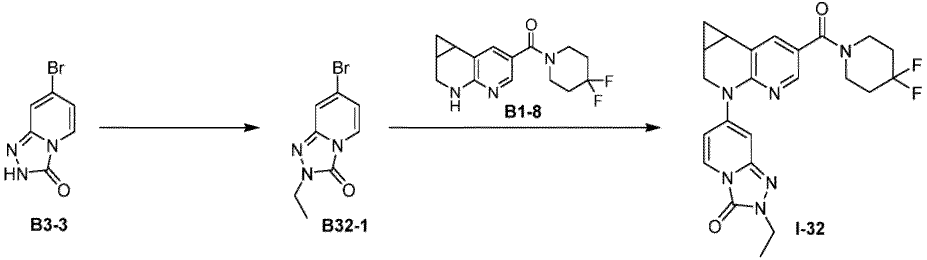

### Step 1: Synthesis of 7-bromo-2-ethyl-[1,2,4]triazolo[4,3-a]pyridin-3(2H)-one (B32-1)

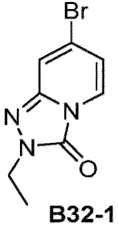

7-Bromo-[1,2,4]triazolo[4,3-*a*]pyridin-3(2*H*)-one **(B3-3)** (100 mg, 467 µmol) was dissolved in *N,N*-dimethylformamide (5 mL), then cesium carbonate (457 mg, 1.40 mmol) was added thereto, and iodoethane (219 mg, 1.40 mmol) was added thereto at 0 to 10°C under nitrogen atmosphere. After the addition was completed, the reaction mixture was reacted at 25°C for 2 hours, poured into water (10 mL), and extracted with ethyl acetate (20 mL × 3). The organic phases were combined, washed with saturated brine (20 mL), dried over anhydrous sodium sulfate, filtered, and concentrated to obtain compound 7-bromo-2-ethyl-[1,2,4]triazolo[4,3-*a*]pyridin-3(2*H*)-one **(B32-1)** (113 mg, yield: 99.4%).

LC-MS, M/Z (ESI): 242.1 [M+H]⁺.

### Step 2: Synthesis of 7-(6-(4,4-difluoropiperidine-1-carbonyl)-1,1a,2,7b-tetrahydro-3H-cyclopropa[c][1,8]naphthyridin-3-yl)-2-ethyl-[1,2,4]triazolo[4,3-a]pyridin-3(2H)-one (I-32)

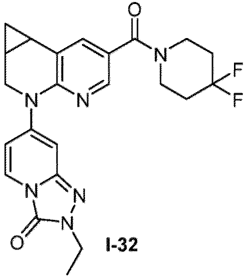

To 1,4-dioxane (10 mL) were added (4,4-difluoropiperidin-1-yl)(1a,2,3,7b-tetrahydro-1*H*-cyclopropa[*c*][1,8]naphthyridin-6-yl)methanone (145 mg, 493 µmol), cesium carbonate (201 mg, 477 µmol), 7-bromo-2-ethyl-[1,2,4]triazolo[4,3-*a*]pyridin-3(2*H*)-one (60.0 mg, 247 µmol), bis(dibenzylideneacetone)palladium (22.6 mg, 24.7 µmol), and 4,5-bis(diphenylphosphino)-9,9-dimethylxanthene (28.5 mg, 49.3 µmol) under nitrogen atmosphere, and the reaction mixture was stirred and reacted at 100°C for 2 hours. After the reaction was completed, the reaction mixture was poured into water (10 mL), and extracted with ethyl acetate (10 mL × 3). The organic phases were combined, washed with saturated brine (10 mL), dried over anhydrous sodium sulfate, filtered, and concentrated to obtain a crude product. The crude product was then subjected to preparative high performance liquid chromatography (column: Phenomenex Synergi C₁₈ 150 * 25 mm * 5 µm; solvent: A = water + 0.1 vol% formic acid (99%), B = acetonitrile; gradient: 30% to 60% B, 7 min) to obtain compound 7-(6-(4,4-difluoropiperidine-1-carbonyl)-1,1a,2,7b-tetrahydro-3*H-*cyclopropa[*c*][1,8]naphthyridin-3-yl)-2-ethyl-[1,2,4]triazolo[4,3-*a*]pyridin-3(2*H*)-one **(I-32)** (80.0 mg, yield: 71.2%).

¹H NMR (400 MHz, DMSO *d*₆) *δ* = 8.04 (d, 1H), 7.82 (d, 1H), 7.63 (d, 1H), 6.72 (s, 1H), 6.57-6.60 (m, 1H), 3.87-3.93 (m, 3H), 3.61-3.67 (m, 5H), 2.15-2.18 (m, 1H), 2.01-2.09 (m, 5H), 1.28 (t, 3H), 1.06-1.09 (m, 2H).

LC-MS, M/Z (ESI): 455.2 [M+H]⁺.

### Test example 1: Inhibition test of compounds on 15-PGDH enzyme

15-PGDH (R&D Systems, Cat. No.: 5660-DH-010) was prepared to twice the final concentration, *i.e*., 30 nM, using Assay Buffer (50 mM Tris-HCl, pH 7.5, 0.01vol% Tween 20). The obtained mixture was then added to a 384-well white plate (Cisbio Bioassays, Cat. No. 66PL3 84025) at 8 µL/well. Negative control wells (with Assay Buffer only and without enzyme) were set. The compound was then prepared to 4 times the final concentration using Assay Buffer, *i.e.,* diluted 3-fold to 10 concentrations starting from 4000 nM. The obtained mixture was added to the above white plate at 4 µL/well, mixed well, centrifuged at 1000 rpm for 1 minute, and incubated at 25°C for 10 minutes. Both positive control wells (with 15-PGDH only) and negative control wells (without 15-PGDH) were set. A mixture of NAD⁺ (Select, Cat. No. S2518) and PGE₂ (R&D Systems, Cat. No.: 2296/10) was then prepared using Assay Buffer. NAD⁺ and PGE₂ were prepared to four times their final concentrations, *i.e.,* 2 mM and 0.12 mM, respectively, using Assay Buffer. The obtained mixture was then added to the above white plate at 4 µL/well, mixed well, centrifuged at 1000 rpm for 1 minute, and incubated at 25°C for 30 minutes for the reaction. The fluorescence was detected at an excitation wavelength of 340 nm and an emission wavelength of 485 nm using instrument TECAN SPARK 20M. The IC₅₀ value was calculated using four-parameter fitting with GraphPad Prism 8.0.

**Table 1: Inhibition test results of compounds on 15-PGDH**

| **Compound No.** | **IC₅₀ (nM)** |
|---|---|
| Compound I-1 | 4.3 |
| Compound I-2 | 5.15 |
| Compound I-19 | 3.72 |
| Compound I-3 | 4.2 |
| Compound I-3A | 12.16 |
| Compound I-3B | 3.58 |
| Compound I-9 | 9.51 |
| Compound I-11 | 3.8 |
| Compound I-12 | 2.1 |
| Compound I-12A | 1.52 |
| Compound I-12B | 7.42 |
| Compound I-13 | 4.03 |
| Compound I-14 | 8.6 |
| Compound I-15 | 5.7 |
| Compound I-16 | 5.4 |
| Compound I-17 | 4.5 |
| Compound I-18 | 19.98 |
| Compound I-25 | 2.59 |
| Compound I-25A | 15.8 |
| Compound I-25B | 3.78 |
| Compound I-20 | 2.83 |
| Compound I-21 | 4.95 |
| Compound I-22 | 10.17 |
| Compound I-23 | 4.26 |
| Compound I-24 | 4.23 |
| Compound I-26 | 9.9 |
| Compound I-27 | 5.7 |
| Compound I-28 | 1.71 |
| Compound I-29 | 5.3 |
| Compound I-30 | 6.0 |
| Compound I-31 | 4.1 |
| Compound I-32 | 6.3 |

The experimental results show that the compounds of the present disclosure have a significant inhibitory effect on 15-PGDH.

### Test example 2: Effect of compounds on PGE₂ levels in A549 cell supernatant

A549 cells (Procell, Wuhan) were cultured in F12K + 10% FBS. The cells with good status in log phase were selected for the experiment, digested, counted, and inoculated into a 24-well plate at 8000 cells per well. The cells were cultured in a 37°C, 5% CO₂ incubator overnight. After adherence, the cells were transferred to a culture medium containing 0.5% FBS and cultured for about 10 hours. IL-1β was added to each well (final concentration of 20 ng/mL, 1 mL per well), and a control group (without IL-1β) was set up. After stimulation with IL-1β for about 24 hours, the cell culture medium of each well was aspirated and discarded, and the wells were gently washed with fresh culture medium containing 0.5% FBS. 400 µL of culture medium containing the compound of different concentrations (20 nM and 2500 nM) was added to each well and cultured for about 12 hours. The supernatant was collected and PGE₂ was detected using ELISA kit (R&D Systems, Cat. No.: KGE004B).

**Table 2: Fold increase of PGE₂ in A549 cell supernatant by compounds**

| **Compound No./concentration** | **20 nM** |
|---|---|
| Compound I-1 | 4.3 |
| Compound I-2 | 5.23 |
| Compound I-19 | 4.72 |
| Compound I-3A | 0.9 |
| Compound I-3B | 4.8 |
| Compound I-11 | 1.88 |
| Compound I-12 | 5.44 |
| Compound I-13 | 6.1 |
| Compound I-14 | 2.3 |
| Compound I-15 | 3.7 |
| Compound I-16 | 3.7 |
| Compound I-17 | 2.2 |
| Compound I-25A | 0.9 |
| Compound I-25B | 4.4 |
| Compound I-20 | 2.0 |
| Compound I-21 | 2.3 |
| Compound I-23 | 2.93 |
| Compound I-24 | 2.95 |
| Compound I-26 | 4.82 |
| Compound I-27 | 2.60 |
| Compound I-28 | 4.05 |
| Compound I-29 | 2.3 |
| Compound I-30 | 2.72 |
| Compound I-31 | 2.90 |
| Compound I-32 | 3.7 |

The experimental results show that the compounds of the present disclosure can significantly increase the production of PGE₂.

### Test example 3: Efficacy test on mouse IPF prevention model

Male mice were adaptively fed for 1 to 2 weeks and after reaching the standard weight (25 g), the IPF model (idiopathic pulmonary fibrosis model) was induced with a certain dose of Bleomycin. On the day of modeling, the animals were randomly divided into a model group and an administration group according to their weight, in which the administration groups were administered Nintedanib (60 mg/kg, once a day (qd)) and compound I-3B of the present disclosure (2.5 mg/kg, twice a day (bid)) by oral gavage for 21 consecutive days while the vehicle control group was administered blank vehicle. During the administration period, the animals were weighed every 3 days. At the end of the last day of administration, the animals were euthanized. The lungs were removed from the thyroid cartilage (without perfusion), slowly perfused with 10% formalin until both lungs were filled, and fixed in 10% formalin 5 to 10 times the volume of the tissues after the trachea was ligated. The left lung tissues were embedded in paraffin wax, sectioned, and stained with HE and Masson Trichrome. The sections were subjected to panoramic scanning using a Hamamatsu NanoZoomer Digital Pathology (S210) slide scanner for pathological analysis.

**Table 3: Indicators for pathological evaluation of pulmonary fibrosis**

| Fibrosis grade | Ashcroft scoring criteria |
|---|---|
| 0 | Alveolar septum: no fibrotic lesions; |
| | lung structure: normal. |
| 1 | Alveolar septum: isolated gentle fibrotic changes (thickened alveolar septum, but less than three times that of normal lung); |
| | lung structure: partly enlarged alveolar cavity, small amount of exudate, no fibrotic material. |
| 2 | Alveolar septum: definite fibrotic changes (thickened alveolar septum, larger than three times that of normal lung), formation of small nodules, but not connected; |
| | lung structure: partly enlarged alveolar cavity, small amount of exudate, no fibrotic material. |
| 3 | Alveolar septum: non-intermittent fibrosis (thickened alveolar septum, larger than three times that of normal lung) is visible in almost all alveolar walls in each high-power field; |
| | lung structure: partly enlarged alveolar cavity, small amount of exudate, no fibrotic material. |
| 4 | Alveolar septum: still visible; |
| | lung structure: isolated fibrotic nodules in alveolar cavity (≤10% of high-power field). |
| 5 | Alveolar septum: still visible; |
| | lung structure: confluent fibrotic nodules in alveolar cavity (>10% to ≤50% of high-power field), lung architecture severely damaged but still existent. |
| 6 | Alveolar septum: visible but almost non-existent. |
| | lung structure: large contiguous fibrotic nodules (>50% of high-power field), lung architecture almost non-existent. |
| 7 | Alveolar septum: non-existent; |
| | lung structure: alveolar cavity almost filled with fibrotic material but still existing up to five vacuole-like structures. |
| 8 | Alveolar septum: non-existent; |
| | lung structure: alveolar cavity filled with fibrotic tissue in high-power field. |

**Table 4: Summary of pulmonary fibrosis scores (mean)**

| **Group** | **Total pulmonary fibrosis score** |
|---|---|
| G1 sham operation group (sham) | 0 |
| G2 model group | 4.69 |
| G3 Nintedanib | 3.39 |
| Compound I-3B | 3.34 |

The experimental results show that the compounds of the present disclosure can significantly reduce the degree of fibrosis in the mouse IPF prevention model.

### Test example 4: Efficacy test on mouse IPF treatment model

Male mice were adaptively fed for 1 to 2 weeks and after reaching the standard weight (25 g), the IPF model (idiopathic pulmonary fibrosis model) was induced with a certain dose of Bleomycin. On the day of modeling, the animals were randomly divided into a model group and an administration group according to their weight, in which the administration groups were administered Nintedanib (60 mg/kg, qd), low-dose compound I-3B of the present disclosure (1 mg/kg, bid), and high-dose compound I-3B of the present disclosure (2.5 mg/kg, bid) by oral gavage for 14 consecutive days starting from Day 7 while the vehicle control group was administered blank vehicle. During the administration period, the animals were weighed twice a week. At the end of the last day of administration, the animals were euthanized. The lungs were removed from the thyroid cartilage (without perfusion), slowly perfused with 10% formalin until both lungs were filled, and fixed in 10% formalin 5 to 10 times the volume of the tissues after the trachea was ligated. The left lung tissues were embedded in paraffin wax, sectioned, and stained with HE and Masson Trichrome. The sections were subjected to panoramic scanning using a Hamamatsu NanoZoomer Digital Pathology (S210) slide scanner for pathological analysis.

**Table 5: Indicators for pathological evaluation of pulmonary fibrosis**

| Fibrosis grade | Ashcroft scoring criteria |
|---|---|
| 0 | Alveolar septum: no fibrotic lesions; |
| | lung structure: normal. |
| 1 | Alveolar septum: isolated gentle fibrotic changes (thickened alveolar septum, but less than three times that of normal lung); |
| | lung structure: partly enlarged alveolar cavity, small amount of exudate, no fibrotic material. |
| 2 | Alveolar septum: definite fibrotic changes (thickened alveolar septum, larger than three times that of normal lung), formation of small nodules, but not connected; |
| | lung structure: partly enlarged alveolar cavity, small amount of exudate, no fibrotic material. |
| 3 | Alveolar septum: non-intermittent fibrosis (thickened alveolar septum, larger than three times that of normal lung) is visible in almost all alveolar walls in each high-power field; |
| | lung structure: partly enlarged alveolar cavity, small amount of exudate, no fibrotic material. |
| 4 | Alveolar septum: still visible; |
| | lung structure: isolated fibrotic nodules in alveolar cavity (≤10% of high-power field). |
| 5 | Alveolar septum: still visible; |
| | lung structure: confluent fibrotic nodules in alveolar cavity (>10% to ≤50% of high-power field), lung architecture severely damaged but still existent. |
| 6 | Alveolar septum: visible but almost non-existent. |
| | lung structure: large contiguous fibrotic nodules (>50% of high-power field), lung architecture almost non-existent. |
| 7 | Alveolar septum: non-existent; |
| | lung structure: alveolar cavity almost filled with fibrotic material but still existing up to five vacuole-like structures. |
| 8 | Alveolar septum: non-existent; |
| | lung structure: alveolar cavity filled with fibrotic tissue in high-power field. |

**Table 6: Summary of pulmonary fibrosis scores (mean)**

| **Group** | **Total pulmonary fibrosis score** |
|---|---|
| G1 sham | 0 |
| G2 model group | 3.68 |
| G3 Nintedanib | 2.84 |
| G4 low-dose compound I-3B group | 2.74 |
| G4 high-dose compound I-3B group | 2.6 |

The experimental results show that the compounds of the present disclosure can significantly reduce the degree of fibrosis in the mouse IPF treatment model.

### Test example 5: Efficacy test on mouse liver regeneration after resection

Male C57BL/6J mice of 8 weeks old (20 to 24 g) were anesthetized and fixed with the abdomen facing upwards. The surgical site was shaved and disinfected with iodophor. A transverse incision of about 1.5 to 2 cm was made at the abdomen, and the epigastric arteries on both sides were clamped with a hemostat. After opening the abdominal cavity, each liver lobe was freed, and the hilum of the liver lobe to be resected was ligated with surgical suture. The left outer lobe and middle lobe of the liver were resected as the color became darker. After surgery, the residual blood in the abdominal cavity was cleaned up, and the muscle layer and fur layer were sutured layer by layer. Postoperative care should be taken into consideration. Administration was started on the day of modeling, and 8 animals were killed on day 1 and day 3 of the administration, respectively. Intact liver tissues were collected and weighed for comparison with the model group to evaluate the effect of the drug on promoting liver regeneration.

The experimental results show that the compounds of the present disclosure can significantly promote liver regeneration.

### Test example 6: Pharmacokinetics in mice

The pharmacokinetic properties of the compounds of the present disclosure in mice were determined with reference to the following experimental methods.

Three male CD-1 mice were used, administered at a dose of 10 mg/kg, the route of administration was by gavage, and the vehicle was 5% DMSO + 10% Solutol + 85% Saline. The mice were fasted overnight, and the time points of blood collection were before administration and at 15 minutes, 30 minutes, 1 hour, 2 hours, 4 hours, 8 hours, and 24 hours after administration. Blood samples were centrifuged at 6800 g for 6 minutes at 2 to 8°C, and plasma was collected and stored at -80°C. 10 µL of plasma at each time point was taken, and added with 200 µL of methanol containing 100 ng/mL internal standard. The mixture was vortexed, mixed evenly, and then centrifuged at 18000 g for 7 minutes at 2 to 8°C. 200 µL of the mixture was transferred to a 96-well injection plate for LC-MS/MS quantitative analysis. The main pharmacokinetic parameters were analyzed using WinNonlin 7.0 software with non-compartmental model.

**Table 7: Pharmacokinetic data in mice**

| **Compound No.** | **Cₘₐₓ (ng/mL)** | **AUC₍₀₋ₜ₎ (h·ng/mL)** |
|---|---|---|
| Compound I-3 | 3710 | 35502 |
| Compound I-3B | 4480 | 33173 |
| Compound I-12 | 14800 | 107087 |
| Compound I-25 | 3710 | 35771 |
| Compound I-28 | 5040 | 10661 |

The experimental results show that the compounds of the present disclosure exhibit excellent pharmacokinetic properties in mice.

### Test example 7: Efficacy on IBD in mice

Female C57BL/6 mice aged 6 to 8 weeks were divided into 5 groups of G1 to G5, namely normal control group, model control group, positive control group, low-dose compound I-3B group, and high-dose compound I-3B group, respectively. The mice in G2 to G6 were given 2% DSS aqueous solution on day 0 to day 6, normal water on day 0 to day 10, vehicle/positive/test substance on day 0 to day 9, and euthanized on day 10 for necropsy. Colon weight (CW) and colon length (CL) were measured and colon tissues (BW) were subjected to histopathological examination. The test results showed that compared with the animals in the G2 model group, there was a significant increase in body weight of the animals in the G3-positive control group (cyclosporine CsA 25 mg/kg-qd); there was a significant decrease in DAI, a significant increase in CL, and a significant decrease in CW, CL/CW/BW, and CL/CW of the model animals; the results of the histopathological examination of colon tissues of the model animals showed that there was a decrease in inflammatory cell infiltration and tissue injury scores, but there was no significant difference. There was an increase in body weight of the animals in both G4-compound I-3B (2.5 mg/kg-bid) and G5-compound I-3B (5 mg/kg-bid) groups, with a significant increase in body weight of the animals in G5; there was a significant decrease in DAI, a significant increase in CL, and a significant decrease in CW, CL/CW/BW, and CL/CW of the animals in both groups; the results of the histopathological examination of colon tissues showed that there was a significant decrease in inflammatory cell infiltration and tissue injury scores in both groups.

**Table 8: Summary of colon length (CL), colon weight (CW), CL/CW/BW, and CW/CL of animals in each group**

| **Group** | **Colon length CL (cm)** | **Colon weight CW (g)** | **CW/CL/BW * 1000** | **CW/CL * 10** |
|---|---|---|---|---|
| G1-normal control group | 6.90 | 0.15 | 1.11 | 0.21 |
| G2-model control group | 5.52 | 0.21 | 2.31 | 0.38 |
| G3-CsA group 25 mg/kg | 6.70 | 0.18 | 1.42 | 0.27 |
| G4-low-dose compound I-3B group | 6.67 | 0.17 | 1.39 | 0.26 |
| G5-high-dose compound I-3B group | 6.50 | 0.16 | 1.36 | 0.24 |

The experimental results show that compound I-3B (2.5 mg/kg and 5 mg/kg) can significantly improve IBD symptoms and tissue injury in mice with the efficacy better than that of the positive control.

Although the examples of the present disclosure are illustrated and described above, it can be understood that the above examples are illustrative and should not be construed as limiting the present disclosure. Those skilled in the art can make changes, modifications, substitutions, and variations based on the above examples within the scope of the present disclosure.

## Claims

1. A heterocyclic compound of formula I, a solvate, a pharmaceutically acceptable salt, a solvate of the pharmaceutically acceptable salt, or a prodrug thereof: 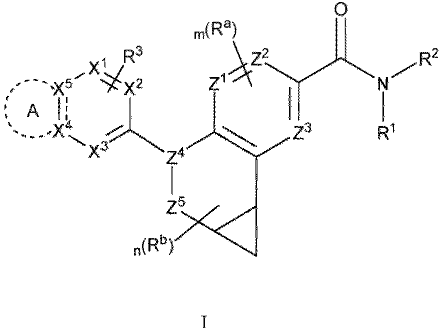 wherein
Z¹, Z², Z³, Z⁴, and Z⁵ each independently represent a ring atom;
Z¹, Z², Z³, Z⁴, and Z⁵ are each independently N, NH, O, S, CH₂, CH, or C;
R¹ and R² are each independently hydrogen, C₁-C₆ alkyl, or C₃-C₈ cycloalkyl,
or, R¹ and R² together with the N atom to which they are attached form a 3- to 11-membered heterocycloalkyl ring; wherein the 3- to 11-membered heterocycloalkyl ring is further substituted by 0, 1, or more than one R¹⁻¹; when there is more than one substituent, the substituents are the same or different;
each R¹⁻¹ is independently halogen, hydroxyl, amino, nitro, cyano, carbonyl, oxo, carboxyl, C₁-C₆ alkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
R^{a} and R^{b} are each independently halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, or halo-C₁-C₆ alkoxy;
m and n are each independently 0, 1, 2, or 3;
X¹, X², X³, X⁴, and X⁵ each independently represent a ring atom;
X^{l}, X², X³, X⁴, and X⁵ are each independently N, O, S, CH₂, CH, or C;
the bond between X⁴ and X⁵ is a single bond or a double bond;
R³ is hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
in the group moiety 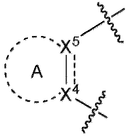 formed by the connection of X⁴ and X⁵, A is absent, or A together with the ring atoms X⁴ and X⁵ forms a 3- to 11-membered heterocycloalkyl ring, 5-membered heteroaromatic ring, or 6-membered heteroaromatic ring; the heteroatom is independently selected from one or more than one of N, O, and S;
when A is absent, X⁴ and X⁵ are each independently substituted by R⁴ or R⁵;
when A is present, the A is further substituted by R³⁻¹; the R³⁻¹ substitution is one or more than one substitution, and when there is more than one substituent, the substituents are the same or different;
R⁴ and R³⁻¹ are each independently hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
R⁵ is hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally substituted by 1 or 2 R⁵⁻¹ groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
each R⁵⁻¹ is independently hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups.

2. The heterocyclic compound of formula I, the solvate, the pharmaceutically acceptable salt, the solvate of the pharmaceutically acceptable salt, or the prodrug thereof according to claim 1, wherein the heterocyclic compound of formula I satisfies one or more than one of the following conditions:
(1) the R¹ and R² together with the N atom to which they are attached form a 3- to 11-membered heterocycloalkyl ring, in which the heterocycloalkyl ring is a monocyclic heterocycloalkyl ring, a bridged bicyclic heterocycloalkyl ring, or a spiro bicyclic heterocycloalkyl;
(2) the R¹ and R² together with the N atom to which they are attached form a 3- to 11-membered heterocycloalkyl ring, in which the heteroatom is one or more than one of N, O, or S, and the number of heteroatoms is 1, 2, 3, or 4;
(3) the R¹ and R² together with the N atom to which they are attached form a 3- to 11-membered heterocycloalkyl ring, in which the 3- to 11-membered heterocycloalkyl ring is a 3-to 8-membered heterocycloalkyl ring;
(4) in R¹⁻¹, the halogen and the halogen in the halo-C₁-C₆ alkyl are each independently F, Cl, or Br, such as F;
(5) in R¹⁻¹, the C₁-C₆ alkyl and the C₁-C₆ alkyl in the halo-C₁-C₆ alkyl are each independently methyl, ethyl, n-propyl, isopropyl, n-butyl, isobutyl, sec-butyl, or tert-butyl, such as methyl;
(6) in R⁴ and R⁵, each halogen is independently F, Cl, or Br, such as F;
(7) in R⁴ and R⁵, each C₁-C₆ alkyl in the aminocarbonyl optionally having 1 or 2 C₁-C₆ alkyl groups is independently methyl, ethyl, n-propyl, isopropyl, n-butyl, isobutyl, sec-butyl, or tert-butyl, such as methyl;
(8) the A together with the ring atoms X⁴ and X⁵ forms a 3- to 11-membered heterocycloalkyl ring, in which the heterocycloalkyl ring is a monocyclic heterocycloalkyl ring or a spiro bicyclic heterocycloalkyl ring;
(9) the A together with the ring atoms X⁴ and X⁵ forms a 3- to 11-membered heterocycloalkyl ring, in which the 3- to 11-membered heterocycloalkyl ring is a 3- to 7-membered heterocycloalkyl ring;
(10) the A together with the ring atoms X⁴ and X⁵ forms a 3- to 11-membered heterocycloalkyl ring, in which the number of heteroatoms in the heterocycloalkyl ring is 1, 2, 3, or 4;
(11) the A together with the ring atoms X⁴ and X⁵ forms a 5-membered heteroaromatic ring or 6-membered heteroaromatic ring, in which the number of heteroatoms is 1, 2, 3, or 4;
(12) when A is present and the A is further substituted by R³⁻¹, the number of the R³⁻¹ is 2;
(13) in R³⁻¹, the halogen in the halo-C₁-C₆ alkyl is F, Cl, or Br, such as F;
(14) in R³⁻¹, the C₁-C₆ alkyl and the C₁-C₆ alkyl in the halo-C₁-C₆ alkyl and C₁-C₆ deuteroalkyl are each independently methyl, ethyl, n-propyl, isopropyl, n-butyl, isobutyl, *sec-*butyl, or tert-butyl, such as methyl or ethyl;
(15) in R³⁻¹, the cycloalkyl in the C₃-C₈ cycloalkyl is monocyclic cycloalkyl;
(16) in R³⁻¹, the cycloalkyl in the C₃-C₈ cycloalkyl is cyclopropyl, cyclobutyl, cyclopentyl, or cyclohexyl;
(17) 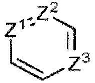 is phenyl or "a 6-membered heteroaromatic ring having 1, 2, or 3 heteroatoms selected from one or more than one of N, O, and S", such as a pyridine ring;
the phenyl is preferably 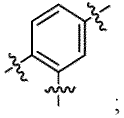
the pyridine ring is preferably 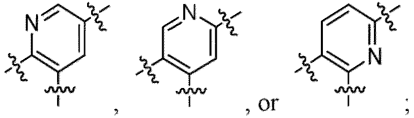
(18) 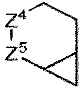 is "a 6-membered heteroalkyl ring substituted by cyclopropane having 1 heteroatom selected from N and O" or "a 6-membered heteroalkyl ring substituted by cyclopropane having 1 heteroatom selected from N", such as a pyridine ring.

3. The heterocyclic compound of formula I, the solvate, the pharmaceutically acceptable salt, the solvate of the pharmaceutically acceptable salt, or the prodrug thereof according to claim 2, wherein the heterocyclic compound of formula I satisfies one or more than one of the following conditions:
(1) the 3- to 8-membered heterocycloalkyl ring is a monocyclic 3- to 6-membered heterocycloalkyl ring, a bridged bicyclic 6- to 8-membered heterocycloalkyl ring, or a spiro bicyclic 8-membered heterocycloalkyl ring, and the heterocycloalkyl ring has 1 or 2 heteroatoms being N and/or O, such as azetidinyl, pyrrolidinyl, piperidinyl, morpholinyl, pyrrolidinyl-fused cyclopropyl, oxazaspiro[2.5]octyl, azaspiro[2.5]octyl, or octahydrocyclopenta[c]pyrrolidinyl;
(2) the 3- to 8-membered heterocycloalkyl ring is further substituted by halogen and/or halo-C₁-C₆ alkyl;
(3) in R¹⁻¹, the halo-C₁-C₆ alkyl is trifluoromethyl, difluoromethyl, or monofluoromethyl, preferably 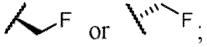
(4) in R⁴ and R⁵, each aminocarbonyl optionally having 1 or 2 C₁-C₆ alkyl groups is independently 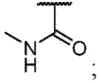
(5) the A together with the ring atoms X⁴ and X⁵ forms a 3- to 11-membered heterocycloalkyl ring, in which the 3- to 11-membered heterocycloalkyl ring is a monocyclic 3- to 6-membered heterocycloalkyl ring or a spiro bicyclic 7-membered heterocycloalkyl ring, and the heterocycloalkyl ring has 1, 2, or 3 heteroatoms independently being N and/or O, such as pyrrolidinyl or azaspiro[2.4]heptyl;
(6) the A together with the ring atoms X⁴ and X⁵ forms a 5-membered heteroaromatic ring or 6-membered heteroaromatic ring, and the heteroaromatic ring has 1, 2, or 3 heteroatoms being N, such as a pyridine ring, a pyrimidine ring, or a triazole ring;
(7) in R³⁻¹, the halo-C₁-C₆ alkyl is 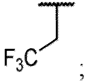
(8) in R³⁻¹, the C₁-C₆ deuteroalkyl is -CD₃;
(9) the rings of 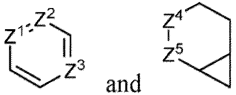 are connected to form 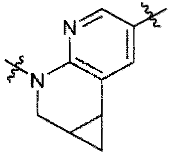 or 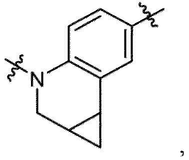 such as 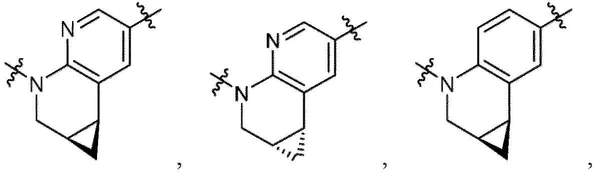 or

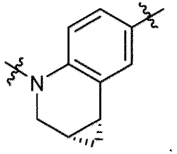

4. The heterocyclic compound of formula I, the solvate, the pharmaceutically acceptable salt, the solvate of the pharmaceutically acceptable salt, or the prodrug thereof according to claim 1, wherein the heterocyclic compound of formula I satisfies (1) and/or (2):
(1) R¹ and R² together with the N atom to which they are attached form the following groups: 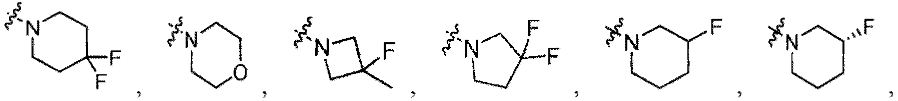 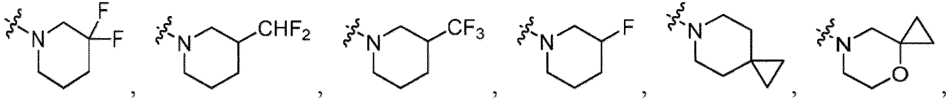 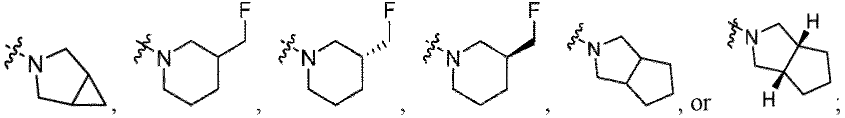
(2)

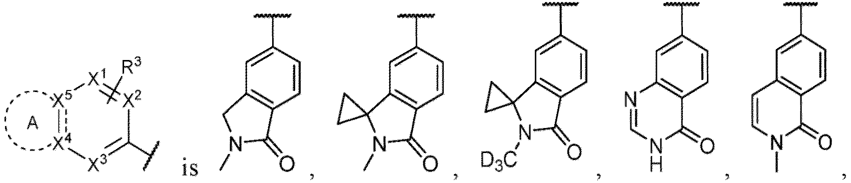

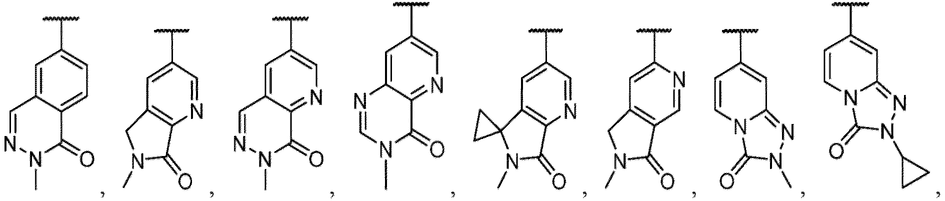

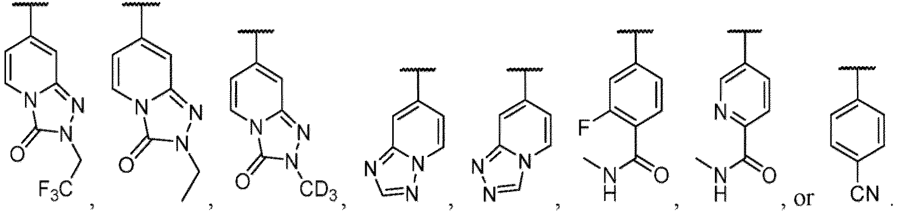

5. The heterocyclic compound of formula I, the solvate, the pharmaceutically acceptable salt, the solvate of the pharmaceutically acceptable salt, or the prodrug thereof according to claim 1, wherein the heterocyclic compound of formula I satisfies one or more than one of the following conditions:
(1) Z¹, Z², and Z³ are each independently N, CH, or C;
(2) Z⁴ and Z⁵ are each independently N, NH, CH₂, or CH;
(3) R¹ and R² together with the N atom to which they are attached form a 3- to 11-membered heterocycloalkyl ring; wherein the 3- to 11-membered heterocycloalkyl ring is further substituted by 0, 1, or more than one R¹⁻¹,
(4) R¹⁻¹ is halogen, C₁-C₆ alkyl, or halo-C₁-C₆ alkyl;
(5) R⁴ and R⁵ are independently halogen, cyano, or aminocarbonyl optionally having 1 or 2 C₁-C₆ alkyl groups;
(6) R³⁻¹ is independently oxo, C₁-C₆ alkyl, halo-C₁-C₆ alkyl, C₃-C₈ cycloalkyl, or C₁-C₆ deuteroalkyl;
(7) the solvate of the pharmaceutically acceptable salt of the heterocyclic compound of formula I is a hydrate;
(8) the heterocyclic compound of formula I has a structure of formula I-1 or formula I-2:

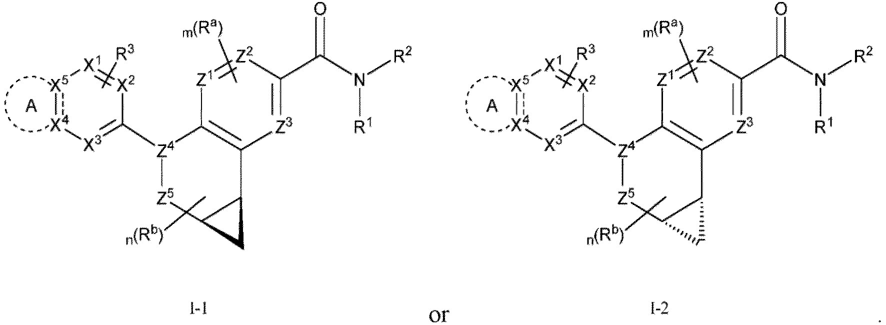

6. The heterocyclic compound of formula I, the solvate, the pharmaceutically acceptable salt, the solvate of the pharmaceutically acceptable salt, or the prodrug thereof according to any one of claims 1 to 5, wherein the heterocyclic compound of formula I is any one of the following schemes:
scheme **1:**
the heterocyclic compound of formula I is a compound of formula I': 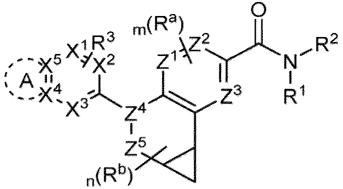 in formula I', Z¹, Z², Z³, Z⁴, and Z⁵ each independently represent a ring atom;
Z¹, Z², Z³, Z⁴, and Z⁵ are each independently N or C;
R¹ and R² are each independently hydrogen, C₁-C₆ alkyl, or C₃-C₈ cycloalkyl;
or, R¹ and R² together with the N atom to which they are attached form a 3- to 11-membered heterocycloalkyl ring; wherein the C₃-C₈ cycloalkyl or 3- to 11-membered heterocycloalkyl ring is further substituted by R¹⁻¹,
each R¹⁻¹ is independently halogen, hydroxyl, amino, nitro, cyano, carbonyl, oxo (=O), carboxyl, C₁-C₆ alkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
R^{a} and R^{b} are each independently halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, or halo-C₁-C₆ alkoxy;
m and n are each independently 0, 1, 2, or 3;
X¹, X², X³, X⁴, and X⁵ each independently represent a ring atom;
X¹, X², X³, X⁴, and X⁵ are each independently N or C;
R³ is halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, or halo-C₁-C₆ alkoxy;
in the group moiety 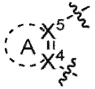 formed by the connection of X⁴ and X⁵, A is absent, or A together with the ring atoms X⁴ and X⁵ forms a 3- to 11-membered heterocycloalkyl ring, 5-membered heteroaromatic ring, or 6-membered heteroaromatic ring; the heteroatom is selected from one or more than one of N, O, and S;
when A is absent, X⁴ and X⁵ are each independently substituted by R⁴ or R⁵;
when A is present, the A is further substituted by R³⁻¹; the R³⁻¹ substitution is one or more than one substitution, and when there is more than one substituent, the substituents are the same or different;
R⁴, R⁵, and R³⁻¹ are each independently hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
scheme 2:
in the heterocyclic compound of formula I:
Z¹, Z², and Z³ are each independently N or C;
Z⁴ and Z⁵ are each independently N, NH, CH₂, or CH;
R¹ and R² together with the N atom to which they are attached form a 3- to 11-membered heterocycloalkyl ring; wherein the 3- to 11-membered heterocycloalkyl ring is further substituted by 0, 1, or more than one R¹⁻¹,
R¹⁻¹ is halogen, C₁-C₆ alkyl, or halo-C₁-C₆ alkyl;
m and n are 0;
R³ is hydrogen;
R⁴ and R⁵ are independently halogen, cyano, or aminocarbonyl optionally having 1 or 2 C₁-C₆ alkyl groups;
R³⁻¹ is independently oxo, C₁-C₆ alkyl, halo-C₁-C₆ alkyl, C₃-C₈ cycloalkyl, or C₁-C₆ deuteroalkyl;
the group moiety 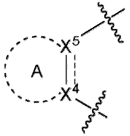 formed by the connection of X⁴ and X⁵ is as defined in claim 1;
scheme 3:
the heterocyclic compound of formula I has a structure of formula II-1 as follows: 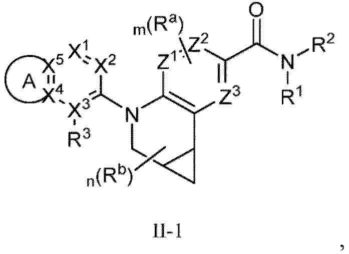 each group in formula II-1 is defined as follows in case 3-1 or case 3-2:
case 3-1:
X⁴ and X⁵ each independently represent a ring atom;
X⁴ and X⁵ are each independently N, CH, or C; A together with X⁴ and X⁵ forms a 3- to 8-membered heterocycloalkyl ring, and the 3- to 8-membered heterocycloalkyl ring is further substituted by R³⁻¹; the R³⁻¹ substitution is one or more than one substitution, and when there is more than one substituent, the substituents are the same or different;
each R³⁻¹ is independently hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
Z¹, Z², Z³, X¹, X², X³, R¹, R², R³, R^{a}, R^{b}, m, and n are as defined in claim 1;
case 3-2:
X⁴ and X⁵ each independently represent a ring atom;
X⁴ and X⁵ are each independently N or C; A together with X⁴ and X⁵ forms a 3- to 8-membered heterocycloalkyl ring, and the 3- to 8-membered heterocycloalkyl ring is further substituted by R³⁻¹; the R³⁻¹ substitution is one or more than one substitution, and when there is more than one substituent, the substituents are the same or different;
each R³⁻¹ is independently hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
Z¹, Z², Z³, X¹, X², X³, R¹, R², R³, R^{a}, R^{b}, m, and n are as defined in claim 1;
scheme 4:
the heterocyclic compound of formula I has a structure of formula II-2 as follows: 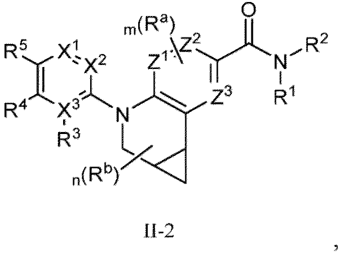
each group in formula II-2 is defined as follows in case **4-1** or case **4-2:**
case **4-1:**
R³, R⁴, and R⁵ are each independently hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, and amino optionally having 1 or 2 C₁-C₆ alkyl groups;
Z¹, Z², Z³, X¹, X², X³, R¹, R², R^{a}, R^{b}, m, and n are as defined in claim 1;
case **4-2:**
R³ is halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, or halo-C₁-C₆ alkoxy;
R⁴ and R⁵ are each independently hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
Z¹, Z², Z³, X¹, X², X³, R¹, R², R^{a}, R^{b}, m, and n are as defined in claim 1;
case **4-3:**
R³ is H, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, or halo-C₁-C₆ alkoxy;
R⁴ and R⁵ are each independently hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
Z¹, Z², Z³, X¹, X², X³, R¹, R², R^{a}, R^{b}, m, and n are as defined in claim 1;
scheme 5:
the heterocyclic compound of formula I has a structure of formula III-1, III-2, III-3, III-4, III-5, III-6, III-7, or III-8 as follows: 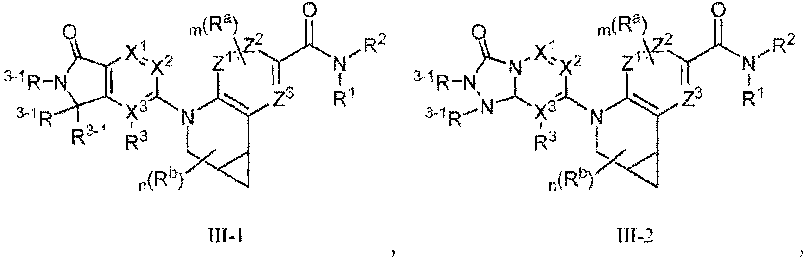 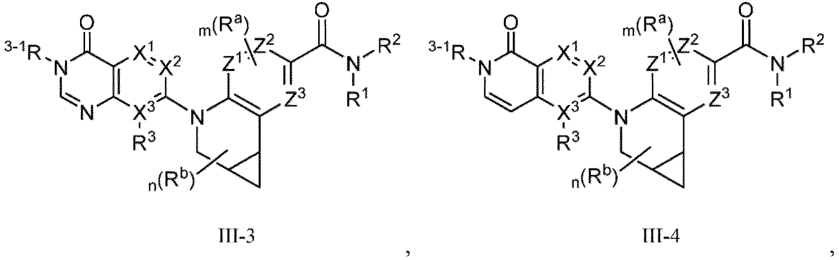 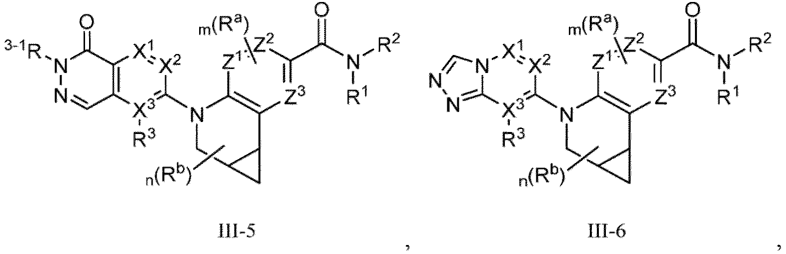 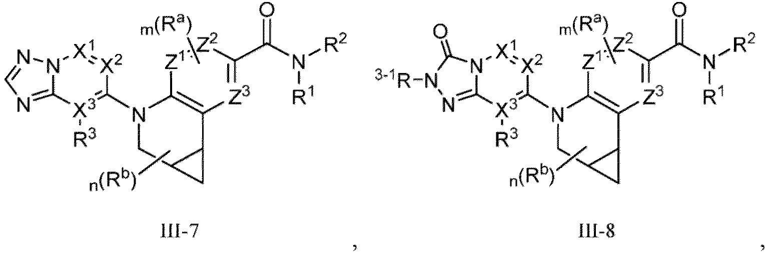 or 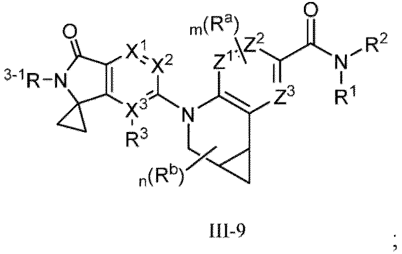
wherein Z¹, Z², Z³, X¹, X², X³, R¹, R², R³, R³⁻¹, R^{a}, R^{b}, m, and n are as defined in claim 1;
scheme 6:
the heterocyclic compound of formula I has a structure of formula IV-1 as follows: 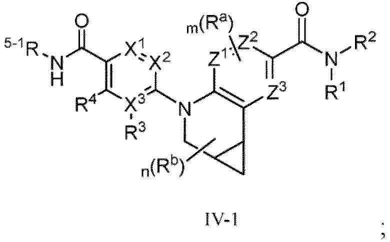
each group in formula IV-1 is defined as follows in case 6-1 or case 6-2:
case 6-1:
R⁵⁻¹ is hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
Z¹, Z², Z³, X¹, X², X³, R¹, R², R³, R^{a}, R^{b}, m, and n are as defined in claim 1;
case 6-2:
R⁵⁻¹ is selected from hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, C₁-C₆ alkylsulfonylamino, or amino optionally having 1 or 2 C₁-C₆ alkyl groups;
Z¹, Z², Z³, X¹, X², X³, R¹, R², R³, R^{a}, R^{b}, m, and n are as defined in claim 1;
scheme 7:
the heterocyclic compound of formula I has a structure of formula IV-2 as follows: 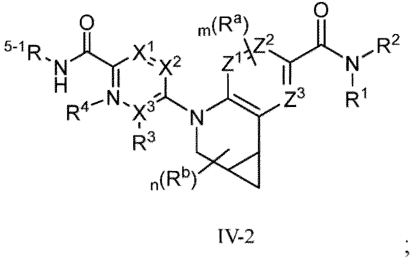
each group in formula IV-2 is defined as follows in case 7-1 or case 7-2:
case 7-1:
R⁵⁻¹ is hydrogen, halogen, hydroxyl, amino, nitro, cyano, carbonyl, oxo (=O), carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, aminocarbonyl optionally having 1 or 2 C₁-C₆ alkyl groups, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
Z¹, Z², Z³, X¹, X², X³, R¹, R², R³, R^{a}, R^{b}, m, and n are as defined in claim 1; R⁴ is absent;
case 7-2:
R⁵⁻¹ is hydrogen, halogen, hydroxyl, amino, nitro, cyano, carbonyl, oxo, carboxyl, C₁-C₆ alkyl, C₁-C₆ deuteroalkyl, C₂-C₆ alkynyl, halo-C₁-C₆ alkyl, hydroxy-C₁-C₆ alkyl, C₁-C₆ alkylcarbonyl, C₁-C₆ alkoxy, halo-C₁-C₆ alkoxy, C₁-C₆ alkoxycarbonyl, C₁-C₆ alkylcarbonyloxy, C₁-C₆ alkoxy-C₁-C₆ alkyl, C₁-C₆ alkoxycarbonyl-C₁-C₆ alkyl, C₃-C₈ cycloalkyl, C₃-C₈ cycloalkylcarbonyl, C₃-C₈ cycloalkoxy, C₁-C₆ alkylsulfonyl, aminosulfonyl optionally having 1 or 2 C₁-C₆ alkyl groups, C₁-C₆ alkylsulfonylamino, or amino optionally having 1 or 2 C₁-C₆ alkyl groups;
Z¹, Z², Z³, X¹, X², X³, R¹, R², R³, R^{a}, R^{b}, m, and n are as defined in claim 1; R⁴ is absent.

7. The heterocyclic compound of formula I, the solvate, the pharmaceutically acceptable salt, the solvate of the pharmaceutically acceptable salt, or the prodrug thereof according to claim 1, wherein the heterocyclic compound of formula I is selected from any one of the following compounds:

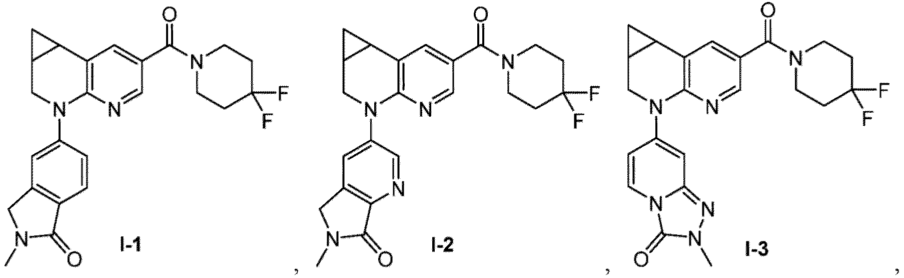

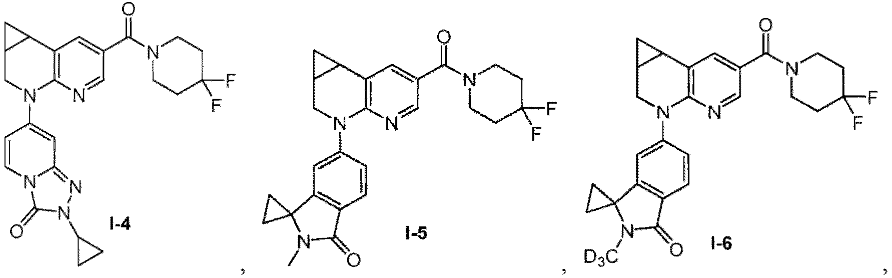

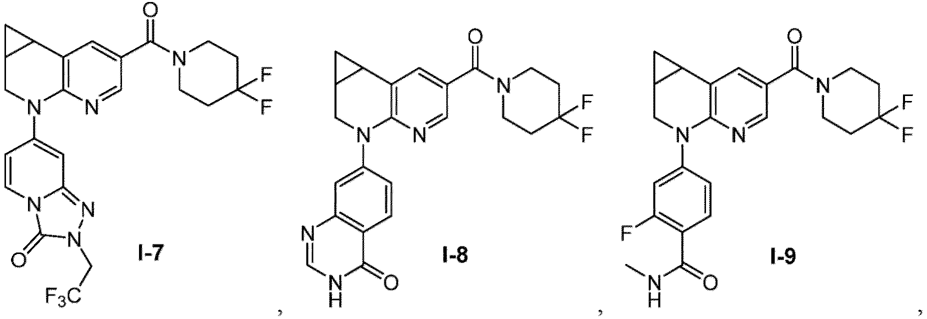

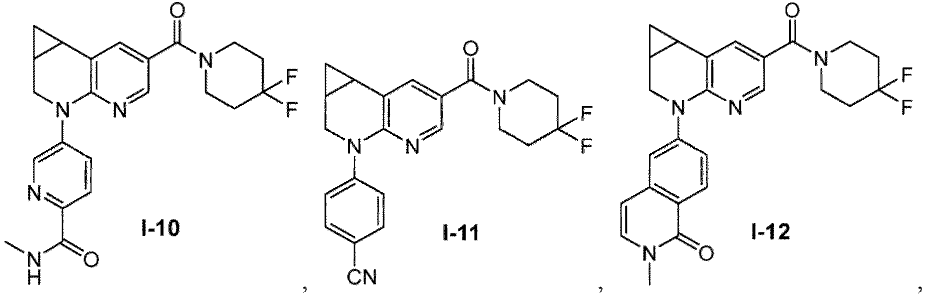

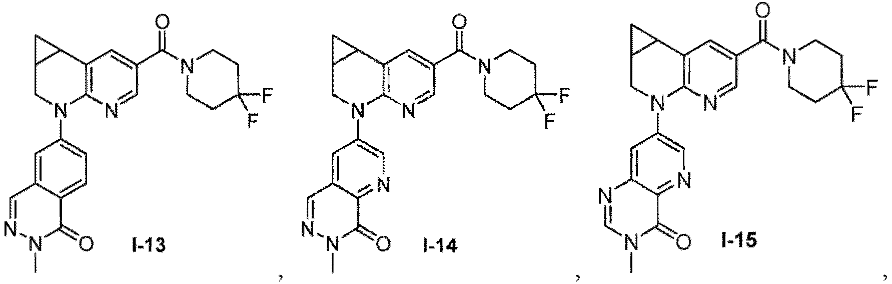

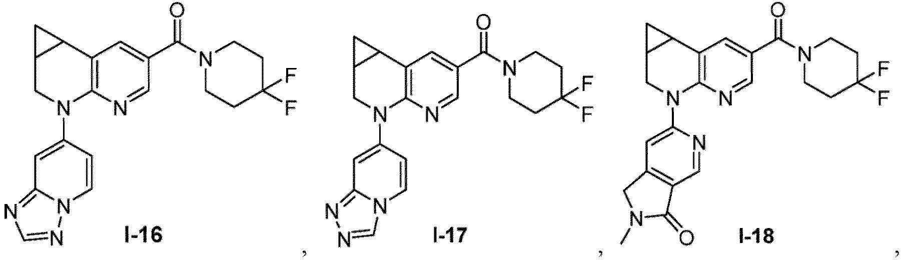

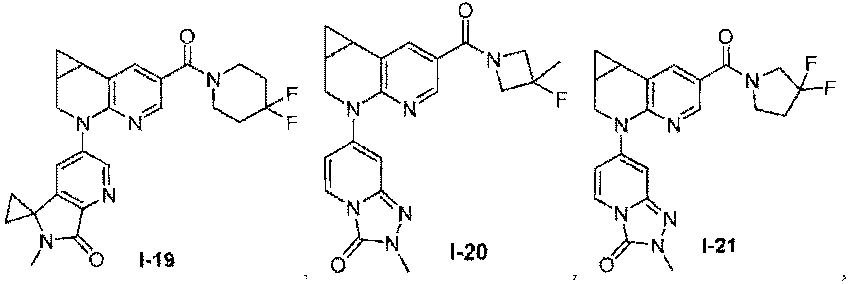

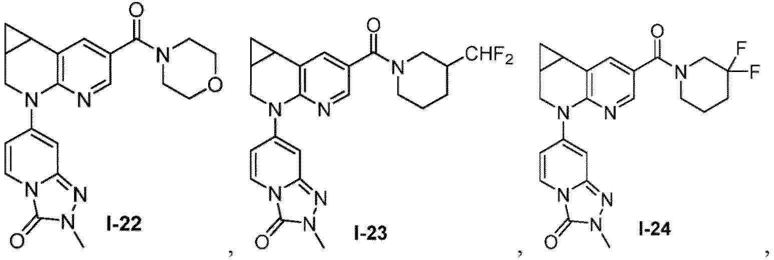

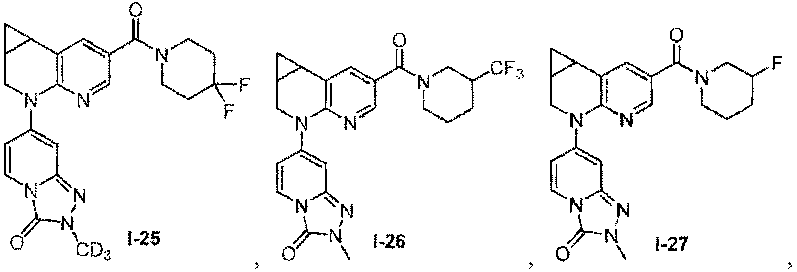

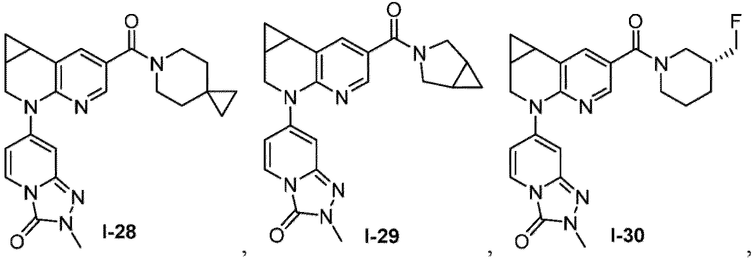

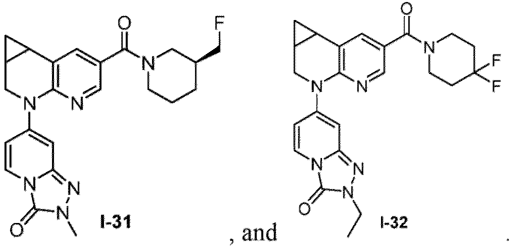

8. The heterocyclic compound of formula I, the solvate, the pharmaceutically acceptable salt, the solvate of the pharmaceutically acceptable salt, or the prodrug thereof according to claim 7, wherein the heterocyclic compound of formula I is selected from any one of the following compounds: 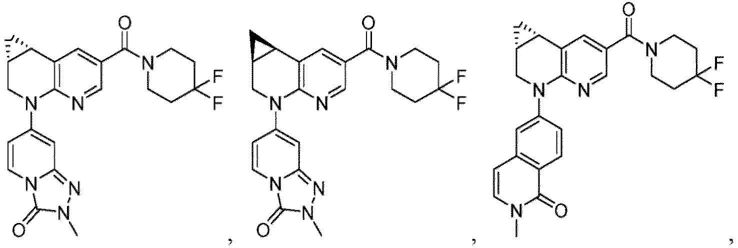 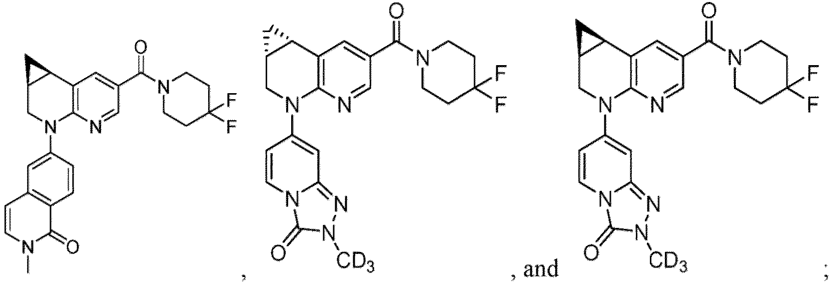
or the heterocyclic compound of formula I is selected from any one of the following compounds: 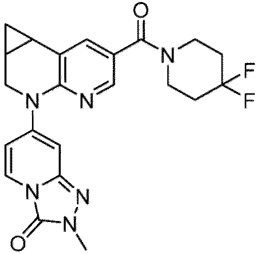 with a retention time of 0.743 min under the following SFC conditions:
chromatographic column: Chiralpak AD-3 50 × 4.6 mm I.D., 3 µm, mobile phase: mobile phase A: COz, mobile phase B: a solution of isopropanol and acetonitrile containing 0.05 vol% diethylamine;
isocratic elution: 50 vol% solution of isopropanol and acetonitrile containing 0.05 vol% diethylamine in COz, flow rate: 3 mL/min; detector: PDA, column temperature: 35°C; column pressure: 100 Bar; 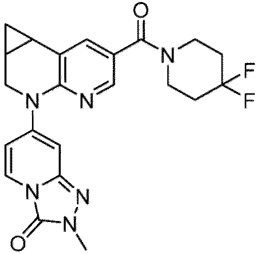 with a retention time of 1.670 min under the following SFC conditions:
chromatographic column: Chiralpak AD-3 50 × 4.6 mm I.D., 3 µm, mobile phase: mobile phase A: COz, mobile phase B: a solution of isopropanol and acetonitrile containing 0.05 vol% diethylamine;
isocratic elution: 50 vol% solution of isopropanol and acetonitrile containing 0.05 vol% diethylamine in COz, flow rate: 3 mL/min; detector: PDA, column temperature: 35°C; column pressure: 100 Bar; 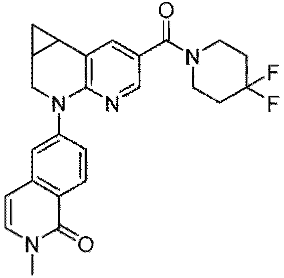 with a retention time of 0.816 min under the following SFC conditions:
chromatographic column: Chiralpak AS-3 50 × 4.6 mm I.D., 3 µm; mobile phase: mobile phase A: COz, mobile phase B: a solution of isopropanol and acetonitrile containing 0.05 vol% diethylamine; isocratic elution: 40 vol% solution of isopropanol and acetonitrile containing 0.05 vol% diethylamine in COz; flow rate: 3 mL/min; detector: PDA, column temperature: 35°C; column pressure: 100 Bar; 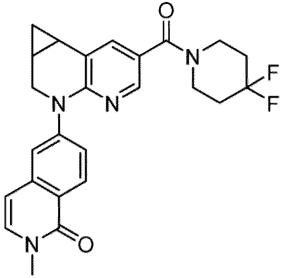 with a retention time of 1.477 min under the following SFC conditions:
chromatographic column: Chiralpak AS-3 50 × 4.6 mm I.D., 3 µm; mobile phase: mobile phase A: COz, mobile phase B: a solution of isopropanol and acetonitrile containing 0.05 vol% diethylamine; isocratic elution: 40 vol% solution of isopropanol and acetonitrile containing 0.05 vol% diethylamine in COz; flow rate: 3 mL/min; detector: PDA, column temperature: 35°C; column pressure: 100 Bar; 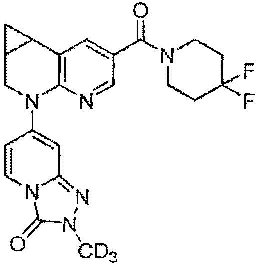 with a retention time of 0.764 min under the following SFC conditions:
chromatographic column: Chiralpak AD-3 50 × 4.6 mm I.D., 3 µm, mobile phase: mobile phase A: COz, mobile phase B: a solution of isopropanol and acetonitrile containing 0.05 vol% diethylamine; isocratic elution: 50 vol% solution of isopropanol and acetonitrile containing 0.05 vol% diethylamine in COz, flow rate: 3 mL/min; detector: PDA, column temperature: 35°C; column pressure: 100 Bar;
and 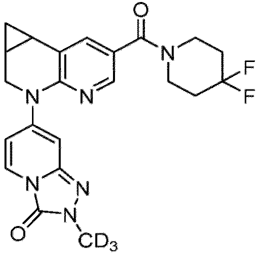 with a retention time of 1.702 min under the following SFC conditions:
chromatographic column: Chiralpak AD-3 50 × 4.6 mm I.D., 3 µm, mobile phase: mobile phase A: COz, mobile phase B: a solution of isopropanol and acetonitrile containing 0.05 vol% diethylamine; isocratic elution: 50 vol% solution of isopropanol and acetonitrile containing 0.05 vol% diethylamine in COz, flow rate: 3 mL/min; detector: PDA, column temperature: 35°C; column pressure: 100 Bar;
or the heterocyclic compound of formula I is selected from any one of the following compounds: 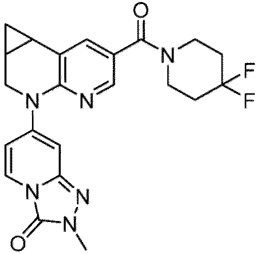 with an IC₅₀ of 12.16 nM under the conditions of test example 1; 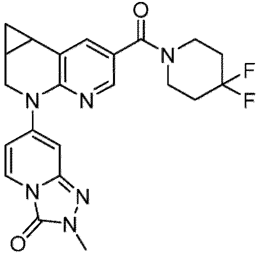 with an IC₅₀ of 3.58 nM under the conditions of test example 1; 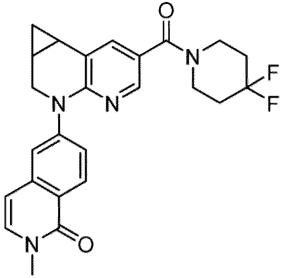 with an IC₅₀ of 1.52 nM under the conditions of test example 1; 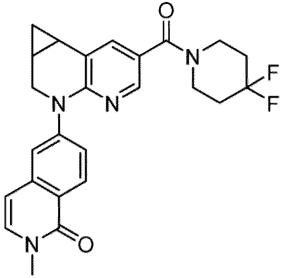 with an IC₅₀ of 7.42 nM under the conditions of test example 1; 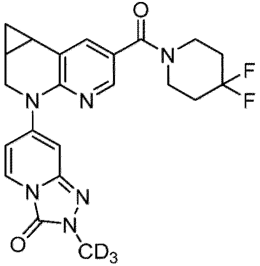 with an IC₅₀ of 15.8 nM under the conditions of test example 1;
and 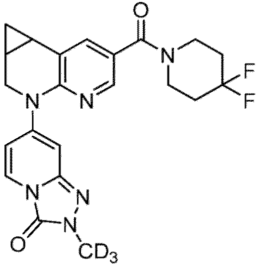 with an IC₅₀ of 3.78 nM under the conditions of test example 1;
or 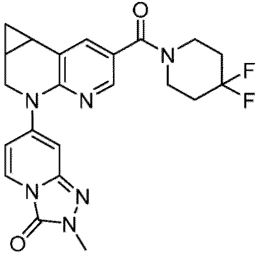 with a total pulmonary fibrosis score of 3.34 under the conditions of test example 3;
or 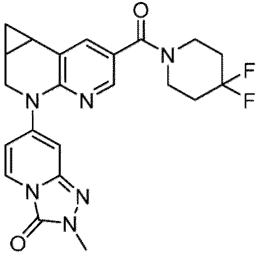 with a Cₘₐₓ of 4480 ng/mL under the conditions of test example 5;
or 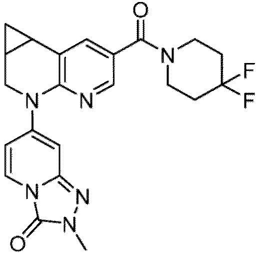 with an AUC₍₀₋ₜ₎ of 33173 h·ng/mL under the conditions of test example 5.

9. A pharmaceutical composition, comprising: the heterocyclic compound of formula I, the solvate, the pharmaceutically acceptable salt, the solvate of the pharmaceutically acceptable salt, or the prodrug thereof according to any one of claims 1 to 8; and a pharmaceutically acceptable carrier.

10. A use of a substance, wherein the substance is the heterocyclic compound of formula I, the solvate, the pharmaceutically acceptable salt, the solvate of the pharmaceutically acceptable salt, or the prodrug thereof according to any one of claims 1 to 8, or the pharmaceutical composition according to claim 9;
the use is for the preparation of a 15-PGDH inhibitor, or the preparation of a drug for the prevention and/or treatment of a disease related to 15-PGDH.

11. The use according to claim 10, wherein the disease related to 15-PGDH is one, two, or more of fibrotic disease, inflammatory disease, cardiovascular disease, trauma, autoimmune disease, graft-versus-host disease, hair growth, osteoporosis, ear disease, eye disease, neutropenia, diabetes, underactive bladder, implant promotion in stem cell or bone marrow transplantation or organ transplantation, neurogenesis and neuronal cell death, hematopoietic reconstruction, tissue injury, cervical disease, or kidney disease; preferably one or more than one of fibrotic disease, inflammatory disease, or tissue injury.

12. The use according to claim 11, wherein the fibrotic disease is one or more than one of pulmonary fibrosis, liver fibrosis, renal fibrosis, myocardial fibrosis, scleroderma, or myelofibrosis, preferably pulmonary fibrosis and/or liver fibrosis, such as pulmonary fibrosis;
the pulmonary fibrosis is preferably idiopathic pulmonary fibrosis;
and/or, the inflammatory disease is one or more than one of chronic obstructive pulmonary disease, acute lung injury, sepsis, exacerbation of asthma and pulmonary disease, inflammatory bowel disease, peptic ulcer, autoinflammatory disease, vasculitis syndrome, acute liver injury, acute kidney injury, non-alcoholic fatty liver disease, atopic dermatitis, psoriasis, interstitial cystitis, or prostatitis syndrome; preferably inflammatory bowel disease;
the inflammatory bowel disease is preferably ulcerative colitis and/or Crohn's disease;
the peptic ulcer is preferably NSAID-induced ulcer;
the autoinflammatory disease is preferably Behcet's disease;
the prostatitis syndrome is preferably chronic prostatitis and/or chronic pelvic pain syndrome;
and/or, the cardiovascular disease is one or more than one of pulmonary hypertension, angina, myocardial infarction, heart failure, ischemic heart disease, stroke, or peripheral circulatory disorder;
and/or, the trauma is one or more than one of diabetic ulcer, burn, pressure ulcer, acute mucosal injury, including Stevens-Johnson syndrome, mucosal injury, injury related to an anticancer chemotherapeutic agent, or injury related to antimetabolites, cellular or humoral immunotherapy or radiation;
the anticancer chemotherapeutic agent is preferably one or more than one of an alkylating agent, a DNA synthesis inhibitor, or a DNA gyrase inhibitor;
and/or, the autoimmune disease is multiple sclerosis and/or rheumatoid arthritis;
and/or, the ear disease is one or more than one of hearing loss, tinnitus, vertigo, or balance disorder;
and/or, the eye disease is glaucoma and/or dry eye;
and/or, the neurogenesis and neuronal cell death is one or more than one of neuropsychiatric disorder, neuropathy, neurotoxic disease, neuropathic pain, or neurodegenerative disease;
and/or, the tissue injury is liver injury and/or muscle injury;
the muscle injury is preferably muscle atrophy and/or muscular dystrophy;
and/or, the kidney disease is chronic kidney disease and/or renal failure.

13. The use according to claim 10, wherein the prevention and/or treatment of a disease related to 15-PGDH is the prevention and/or treatment of liver regeneration.

14. A use of the heterocyclic compound of formula I, the solvate, the pharmaceutically acceptable salt, the solvate of the pharmaceutically acceptable salt, or the prodrug thereof according to any one of claims 1 to 8, or the pharmaceutical composition according to claim 9, wherein the use is for the preparation of a drug for the prevention or treatment of a disease; the disease is one or more than one of fibrotic disease, inflammatory disease, or tissue injury;
preferably, the fibrotic disease, the inflammatory disease, and the tissue injury are as described in claim 12.

15. A method for inhibiting 15-PGDH or preventing or treating a disease, comprising the steps of: administering to a subject in need the heterocyclic compound of formula I, the solvate, the pharmaceutically acceptable salt, the solvate of the pharmaceutically acceptable salt, or the prodrug thereof according to any one of claims 1 to 8, or the pharmaceutical composition according to claim 9; the disease is one or more than one of a disease related to 15-PGDH, fibrotic disease, inflammatory disease, or tissue injury;
preferably, the disease related to 15-PGDH is as described in claim 12;
the fibrotic disease, the inflammatory disease, and the tissue injury are as described in any one of claims 10 to 13.
